# EUROPEAN PATENT SPECIFICATION

(11) **EP 2 967 925 B1**
(45) Date of publication and mention of the grant of the patent: **30.10.2019**
(21) Application number: 14770297.1
(22) Date of filing: 14.03.2014
(51) Int. Cl.: A61F 2/80, A61F 2/56, A61F 2/62, A61F 2/76, A61F 2/50, A61F 2/60, A61F 2/78

(54) **MODULAR PROSTHETIC SOCKETS**
MODULARE PROTHESENAUFNAHMEN
EMBOÎTURES PROTHÉTIQUES MODULAIRES

(30) Priority: 14.03.2013 US 201361783662 P; 21.11.2013 US 201361907287 P
(43) Date of publication of application: 20.01.2016
(73) Proprietor: LIM Innovations, Inc., San Francisco, CA 94103 (US)
(72) Inventor: HURLEY, Garrett Ray, San Francisco, CA 94110 (US); WILLIAMS, Jesse Robert, San Francisco, CA 94117 (US)
(74) Representative: Kazi, Ilya
(86) International application number: PCT/US2014/029773
(87) International publication number: WO 2014/153244

(56) References cited:
- GB-A- 2 080 114
- US-A- 4 459 709
- US-A- 4 783 293
- US-A- 5 201 775
- US-A- 5 312 669
- US-A1- 2004 158 332
- US-A1- 2006 020 349
- US-A1- 2010 082 116
- US-A1- 2013 123 940

## Description

### TECHNICAL FIELD OF THE TECHNOLOGY

The technology relates to the field of prosthetic systems and devices, and to materials used in their fabrication. The technology further relates to methods of making and using such systems and devices.

### BACKGROUND

Prosthetic limbs for the upper and lower extremities typically include a residual limb socket, an alignment system, and a distal prosthetic component, such as a knee, foot, arm, or hand. For any prosthetic limb, the prosthetic socket is the portion of the prosthesis that is designed to fit on the residual limb and connect with the rest of the prosthetic components. The prosthetic socket contains the residual limb and provides the functional connection to the distal components. If the prosthetic socket does not fit properly, it will often be very uncomfortable for the patient, and the function of distal components of the prosthetic may be severely compromised.

The process of designing and making a prosthetic socket is very patient-specific, and current methods are labor intensive and require highly skilled craftsmen. The process begins by a prosthetist evaluating a patient's condition and needs and taking measurements of the patient's residual limb. Typically, the prosthetist then casts a negative mold of the residual limb with casting tape. This negative mold is filled with Plaster of Paris and allowed to harden. The negative cast is then peeled off to reveal the newly formed positive mold. The prosthetist may then modify the positive mold in an effort to create a form that best supports the creation of a limb socket that distributes pressure optimally on the residual limb when the socket is worn. The prosthetic socket is then built up by laminating layers of polymer material over the positive mold. Finally, the positive mold is broken and removed from within the fabricated socket, and the prosthetic socket may then be cut or further modified to fit the residual limb of the patient.

Additional steps of the prosthetic socket fabrication process may also be required in many cases, such as making and integrating flexible inner liners, locking mechanisms, alignment mechanisms, and other components, to create the final prosthetic socket product. When fabrication of the socket is complete, the socket is typically tested on the patient for fit and for the patient's sense of how it feels and works. Although a few very minor modifications of the socket are possible at this stage, such modifications are very limited, and the shape of the socket at this stage is the key factor in determining how well the socket will fit the residual limb and, thus, how comfortable the patient will be when wearing the prosthetic. As just mentioned, the ability to modify the shape and fit of the socket after fabrication to better accommodate the residual limb is very limited. Accordingly, it is common practice to make a number of "check sockets" or "diagnostic sockets," from which the best option is chosen as the final product for the patient.

Various aspects of this conventional prosthetic fabrication process, which is practiced in a cottage industry of local prosthetic clinics and shops, are less than satisfactory. The central role played by physical molds in the fitting process and the transfer of size and shape information from the residual limb to the final prosthetic socket product is a limiting technological factor. The fabricating process itself can take weeks or even a month or more and it is an inexact process. And although the finished prosthetic socket product may often be quite satisfactory when produced by skilled prosthetists, it is still substantially fixed in form and cannot be easily modified (or in some cases modified at all). The residual limb itself, however, is not fixed in form. In fact, the residual limb often changes shape and condition radically, both in the short term and the long term. First of all, even if a prosthetic socket seems to fit perfectly in a prosthetist's office, the socket may rub or place pressure on the patient's residual limb when the limb is used repeatedly over days and weeks. Additionally, patients often lose or gain weight rather quickly as a result of their amputations, thus causing the residual limb to grow or shrink. Similarly, as patients use their residual limbs with their prosthetic devices, they may build muscle and/or portions of the residual limb may change shape due to stresses placed on it during use. Finally, as the patient ages, the residual limb will continue to change, in response to continued use and environmental conditions. Using currently available techniques for making prosthetic sockets, any time a significant change is needed in a socket for a patient, the only solution is to start the process again from step one and make a brand new socket.

Although improvements in prosthetic socket technology have been made, currently available prosthetic sockets still remain substantially fixed in shape and circumferential dimensions, particularly at their distal ends. Additionally, the manufacturing process for prosthetic sockets continues to be labor intensive, time consuming and requiring of highly skilled prosthetists. Due to all the shortcomings of conventional prosthetic sockets and their manufacture, it would be desirable to have new prosthetic socket devices and systems as well as new methods for making them. Ideally, such prosthetic sockets and manufacturing methods would lend themselves to modern manufacturing techniques and would help provide improved quality control and ability to scale production. Also welcome would be prosthetic sockets that could be manufactured in large quantities while still being highly customizable for each individual patient. It would also be advantageous to have a method of prosthetic socket manufacture that significantly shortened the average time required to deliver a finished, individualized prosthetic socket to a patient, compared to current techniques. Finally, it would be ideal to have prosthetic sockets that could be easily adjusted in order to handle changes in residual limb size and shape over time, including both short and long term changes. At least some of these objectives will be met by the embodiments described herein.

US2006/020349 discloses immediate postoperative prosthesis.

Aspects of the invention are recited in the independent claim; and preferred features are recited in the dependent claims.

### BRIEF SUMMARY

A first structural aspect of the technology and its associated embodiments provide a modular prosthetic socket assembly for a residual limb of a lower extremity as set out in claim 1. Various embodiments include assembleable components selected from various groups of components, such as but not limited to (1) struts including a thermoplastic-fiber composite material, the struts alignable with a central longitudinal axis of the socket assembly, and (2) socket bases configured to be arranged at the distal end of the socket assembly. Each strut has a proximal end and a distal end, the distal end being configured to be connectable to a base. At least one of the two assembleable component groups includes multiple sizes and/or shapes of the respective assembleable component. Thus, the modular prosthetic socket, when assembled, includes multiple struts and a distal base.

In other examples of the technology, as summarized below, the component groups may take the form of an inventory of components, either an actual physical inventory, or a virtual or catalogue-based inventory. In other embodiments, each of the enumerated prosthetic socket components may be modular, in that they include components of multiple sizes or shapes. In still other embodiments, structural components other than the two groups enumerated in this aspect of the technology may also be modular in this same sense.

A number of features may be further included in this first aspect of a modular prosthetic socket, and structural elements within the assembly may include optional forms and variations, as will be enumerated below. Although these features and structural variations are being summarized in the context of this particular aspect or embodiment of the technology. Further, the scope of this particular first embodiment includes any aspect or feature of all other embodiments of the technology described herein.

Structural embodiments of the technology may be referred to as a prosthetic socket, a modular prosthetic socket, or a prosthetic socket assembly. "Modular" generally refers to the modular aspects of assembly components, in other words the concept that the components can be drawn from groups of components that vary in size and/or shape, but nevertheless have connecting features that remain common. "Assembly" generally refers to aspects of the structure associated with being assembled from discrete components. All fully assembled structural embodiments, by virtue of being socket-shaped, circumscribe a space or cavity that is open at its proximal end, closed or substantially closed at its distal end, and has dimensions and shape. That space is generally circumscribed or defined by a distal base and longitudinal struts. One or more pressure distributing elements or components (or "interfacing elements or components"), such as but not limited to one or more proximal brim members, inner liners and/or strut caps, may further contribute to defining the dimensions of the circumscribed space.

The technology may further take the form of one or more individual components of a prosthetic socket, rather than the assembled socket as a whole. For example, a stand-alone strut represents an embodiment of the technology. Still further embodiments include systems, inventories, or kits, from which a modular prosthetic socket may be assembled. Various aspects of assembled prosthetic socket embodiments include (1) a prosthetic socket that does not have an integral circumferential structure in its proximal portion, (2) a residual limb-stabilized foundational base for distal prosthetic elements, and (3) a connecting structure that functionally connects a residual limb to distal prosthetic elements.

Useful in connection with the present invention are methods of forming modular components, methods of reforming modular components, methods of assembling a prosthetic socket, methods of replacing prosthetic socket components, methods of delivering a complete and fitted prosthetic socket within a short period of time, and methods of adjusting the assembled socket, either by mechanical adjustments or by controllable exercising inwardly directed tension. As above, any step described in the context of one type of method may also be wholly relevant and applicable to any other described method embodiment.

Structural aspects, embodiments, and examples of the provided technology are suitable for application to the providing a modular prosthetic socket for a residual limb that remains after an amputation. Examples described and depicted herein, when specific, generally refer to an amputation of a lower limb, and more particularly to a transfemoral (above knee) amputation or a knee-disarticulation (through the knee) amputation. However, embodiments of the technology are also suitable for providing a modular prosthetic socket for leg amputations that occur below the knee, and for amputations of an arm, as they may occur above the elbow, at the elbow, and below the elbow.

As mentioned above, the prosthetic socket assembly embodiments described herein generally includes multiple longitudinal struts and a base connected to the multiple struts. In particular embodiments, the prosthetic socket assembly has three struts or four struts. Regarding modularity of the components, whereby such components are members of a group of closely related components that differ in terms of size and/or shape yet remain connectable, in some embodiments the enumerated assembleable component groups are modular. Accordingly, in typical prosthetic socket embodiments, the longitudinal struts and the distal bases are mutually connectable even as the longitudinal struts and the distal bases each are drawn from groups of components that have multiple sizes and/or shapes.

When assembled, the prosthetic socket assembly circumscribes an internal space that accommodates and grasps the residual limb. The assembleable components are selected such that, when assembled together into a complete socket embodiment, the circumscribed internal space is substantially complementary to the residual limb of the patient. Complementary, in this sense, generally references the three dimensional aspects of the residual limb as a positive object, and the complementary "negative" three dimensional space circumscribed by the distal portion of the prosthetic socket.

Although various embodiments of the prosthetic sockets described herein include multiple, discrete components that are connected together, other embodiments may include two or more "components" that are actually formed together as one integral piece. For example, some embodiments include separate struts, strut connectors and a base, while other embodiments may include a discrete base, but struts that have integrated strut connection on their distal ends for connecting to the base.

The struts of the prosthetic sockets described herein are generally integrally separate from each other. The distal ends or portions of the multiple longitudinal struts of a socket are connected to the distal base. The base is generally radially incompressible. Once the struts are connected to the base, therefore, the distal-most portion of the struts, taken collectively, is substantially incompressible. In many embodiments, this is the only site along the length of the struts that is radially incompressible by a connecting structure.

By way of further clarification, the struts, collectively, in their proximal region do resist radially inward compression, but that resistance comes from (1) the integrity of the attachment of the struts to the distal base, and (2) to the strength of struts, which is substantially attributable to their thermoplastic-fiber composite material composition. By way of still further clarification, the resistance to radial compression in the proximal portion of the struts can be advantageously overcome by tensioning elements, as disclosed elsewhere herein.

In addition to characterizing the proximal portion of the struts (collectively, as the proximal portion of a socket) as compressible through the action of a tensioning mechanism, the proximal portion of the struts may also be expandable or distensible in response to applied force. Such expansive force may be applied, for example, by the residual limb when the socket is being worn by a patient. Such expansion is also resisted by the same aspects of the struts that resist inward compression, *i.e*., the integrity of the attachment of the struts to the base and the strength of the struts as it extends through the length of each strut.

Embodiments of struts applicable to the modular prosthetic socket assembly may include or be substantially formed from thermoplastic-fiber composite materials. Some embodiments may include a polymer matrix of polypropylene, polyethylene terephthalate (PET), acrylic, and/or polymethylmethacrylate (PMMA). In some embodiments, the thermoplastic material of the struts may include a fiber embedded within a polymer matrix, and the fiber may be formed from carbon, glass, or any other suitable material. These and other aspects of the composition of strut embodiments are described further below.

Such materials enumerated in the context of strut embodiments may further be included in any prosthetic socket component described herein, including distal base embodiments, pressure-distributing elements, such as proximal brim components, strut caps or flexible inner liners, as described further below. Any prosthetic socket components described herein may also include thermoplastic materials, not necessarily reinforced with fiber. In one example, any of the one or more pressure-distributing elements or the one or more distal socket members may include ethylenevinylacetate (EVA).

The advantages associated with thermoplastics and thermoplastic-fiber composite materials notwithstanding, many advantages associated with the modular and structural aspects of the provided technology remain, even absent the advantages of these particular materials. Accordingly, the scope of the disclosed technology includes components that include or are fabricated from thermoset plastics or resins, curable resins, and two-part resins. Additionally, as may be appropriate in parts of the world where access to advanced materials and processes is limited, components that include metals, aluminum, for example, or natural high strength products such as bamboo may be suitable for fabricating modular components. Thus, modular prosthetic components fabricated from any suitable material are included in the scope of the provided technology, so long as the components are consistent with the modular prosthetic structures disclosed herein and the demands placed on them.

The multiple longitudinal struts of a modular prosthetic socket may be arranged in numerous ways in various embodiments. In typical embodiments, the multiple struts are arranged circumferentially around the central longitudinal axis of the prosthetic socket. In this context, radial refers to the relative distribution of the struts within a 360° circumferential range. Individual struts may also vary in their width, such that the maximal portion of a circumferential arc occupied by each strut may vary among struts. Within the 360° circumferential range, the struts may be radially spaced apart at equivalent angles or at non-equivalent angles.

In some embodiments of the modular prosthetic socket, struts are connected to a distal base by way of strut connectors, the strut connectors occupying an intermediary position between a strut and a site on a distal base embodiment, and connected to both.

In some embodiments, each of the multiple longitudinal struts is connected to a strut connector, the multiple strut connectors being disposed on the distal base at fixed circumferential positions relative to each other. Accordingly, the disposition of the strut connectors around the distal base is determinative of the distribution of the struts. The distribution of the struts circumferentially around the distal base, in turn, is projected into the distribution of the struts as they project proximally from the base. In one example, four strut connectors are disposed on the distal base, the connectors distributed circumferentially around the base at equivalent intervals of 90 degrees. Similarly, three strut connectors (and three struts) may be circumferentially distributed at intervals of 120 degrees.

In another example, four strut connectors are disposed on the distal base, the connectors spaced apart by four intervening angles around the base, such that one of the four angles is greater than 90 degrees. By way of a particular example, the four strut connectors may be separated by consecutive angles of about 90 degrees, about 60 degrees, about 90 degrees, and about 120 degrees. This particular embodiment of prosthetic socket assembly (wherein one intervening angle between struts is greater than 90 degrees) is typically configured such that, when worn, the larger angle accords with the radial portion of the socket that is worn on an inner or medial aspect of the residual lower limb.

In an alternative expression of these angular relationships among the struts, the struts define inter-strut open spaces or sectors that occupy varying degrees of an arc. In some embodiments, these arcs are equivalent, such as four arcs of 90 degrees. In other embodiments, these open space sectors are not equivalent. In some embodiments, one open space sector is larger than all others. In one example of such embodiment, one open space sector occupies about 120 of an arc.

In some embodiments of the struts and their connectors to a distal base, strut connectors, albeit at fixed circumferential positions on the distal base relative to each other, are angularly pivotable at that fixed position. And accordingly, a circumferential position of a strut varies according to the circumferential position of the strut connector.

In still other embodiments, the struts are connected to the distal base by way of a mechanism that provides circumferential mobility of the struts within an arc around the base. In such embodiments, the struts and the circumferentially mobile mechanism are cooperatively operable to lock the struts at particular circumferential positions.

Some embodiments of the modular prosthetic socket assembly may further include a proximally disposed intra-strut connector, wherein at least two of the struts are held at a fixed radial distance from each other.

Returning to the embodiments of the modular prosthetic socket wherein the radial position of struts with respect to the center of the distal base, it can be appreciated that varying or adjusting the radial position has implications for the internal space created by the struts as they project proximally. Accordingly, in some embodiments, wherein the multiple struts together nominally circumscribe an internal volume bounded by the proximal and distal ends of the struts, the internal volume is adjustable by adjusting a radial position of the struts at their distal end relative to the distal base. The relationship, in general, is such that radially outward movement of the radial position of any one or more of the struts (or the strut connectors) increases the volume circumscribed by the struts as a whole. Similarly, moving any one or more of the struts (or strut connectors) radially inward decreases the volume circumscribed by the struts as a whole. Stated in another way, changing the area of a circle, or the approximately circular area, as nominally described by the struts as they are radially arranged on the base is reflected by a similar change in the volume circumscribed by the struts as a whole. Further aspects and functionalities of strut connectors are described in a section that follows below.

The size and shape of the internal volume circumscribed by embodiments of the modular prosthetic socket is highly individualizable for patients, including those who have residual limbs that may be difficult to fit with prior art sockets. In one example of a residual limb that can be challenging to fit is a bulbous-shaped residual limb. It is difficult for many prior art prosthetic sockets to fit or even to be put on a bulbous residual limb; this is particularly true for a prior art device the that cannot flex outwardly, or one that has an integral portion that is circumferentially continuous. In contrast, embodiments of modular prosthetic sockets, as described herein, can easily flex outward when being donned by a patient, and they typically have no integral proximal circumferential continuous portion that could impede entry of a residual limb. Accordingly, in one embodiment of a modular prosthetic socket, the multiple struts together nominally circumscribe an internal volume having a proximal portion and distal portion, and wherein the volume is bulbous such that it has a diameter greater in the distal portion than in the proximal portion.

In embodiments of the modular prosthetic socket described herein, the multiple struts are connected or connectable to the distal base of the socket in various arrangements, by various mechanisms, and having various functionalities with respect to the distal base. The struts are connected to the distal base at strut connecting sites, specific locations having particular features, located on the distal base. Embodiments of the distal base may have one or more cooperating plates, and accordingly, the strut connecting sites may involve one or more of the cooperating plates. The strut connecting site typically is located on a plate surface with a proximal exposure, whether or not the involved distal plate is the uppermost (proximal-most) plate or not; in some embodiments, a top plate includes open space that leaves an underlying plate with proximal exposure. This site, with a proximally exposed surface, typically includes features such as holes and slots. In some embodiments, a strut connector element intervenes or mediates between the distal ends of the strut and the distal base. The strut connector mates with the strut connecting sites on the base, the strut connect having holes that align with holes in the involved distal plate and/or having features that insert in slots. These various structural relationships between the distal plate and the strut connector underlie mechanisms of connection, movement, and lockability between the distal base and the strut connectors, and ultimately between the distal base and the struts.

As referenced above in the context of describing a first embodiment, one of the components of the prosthetic socket assembly that may be modular is a distal base to which the struts attach. In particular embodiments, the distal base may include one or more cooperating plates, a plate among of the cooperating plates having an exposed proximal surface having one or more strut connecting sites. In some embodiments of the prosthetic socket assembly, one or more cooperating plates are modular in character, in that plates within the distal base are selected from a group of plates, each group respectively having plates of multiple sizes or configurations. By way of example, a group or inventory of plates may include plates with maximum diameters of three sizes, for example, 6.99 cm (2.75 inches), 8.89 cm (3.5 inches) and 11.43 (4.5 inches). By way of another example, a top plate may include three or four sites for mounting a strut, typically configured as a strut connector site.

In some embodiments of modular prosthetic socket assembly of claim, the cooperating plates, collectively, include connections to a pressure distributing element of the assembly and distal prosthetic elements. By way of example, a pressure-distributing element may include any of a proximal brim, a strut cap, or a flexible inner liner, as described elsewhere herein. Further still, the cooperating plates may include connections to a prosthetic socket liner, as, for example, a liner adapted to manage moisture, or connections to ancillary moisture removal elements. Elements such as these may not be particularly adapted to distribute impinging pressure. Distal prosthetic elements, by way of example, may include a prosthetic limb or an aligning element disposed between the distal base and the prosthetic limb.

In some embodiments of the distal base of a prosthetic socket assembly, the strut connecting sites take the form of strut connector mounting sites. Particulars of strut connectors are described further below. With regard to the mounting sites, embodiments of a mounting site typically include at least one radially oriented slot configured to accommodate a strut connector. By way of example, particular embodiments may include one radially oriented slot, or two parallel radial slots. In such embodiments, the strut connector is configured to engage the at least one radially oriented slot in a manner such that the strut connector is slidable and swivelable within the slot. In various embodiments, the radial slot allows a variation of up to about 44% in the relative position of the strut connector from the center of the base, and allows a rotational swivel of up to 25 degrees on either side of a neutral position along a radial line. Depending on embodiment configurations, the rotational swivel of a strut connector with respect to the base may also be characterized as a pivoting action.

In some embodiments, the distal base includes two cooperating plates, a distal base plate and a partially overlying proximal plate. In such embodiments, the strut connecting site can include peripheral indentations of the proximal cooperating plate attached proximally to a distal base plate, the distal base plate having radially oriented slots that each slidably accommodate a strut connector, and the proximal plate having peripheral indentations that confine a pivotable range of the strut connectors.

Turning now to embodiments of a strut connector, in some embodiments, the strut connector may be an integrated aspect of a strut. For example, a modular prosthetic assembly may include one or more strut connectors, such strut connector including a distal aspect of a strut that is adapted and configured to connect to a strut connecting site on one or more of the cooperating plates included within the distal base.

In other embodiments, a strut connector may be a discrete element that physically and functionally intervenes in the connection of a strut to distal base. Accordingly, in some embodiments of a prosthetic socket assembly, each strut connector is configured to connect a strut to the distal base at a strut connector site. Typically, each strut connector includes a horizontal base portion connectable to the distal base of the prosthetic socket and a back takeoff portion connectable to a strut. In some embodiments, the strut has a width with two lateral aspects on either side of the width, the strut connector further having a buttress portion connecting the lateral aspects of the base portion and the horizontal base portion.

In some embodiments of a strut connector element, the base portion of the strut connector has a bottom surface to be aligned against a top surface of a plate of the distal base. The strut connector is typically mobile, or can be mobilized, with respect to the top surface of the plate, and the strut connector is adapted and configured to be pressable against the top surface of the plate. When sufficiently so pressed, per the action of a fastening mechanism, the strut connector becomes immobile against the surface. Typically, such immobility or locking relationship occurs by frictional engagement of the two substantially smooth surfaces. In some embodiments, surface features on either the strut connector or base surface may enhance the friction-based immobility.

With particular regard to the configuration of takeoff portion of the strut connector, it may be angled with respect to the horizontal base portion, such angle ranging between about 90 degrees and about 150 degrees from the horizontal. By way of an alternative perspective of the same structural angulation, the takeoff portion of the strut connector includes an angle relative to a central longitudinal axis of the modular prosthetic socket assembly, such angle may range between 0 degrees and about 45 degrees from the central longitudinal axis.

The takeoff angle of the strut connector has implications regarding the strut attached to the strut connector, Accordingly, the takeoff angle of the takeoff portion of the strut connecter relative to the central longitudinal axis of the prosthetic socket assembly determines the angle of the distal-most portion of the strut connected to the strut connector. Of course, the strut may assume or include other angles proximal from the base.

With regard to aspects of the connection of the strut connector to the distal base of the socket, in some embodiments, the base section of the strut connector includes at least one hole alignable with at least one hole within of a distal plate. These holes are mutually arranged and configured to accommodate at least one fastening element that can be variably tightened. The arrangement of holes and fastening elements serves to secure the strut connector to the distal base, further allowing mobility and lockability. Accordingly, when the fastening element is not tight, the strut connector is unlocked with respect to the base and thereby radially slidable and free to rotate within an arc. When the fastening element is tight, the strut connector is immobilized with respect to the base.

As summarized in a preceding section, some embodiments of the modular prosthetic socket assembly include multiple connecting sites on the distal base; in some embodiments the connection includes intervening strut connectors configured to accept a distal end of a strut. The multiple strut connectors are typically connectable or connected to the distal base, the multiple strut connectors thereby mediating the connection of each strut to the distal base. Strut connectors, typically on their takeoff portion, further include attachment sites for the distal end of a strut. Embodiments of the strut connectors have a number of functional aspects, and such functionalities have implications for the function and operability of the modular prosthetic socket as a whole.

Accordingly, typical embodiments of a modular prosthetic assembly include multiple strut connecting sites on the distal base configured to accept a distal end of a strut. Such strut connecting sites may be adapted and configured to provide the strut slidability along a radial line, and swivelability from any point on the radial line. In such embodiments where the multiple struts are connected to the distal base by way of strut connectors, said connectors may be radially adjustable such that the strut may be lockably positioned at a minimal radial position, a maximal radial position, and at any position therebetween. The radial position in this context refers to the distance from the center point of the distal base plate, or from central longitudinal axis of the prosthetic socket. Merely by way of example, a base plate, substantially circular, may have a radius of 5.72 cm (2.25 inches), and a strut connector may have a slidable range along a radial path between a minimal radial position and a maximal radial position of 1 inch. Thus, in this example, the variability in radius represents about 44% of the maximal radius.

In particular embodiments of the modular prosthetic assembly, the radial position of the strut connectors collectively determines a nominal cross sectional area circumscribed by the struts collectively, such cross sectional area of the struts being taken as a plane orthogonal to the central longitudinal axis of the prosthetic socket assembly along the length of the struts. Further, the radial position of the strut connectors collectively can determine a nominal volume circumscribed by the struts collectively, the volume being longitudinally bounded by the proximal and distal ends of the struts.

In particular embodiments of the modular prosthetic assembly, the multiple struts are connected to the distal base by way of strut connectors that are adjustably pivotable within an arc coplanar with the base plate, the arc centered on a radial line. In one example, the strut connectors are pivotable within an arc ranging between about +25 angular degrees from the radial line. In a second example, the strut connectors are pivotable within an arc ranging between about + 15 angular degrees from the radial line. Further still and accordingly, the multiple struts, by being connected to the adjustably pivotable strut connectors, are pivotably adjustable within an arc in a plane transverse to the longitudinal axis of the prosthetic assembly.

Configuring and reconfiguring a prosthetic socket may be provided by exploiting structural options provided by the technology even within a given set of components. By way of contrast, even absent the adjustable capabilities provided by tensioning, the configuration of a prosthetic socket is not wholly or fixedly determined by the selection of components. By way of example, as provided by some embodiments, the positioning of struts with respect to a distal base can be changed by adjusting the radial position of the struts from the center of the base, or by rotationally pivoting the strut from a given radial position such that its circumferential location relative to other struts can be changed.

These adjustments that are made with a given set of prosthetic socket components are generally smaller than the variation that can be produced by using components of entirely different size or shape as may be found in an inventory of components. These latter types of prosthetic socket size or shape variation typically represent choices made when selecting components for different patients. The adjustments that can be made in socket configuration with a given set of components are typically performed by a prosthetist working with a patient on his own prosthetic socket. Although these configurational changes made with a given socket are relatively small compared to the range of options that can be exercised when selecting modular components of different size or shape, these comparatively minor changes can be extremely important for that patient. As discussed below in the detailed description, socket functionality is highly dependent on fit of the socket to the residual limb. Inasmuch as the size and shape of a residual limb can change over time, and sites of skin irritation or muscle soreness can change over time, these configuration adjustments within the range of options available for a given socket can accommodate these changes, maintaining fit and functionality of the socket.

In yet another structural option available to the assembly of a modular prosthetic socket, the "takeoff' angle that characterizes the angle of the distal end of a strut with respect to the central longitudinal axis of the prosthetic socket can be a variable. Accordingly, in some modular prosthetic socket embodiments, at the site of attachment to the distal base, the multiple struts are connected thereto in an arrangement that provides for variable angles of deviation from the longitudinal axis of the prosthetic socket assembly. Further, in some of such embodiments, the angle of deviation from the longitudinal axis of the prosthetic socket assembly for each strut can be varied independently of the other strut(s). In any of these embodiments, any of the multiple struts may be connected to the distal base by way of an intermediary strut connector, variations in the structure of the strut connector accounting for the variation and options in the takeoff angle.

Some embodiments of the longitudinal struts included modular prosthetic socket assembly embodiments will now be summarized.

Such socket embodiments may be characterized in terms of total strut length, a projected strut length, a distal curvature angle, a maximal radius from the central longitudinal axis when assembled into a prosthetic socket, and a cross sectional area. The cross sectional area of the strut, for example, can vary within the length of the strut. Inasmuch as the struts may be included with a group of struts that serve a modular assembly, a group of struts typically includes struts that vary in their total strut length or in their projected strut length. Strut embodiments may include one or more angles within a longitudinal plane. Struts may include, by way of example, a distal curvature angle, and accordingly a group of struts may include struts that vary in their distal curvature angle. Similarly, struts may be characterized in terms of their maximal radial width from the central longitudinal axis of the prosthetic socket when assembled thereinto, and a group of struts may include struts that vary in their maximal radial width. Further, strut embodiments can vary in their maximal cross sectional area, and a group of struts may include struts that vary in their maximal cross sectional area.

The struts of embodiments of a modular prosthetic socket assembly may be understood to collectively circumscribe an internal space with a distal, a proximal end, and a circular profile. A cross section of that internal space, taken at a midpoint between the distal and proximal ends defines a cross sectional area with a circumference. In various embodiments, each strut occupies an arc no greater than about 25 angular degrees of that circumference. In particular embodiments, that arc is no greater than about 15 angular degrees. Prosthetic socket embodiments may also be characterized in terms of the additive or collective width of all struts within the midpoint circumference. In some embodiments, the additive combined minimal width of all the struts sums to an arc no greater than 100 degrees.

Just as struts within a group, collection, or inventory of struts may vary among each other with regard to size and shape, so too may struts among those included within a given modular prosthetic socket. Such variation may arise from the selection of struts that vary; variation may also arise during a reforming process of one or more the struts. For example, two strut within a set of four selected for a given socket may be identical in size and shape, as drawn from an inventory. However, due to changes in curvature made by a prosthetist on one of the struts, changes made so as to improve the overall fit of a socket to a patient's residual limb, the two struts, originally identical, may be different in shape. In addition to differences in shape in terms of curvature, struts within a group included within a given socket may also vary in terms of any of width, thickness, or length.

In some embodiments of a modular prosthetic socket assembly, at least one of the multiple struts may include a centrally disposed metal core in the distal portion of the strut, the metal core projecting distally from the thermoplastic-fiber composite composition portion of the strut, the metal core having a length and cross sectional area. Such metal core may be included as part of an attachment mechanism, whereby the strut is attached directly to a distal component, such as a distal base. In other prosthetic embodiments, as summarized above, a strut connector (described elsewhere herein) may be alternatively or additionally involved in the attachment of a strut to a distal component.

Embodiments of a modular prosthetic socket may also be characterized in terms of the cross sectional area they circumscribe, and further, such area may be compared to the cross sectional area of a residual limb. Accordingly, a residual limb is characterizable in terms of dimension and shape such that there is a cross sectional area at each point along the length of the residual limb where the limb is to be contacted by the prosthetic socket assembly when being worn. The strut embodiments within a socket may collectively and nominally describe a circle having a cross sectional area at each point along the length of the struts. At any point along the length of the socket, the struts have a neutral radial position absent any inward or outward impinging force. In some embodiments of the prosthetic socket, as assembled from strut embodiments, the area of a circle described by the struts in the neutral position along the length of the struts is not less than 75% of the cross sectional area of the residual limb at the corresponding point along the length of the struts. In some particular embodiments, the surface area of a circle defined by the struts in the neutral position along the length of the struts is not less than the cross sectional area of the residual limb at the corresponding point along the length of the struts.

In some embodiments of the modular prosthetic socket assembly, the struts are particularly arranged, configured, and of appropriate strength relative to the weight of the patient such that upon the patient taking strides, the struts flex under a stride impact of a stride, store kinetic energy in a flexed position, and release kinetic energy upon termination of stride impact. Factors included in the strut having an appropriate amount of strength and being appropriately arranged and configured include: the cross sectional area and cross sectional profile of the strut, the specifications of both the thermoplastic and the embedded fiber, and particulars of angulation within a strut, and the arrangement of the struts collectively with respect to each strut individually.

A second example of background information is embodiments which provide a modular prosthetic socket assembly for a residual limb of a lower extremity that includes a basic three-component structure. Accordingly, this embodiment of a modular prosthetic socket assembly for a residual limb of a lower extremity of a patient includes assembleable components selected from various component groups that include (1) struts including a thermoplastic-fiber composite material, the struts alignable with a central longitudinal axis of the socket assembly, (2) distal socket bases configured to be arranged at the distal end of the socket assembly, and (3) pressure-distributing elements, each pressure-distributing element configured to be supported by the multiple struts. These pressure-distributing elements may alternatively be referred to as a pressure-distributing surface or a pressure-distributing structure. In some aspects, the pressure-distributing element serves as an interfacing element, interfacing between hard structural components of the socket and the body of the patient. In various embodiments, the pressure-distributing structure may further or alternatively have features (or support such features) that functional to pad the struts, to absorb shock, to cover hard edges of struts, and to serve as a tensioning anchor for various tensioning systems.

The pressure being referred to is the pressure that the main structural components of the socket visit on the residual limb as the patient bears weight on the limb, as typically in the practice of these embodiments, a residual lower limb. In this clinical example, the residual lower limb would be the recipient of pressure being exerted by the combination of the struts and distal base. In the absence of an intervening pressure-distributing structure, the entirety of body weight could be visited on the residual limb along the sites of contact between the socket and the residual limb. Pressure-distributing structures, as disclosed herein, distribute that weight away from the profile of the contact sites on the residual limb, and more broadly onto the surface of the residual limb. This is a generally desirable feature of prosthetic sockets, although in some clinical instances and device embodiments, it also may be desirable to maintain some preponderance of the totality of pressure within the relatively narrow profile of the struts and distal base.

Each longitudinal strut has a proximal portion and a distal end, the distal end being configured to be connectable to a distal base. The assembleable component groups include at least one group that has multiple sizes or shapes of the respective assembleable component. A fully assembled socket includes multiple struts, a distal base, and at least one pressure-distributing element. The prosthetic socket assembly circumscribes a distally open internal space; the assembleable components are selected such that, when assembled, the circumscribed internal space is substantially complementary to the residual limb of the patient.

In some examples of this embodiment of a modular prosthetic socket assembly, each of the assembleable component groups includes at least one of multiple sizes or shapes of the respective assembleable component. Further, in some embodiments, the at least one pressure-distributing element and the multiple struts are formed as an integral structure. In other embodiments, the multiple struts and the distal base may be formed as an integral structure. In still further embodiments, all structural components may be formed integrally.

In some examples of this embodiment, the one or more pressure-distributing elements are adapted to accommodate an inwardly directed tensioning mechanism. In particular embodiments, the inwardly directed tensioning mechanism is arranged external to the struts, such that inwardly directed tension is transmitted substantially by way of the struts. The general purpose of an inward tensioning of the struts, collectively, is to provide the socket, as a whole, an ability to assertively grasp the residual limb. In typical embodiments, the struts of an appropriately fitted socket, absent tensioning, provide a minimal, if any substantial amount of inward pressure. This may be understood generally as a neutral position of the struts, collectively. As provided by tensioning elements, the inwardly applied tension becomes a controllable aspect of the modular prosthetic socket.

In some examples of this embodiment, the one or more pressure-distributing elements are arranged and configured, and of a suitable composition to absorb or bear pressure directed inwardly from any of the struts or the distal base, and to distribute such pressure over an area of the one or more pressure-distributing elements larger than an area of contact of the struts or the distal base against the pressure-distributing element. In particular embodiments, the one or more pressure-distributing elements and the struts, collectively, are arranged and configured to absorb substantially all pressure that would otherwise be directed toward the distal end of the residual limb by the distal base.

In some embodiments of this modular prosthetic assembly, the pressure-distributing element may include or be formed form any a polymer or a soft good material. By way of example, a polymer may include any of ethylenevinylacetate (EVA), low density polyethylene (LDPE), or a blend or a copolymer thereof. Pressure-distributing elements that include a polymer typically have sufficient strength and resilience that the element bears pressure well without compromising the form of the element. Such embodiments may be able to distribute pressure impinging from struts with substantial uniformity across the surface of the element. In embodiments that include a soft good material, such soft goods may, way of non-limiting example, include any of a fabric or a leather. Such fabric, may include any of a knit fabric or a woven fabric, or any suitable fabric, and may include additives or impregnated materials that add desirable properties to the fabric. Pressure-distributing elements that include a soft good material such as a fabric typically conform to an underlying support when absorbing external pressure, and do not, by themselves, distribute pressure substantially away from an impinging pressure source.

In some embodiments of this example of a modular prosthetic socket assembly, the one or more pressure-distributing elements may include at least one proximal brim member; typically such a brim is connectable to at least one of the multiple struts of the socket. Proximal brims typically attach to one or more the struts, at proximal portion of end of the struts.

Further, and typically, the at least one proximal brim member interfaces between the proximal portion of the multiple struts and a proximal portion of the residual limb when the prosthetic socket assembly is being worn by a patient. The proximal brim or brim arrangement extends proximally from the proximal ends of the struts, and engages the body. Such engagement may extend to a point beyond the nominal range of the limb itself, as for example, a brim may engage the part of the body proximate the ischial aspect of the pelvis.

Further, and typically, the longitudinal struts and the at least one proximal brim member are mutually connectable even as the longitudinal struts and the at least one proximal brim member each may include components of multiple sizes or shapes. As noted above, any component of the prosthetic socket may be modular in this sense. In some embodiments of this example of a modular prosthetic socket assembly, the multiple struts and the at least one proximal brim members are formed as an integral structure.

In some embodiments, the at least one proximal brim members include or incorporate an integrated circumferential tensioning mechanism. Typically in such embodiments, the circumferential tensioning mechanism is arranged to apply inwardly directed tension from a position external to or proximate the struts. In particular embodiments, the at least one brim member includes laterally extending tabs.

In some embodiments of this example of a modular prosthetic socket assembly, the one or more pressure-distributing elements includes a flexible inner liner sized and configured to be disposed internal to the multiple struts and external to the residual limb when the prosthetic socket assembly is being worn by the patient. In particular examples of this embodiment, the flexible inner liner is of sufficient stiffness and resilience that it can support distribution of pressure with substantial uniformity across its surface.

In some embodiments of this example of a modular prosthetic socket assembly, the one or more pressure-distributing elements include a strut cap adapted and configured to be disposed at the proximal end of the struts. Typically, the strut cap encloses or surrounds the tip of the strut. In some embodiments of the modular prosthetic socket assembly, one or more of the multiple struts may be capped by a strut cap. And in some embodiments, the strut caps have lateral elements that connect the strut caps together.

In some embodiments of this example of a modular prosthetic socket assembly, the assembleable components further includes strut sleeves, each strut sleeve being configured to fit over a strut, the completely assembled prosthetic socket including a strut sleeve fitted over a strut. In various embodiments, it is not necessarily the case that all struts are fitted with a sleeve. In particular embodiments, the strut sleeves include sites for tensioning members, such members being attachable to any of other strut sleeves or a directly to a pressure-distributing element. Strut sleeves can thus generally be directed to the functioning of a tensioning system, but they may also serve as padding, or as a support for padding.

The scope of this second aspect and associated embodiments of the technology may include any aspect or feature of other embodiments of the technology described herein.

A third example of background information is embodiments which provide a modular prosthetic socket assembly for a residual limb of a lower extremity that includes a basic three-component structure. A first component includes multiple longitudinal struts formed from a thermoplastic-fiber composite material; a second component is at least one pressure-distributing element such as a proximal brim, strut cap, or flexible internal liner supported by the struts; and a third component is a distal socket base arranged distal to the struts and connected thereto. As may be understood that the residual limb of the patient has an individual dimensionality and shape. When assembled, the prosthetic socket circumscribes an internal space that is substantially complementary to the dimensionality and shape of the residual limb of the patient. The scope of this third structural aspect of the technology may include any aspect or feature of other embodiments of the technology described herein.

A fourth example of background information is embodiments which provide a modular prosthetic socket assembly for a residual limb of a lower extremity that has an internal volume that is adjustable by varying the radial position of struts with respect to a base. This embodiment includes multiple longitudinal struts that include a thermoplastic-fiber composite material, each strut has a proximal portion and a distal portion; and a distal socket base arranged distal to the struts and connected to a distal portion of each strut, the base being particularly adapted and configured to support the struts. The assembled prosthetic socket circumscribes an internal space having a volume. The assembleable components are selected such that the circumscribed internal space conformally fits the residual limb of the patient. Further, the volume included within the internal space is adjustable by varying a radial site of attachment of the struts to the distal base. A "conformal" fit generally refers to the dimensions and shape of the internal space being a substantial complement to the dimensions and shape of the residual limb.

Accordingly, in some embodiments of the modular prosthetic socket circumscribes a space that accommodates and grasps the residual limb of the patient. This circumscribed internal space has a length aligned with a central longitudinal axis of the socket. The internal space has a cross sectional area throughout the length; per the features and functionalities of these embodiments, the cross sectional area is adjustable substantially throughout the length.

In some embodiments, the cross sectional area throughout the length is adjustable via mechanical options available during socket assembly. In one example, the available mechanical options available during socket assembly include a variable radial position for the strut with respect to the distal socket base. In particular examples of these embodiments, the available mechanical options available during socket assembly are also available if, once assembled, the prosthetic socket assembly is at least partially disassembled.

Inasmuch as the internal space circumscribed by the modular prosthetic socket has a cross sectional area associated with its length, it also has a volume. Accordingly, per embodiments as above, such volume is adjustable via mechanical options that can be exercised during socket assembly, or a later point, in a re-assembly process following an at least partial disassembly. The scope of this fourth structural aspect may include any aspect or feature of other embodiments of the technology described herein.

A fifth example of background information is embodiments which provide a modular prosthetic socket for a residual limb of a lower extremity that includes one or more tensioning mechanisms that can controllably vary the inwardly applied force applied by the socket to the residual limb. This aspect of the embodiment allows the patient or wearer of the prosthetic socket to adjust the fit of the socket to his or her preference.

Accordingly, this embodiment of a prosthetic socket includes a proximal socket portion having multiple longitudinal struts; the struts are formed from a thermoplastic-fiber composite material, and each strut has a proximal portion and a distal portion. The proximal portion of the socket further includes at least one tensioning mechanism arranged to controllably apply inwardly directed force to the proximal distal socket portion. A distal portion of the socket includes a distal socket base arranged distal to the struts and connected to a distal portion of each strut. The assembleable components of the modular prosthetic socket are selected such that the circumscribed internal space conformally fits the residual limb of the patient.

Some of these embodiments of a modular prosthetic socket include a tensioning mechanism that is operable to adjust the volume of the circumscribed internal space of the proximal portion of the socket. Similarly, when the prosthetic socket is being worn by the patient, the tensioning mechanism is operable to adjust the compressive force applied to the residual limb by the prosthetic socket.

With regard to the circumscribed internal space of these modular prosthetic socket embodiments, such space has a length aligned with a central longitudinal axis of the socket, and the tensioning mechanism is disposed so as to controllably apply a compressive force to a tensionable span of the length within the proximal portion of the socket. In some of these embodiments, the tensionable span of the length includes a portion of the length of the struts. In some embodiments, the tensionable span of the length includes a portion of the length of the brim.

In some embodiments of the modular prosthetic socket, the tensioning mechanism is arranged such that tensioning is applicable with substantial uniformity throughout the tensionable span of the length. In alternative embodiments, the tensionable span includes sections wherein the tensioning mechanism can operate independently. In some particular embodiments, the tensioning mechanism includes a pulley arrangement with a mechanical advantage.

Some embodiments of the modular prosthetic socket include multiple strut sleeves configured to at least partially enclose each strut, each strut sleeve having one or more tensioning mechanism attachments. And in some embodiments, the one or more brim members include tensioning mechanism attachments. The scope of this fifth structural aspect may include any aspect or feature of other embodiments of the technology described herein.

A sixth example of background information is embodiments which provide a modular prosthetic socket system for a residual limb of a lower extremity, the prosthetic socket being assembled from components selected from inventories of each respective component. In addition to a basic arrangement of three components as enumerated above (multiple struts and a distal base), the prosthetic socket system may include other components.

Accordingly, a modular prosthetic socket system for a residual limb of a lower extremity of a patient may include assembleable components having multiple longitudinal struts that include a thermoplastic-fiber composite material, at least one pressure-distributing element such as a proximal brim, strut cap, or flexible internal liner supported by the struts, and a distal socket base arranged distal to the struts, the base being particularly adapted and configured to support the struts. In this embodiment, the multiple struts and the at least one pressure-distributing element are mutually connectable, and the multiple struts are and the socket base are mutually connectable. Further in this embodiment, each of the assembleable components is selected from an inventory having at least one of multiple sizes or shapes of the respective assembleable component. The scope of this sixth structural aspect of the technology, beyond the aspects and features enumerated below, may include any aspect or feature of other embodiments of the technology described herein.

This aspect of the technology may include a heating sleeve configured to at least partially enclose a strut, the sleeve further configured to be able to deliver sufficient heat to the strut such that it becomes heat reformable.

This aspect of the technology may a distal end pad configured to be accommodatable within the distal base, and configured to conform to a distal end of the residual limb. Such a distal end pad may include any of a conformable gel or foam, or a composition amenable to heat molding or injection molding.

This aspect of the technology may include an informational guide to assembling the prosthetic socket. Such an information guide may include informational guidelines as to fitting the prosthetic socket, as assembled from modular components, such that the completed prosthetic assembly fits the patient.

A seventh example of background information is embodiments which provide a kit from which a modular prosthetic socket for a residual limb of a lower extremity can be assembled. Accordingly, this kit embodiment may include one or more assembleable components for each of three groups of assembleable components, the assembleable components including (1) multiple longitudinal struts including a thermoplastic-fiber composite material, and a (2) distal socket base configured to be arranged distal to the struts. The physical relationships or mutual connectedness of these components are such that embodiments of the multiple thermoplastic-fiber composite struts and the socket base are mutually connectable. Other prosthetic socket components may be included in the kit, and such components may include variations in form, such as variation in dimensions or shape. By way of example, other components may include proximal brim elements, flexible inner liners, strut caps, tensioning mechanisms, strut sleeves, and any other component described herein.

Strut embodiments within a kit, although being of varying size or shape, may nevertheless have a similar aspect, such as being substantially flat. In a sense, a flat shape is neutral, and usefully amenable to reshaping, as in an embodiment of a reforming method, whereby a thermoplastic-fiber composite material is heated to the point of pliability and then reshaped. Method embodiments such as these are described in detail below. With regard to the struts that may be included in an inventory, although they may commonly be flat or substantially flat, they may also include any shape that can be molded. A thermoplastic-fiber composite strut of any shape can be reshaped. Accordingly, by the nature of thermoplastic materials, with regard to any physical characteristic, other than the provenance of a thermoplastic-fiber composite thermoplastic strut, a given curved strut that is (1) in its initial formed shape or (2) a strut that was initially formed as a flat and subsequently reformed to include a curved aspect are substantially indistinguishable from each other.

Each of the assembleable components within the kit may be selected from an inventory having at least one of multiple sizes or shapes of the respective assembleable component. An inventory, such as an inventory of strut kit pieces, may include an actual physical embodiment in the form of pieces packaged or housed together, whether in a traveling case or remotely located in a warehouse. An inventory may also be represented as items listed in a printed catalog or as published online. Inasmuch as strut kit pieces typically are represented by standardized variations in dimension or shape, an inventory also may be represented by a series of component molds that vary in dimension or shape.

The modular prosthetic socket kit may further include informational materials, as for example, guide to assembling the prosthetic socket, or guidelines as to fitting the prosthetic socket, as assembled from modular components, such that the completed prosthetic assembly fits the patient. The scope of this seventh structural aspect may include any aspect or feature of other embodiments of the technology described herein.

An eighth example of background information is embodiments which provide a prosthetic socket assembly for a residual limb of a lower extremity that does not include an integral structural element in the proximal portion of the socket that forms a circumferential continuity. Expressed alternatively, the proximal portions of the longitudinal struts, collectively, do not have any integral circumferentially continuous structure. More particularly, a circumferentially continuous feature that is excluded from this embodiment is one that is substantially incompressible. Incompressibility, in this context, refers to a compression that would reduce the area of a cross section nominally defined by the struts. Expressed alternatively, an integral circumferentially continuous structure, as excluded in this embodiment, is one that would resist a centrally directed compression.

Accordingly, this embodiment of the technology includes multiple longitudinal struts, each strut having a proximal portion and a distal portion. The multiple longitudinal struts may or may not include a thermoplastic-fiber composite material The proximal portions of the longitudinal struts, collectively, do not have any integral structure that forms a circumferential continuity such that a nominal circumference described by a cross sectional profile of the struts is incompressible. Similarly, such excluded integral structure is also one that would be substantially non-extensible. The prosthetic socket assembly of this embodiment circumscribes an internal space that conformally fits the residual limb of the patient. The scope of this eighth structural aspect of the technology may include any aspect or feature of other embodiments of the technology described herein.

A ninth example of background information is embodiments which provide a proximal structural foundation for more distal prosthetic components. Accordingly, a foundational base for a distal prosthetic substitute for a lower extremity of a patient includes multiple longitudinal struts including a thermoplastic-fiber composite material, and a distal socket base arranged distal to the struts and connected thereto. The distal socket base may include a cup; the cup includes an interior aspect configured to accommodate a distal end of a residual limb of the patient. The distal base may also include a distally directed connection site configured to be connectable to the distal prosthetic substitute for the lower extremity. The foundation base circumscribes an internal space that conformally fits the residual limb of the patient. The scope of this ninth structural embodiment of the technology may include any aspect or feature of other embodiments of the technology described herein.

A tenth example of background information is embodiments which provide a connecting structure that functionally joins a residual limb with distal prosthetic elements. Accordingly, this embodiment includes multiple longitudinal struts including a thermoplastic-fiber composite material, a distal socket base arranged distal to the struts and connected thereto, the distal socket base including a cup configured to support the distal end of the residual limb. The connecting structure embodiment has an interior proximally open aspect configured to embrace or grasp a distal end of a residual limb of the patient. The connecting structure may further include a distally directed site, exterior and opposite to the proximally open space, the distally-directed site configured and adapted to connect to distal prosthetic elements that replace or substitute for the residual limb.

The connecting structure embodiment may be arranged or configured to accommodate either a residual lower limb or a residual upper limb, either limb at any level of amputation. Accordingly, a connecting structure embodiment may be one wherein the distal prosthetic component includes a prosthetic lower limb or portion thereof, or it may be one wherein the distal prosthetic component includes a prosthetic upper limb, or a portion thereof.

In typical embodiments, the connecting structure circumscribes a proximally open internal space that is substantially complementary to the lower limb of the patient. In particular embodiments, the residual limb is a lower limb, and the distal prosthetic component is a prosthetic lower limb. In some such embodiments, the connecting structure has a length that substantially corresponds to a length of the residual lower limb, such length extending from a distal end of the lower limb to a proximal point corresponding to the site of the ischial tuberosity of the pelvis of the patient. The site of the ischial tuberosity, in the context, includes the overlaying tissue, including muscle, fat, and skin. Typically, the patient has a contralateral intact lower limb having a length extending from a knee joint of the lower limb to a proximal point corresponding to an ischial tuberosity of a pelvis, and such length is greater than the length of the residual limb by a differential length. In such embodiments, the distal prosthetic substitute for the residual limb includes a pylon of such differential length, such pylon configured at its distal end of support further elements of a distal prosthetic substitute for the lower limb, including a prosthetic knee joint.

The scope of this tenth example may include any aspect or feature of other embodiments of the technology described herein.

An eleventh example of background information is embodiments which provide a longitudinal strut for inclusion in the assembly of a modular prosthetic socket. Such a strut may include a longitudinal axis and associated length; a distal portion and proximal portion along the length; a lateral axis and associated width; a lateral axis-associated cross sectional area, an internal surface and an external surface with reference to the prosthetic socket as assembled. Such a strut may also include a thermoplastic-fiber composite material arranged as one or more longitudinally aligned layers, said layers arranged through the length of the strut. Such a strut is particularly configured for assembly into a modular prosthetic socket assembly having a central longitudinal axis.

Embodiments of a prosthetic socket assembly for which the above described strut is suitable include sockets that have a plurality of the longitudinal struts, at least one pressure-distributing element such as a proximal brim, strut cap, or flexible internal liner supported by the struts, and a distal socket base arranged distal to the struts. The multiple struts and the at least one pressure-distributing element are mutually connectable, and the multiple struts are and the socket base are mutually connectable. Each of the preceding enumerated prosthetic socket components is selected from an inventory that includes at least one of multiple sizes or shapes of the respective assembleable component. The scope of this eleventh structural aspect (and a modular prosthetic socket in which a strut embodiment may be included) may include any aspect or feature of other embodiments of the technology described herein.

Embodiments of the thermoplastic-fiber composite material include a polymer matrix into which fiber is embodiments. By way of example, the polymer matrix may include any one or more of a polypropylene, a polyethylene, or a polyacrylate. In a particular example, the polymer matrix may include or may consist of polymethylmethacrylate (PMMA). Other polymer matrix examples include any one or more of the polycarbonate/ABS (a copolymer blend), high-density polyethylene, polyethyleneterephthalate (PET), polyetheretherketone (PEEK), Nylon (Pa6), or any suitable polymer.

In various embodiments of the thermoplastic-fiber composite material, the fiber of the thermoplastic-fiber composite material includes a fiber embedded in the polymer matrix; typical fiber composition examples include carbon or glass. Fiber may also include advanced synthetic fibers, generally referred to as "ballistic fibers", or any suitable or particularly advantageous fiber. One example of a suitable advanced fiber is Synthex TM , as manufactured by Fabtech Systems of Everett, WA. The polymer matrix may further include other forms of carbon, such a nanotubes and other nanostructures.

Further with regard to aspects and forms of fiber, in various thermoplastic composite bulk embodiments, fiber is typically arranged as woven bundles; common bundle sizes are, by way of example, 3,000 fibers/bundle and 12,000 fibers/bundle. Unidirectional fiber, in various thermoplastic composite bulk material embodiments, may be present at various densities. For example, a fiber may constitute about 60% by weight, and between about 30% and 55% by volume of the composite material. "Bulk", as used herein, simply refers to material that is commercially available, and useful as a raw material for fabricating processes described herein. "Bulk" has no particular connotation regarding size of a piece of thermoplastic-fiber composite material. Bulk material may be packaged in any suitable form, such a sheets, tapes, or blocks.

Fiber embodiments can also vary in length; short fiber lengths are generally referred to as discontinuous fiber or substantially continuous fiber. Long fiber lengths, for example, a length as long as an article containing such a fiber, is generally referred continuous fiber. In particular embodiments of fiber embedded in the polymer matrix of struts, the fiber includes or consists of substantially continuous fiber.

In various strut embodiments, fibers embedded within the polymer matrix may be aligned any of unidirectionally, bidirectionally, or multi-directionally. In particular embodiments, fibers are arranged as a bidirectional weave, with fiber populations oriented at 90 degrees with respect to each other. Although a 0/90 relative orientation of the warp and weft of a fiber weave is a typical arrangement, embedded fibers in the thermoplastic composite material may be woven in any practical orientation. Further, fiber populations may be overlaid each other without being interwoven. Further still, fibers may be oriented such that they are aligned with the longitudinal axis of the strut and perpendicular to the longitudinal axis, but the fibers may be aligned at any angle relative to the longitudinal axis of the strut. In one particular example, fiber layers or fiber orientations in a weave or overlay may be oriented substantially perpendicular to each other, each layer being oriented approximately 45 degrees deviation from the longitudinal axis of the strut.

In particular embodiments of a longitudinal strut, the thermoplastic-fiber composite material includes or consists of polymethylmethacrylate (PMMA) polymer with carbon fibers embedded therein.

From the foregoing, it is apparent that the thermoplastic-fiber composition of a strut can exist in numerous various embodiments, depending both on the thermoplastic and on the fiber portions of the composition. In terms of the modular aspect of struts, wherein, for example, dimensions and shape of a strut can vary despite having connecting features in common with each other, and which allow assembly with other modular components, it may be understood that a strut of a given size and shape can vary in composition. Accordingly, another modular aspect or attribute of struts encompasses the material composition of the strut.

In a typical process of manufacturing thermoplastic-fiber composite struts, the thermoplastic-fiber composite material, prior to being formed into a strut, is in the form of a bulk material available in the form of any of a sheet or a tape. In some strut embodiments, the thermoplastic-fiber composite material, in a bulk form prior to being formed into a strut, includes a sheet or a tape, and each layer of the multi-layered strut corresponds to a portion of the sheet or the tape. In some embodiments the thermoplastic-fiber composite material, in bulk form even prior to being formed into a strut, includes multiple layers. An example of source for bulk thermoplastic fiber composite material is Tencate Advanced Composites USA, Inc., of Fairfield, CA.

In some embodiments, a strut substantially consists of a thermoplastic-fiber composite material. In this context, a strut that consists substantially of a thermoplastic-fiber composite material refers to a strut having a major structural portion formed from a thermoplastic-fiber composite material. The strut may have other non-structural elements associated with it or bonded to it, or integrated in some manner. An example of such a non-structural feature or element is a surface coating or treatment, or a connector feature, or any element that performs a function that does not provide structural support for the socket as a whole.

In some embodiments, the thermoplastic-fiber composite material of strut embodiments is arranged as a multiple layer structure. By way of example, the thermoplastic-fiber composite multiple layer structure may included any of 2 layers to 10 layers, 11 layers - 20 layers, 21 layers - 30 layers, or more than 31 layers. Bulk materials can vary in thickness; thus total thickness of a formed strut is determined both by the number of layers and by the thickness of the bulk material.

The fibers within any individual layer of the multiple layer structure embodiments may be arranged unidirectionally or as a bidirectional weave. In particular embodiments, the fibers within each individual layer of the multiple layer structure are unidirectionally oriented, and the unidirectional orientations of fibers in adjacent layers are different.

Some embodiments of a strut suitable for use in a modular prosthetic socket include at least one layer wrapped circumferentially around longitudinally arranged layers. The thermoplastic-fiber composite material of the at least one circumferentially wrapped layer includes is typically identical or similar to that of the longitudinal layers, for example, the polymer may be selected from the group including a polypropylene, a polyethylene, or a polyacrylate. The thermoplastic-fiber composite material of the at least one circumferentially wrapped layer may include unidirectionally oriented fiber, the fiber including any one or more of carbon or glass. In particular embodiments, the thermoplastic-fiber composite material of the at least one circumferentially wrapped layer, prior to being formed into the strut, includes a bulk tape, the tape having a longitudinal axis and a longitudinally-aligned fiber embedded therein. An example of source for such a bulk tape material is Tencate Advanced Composites USA, Inc., of Fairfield, CA.

The thermoplastic-fiber tape may be wrapped in any suitable arrangement; for example, it may be wrapped so that the coils are substantially non-overlapping, or minimally overlapping, or the coils may be wrapped with significant overlapping between adjacent coils. Further, the portion of tape used in wrapping may be a single continuous piece, or it may include two or more separate pieces.

In typical embodiments, the thermoplastic-fiber composite composition of the longitudinal layers is the same as that of the circumferentially wrapped layer. This sameness can be generally advantageous because of the sameness of thermal behavior of the longitudinal and circumferentially wrapped layers. However, embodiments of the invention include instances wherein the compositions of the longitudinal layer and the circumferentially wrapped layer are different.

Embodiments of a strut suitable for use in a modular prosthetic socket have a cross sectional profile throughout the length of the strut, and in various embodiments the cross sectional profile may vary through the length of the strut. The region of a structure wherein a cross sectional profile changes is termed a loft. Accordingly, some embodiments of a strut may include one or more lofted transitional portions. The cross sectional profile of strut embodiments may take any one or more of various forms. For example, the cross sectional profile of a strut includes an internal-facing surface and an external-facing surface, and in various embodiments, the internal and external surfaces may independently be any of flat, convex, or concave.

Embodiments of a strut suitable for inclusion in a modular prosthetic socket may include one or sites of curvature within a plane defined by the central longitudinal axis of the strut. For example, in some strut embodiments, a distal portion of the strut includes a curve in its distal portion having an obtuse angle oriented internally with respect to the longitudinal axis of the prosthetic socket, when the strut is assembled thereinto. Such embodiments may include further sites of curvature proximal from the distal curve.

Embodiments of a strut suitable for inclusion in a modular prosthetic socket may be adapted and configured in various ways to facilitate attachment of the distal end of the strut to a distal base. In some embodiments, for example, a distal portion of the strut may include a metal core piece embedded within the thermoplastic-fiber composite material, the metal core piece including a distal end that extends longitudinally beyond the distal end of the thermoplastic-fiber composite material, the distal end of the metal core being adapted to connect with a distal cup portion of the prosthetic socket. In other embodiments, a modular prosthetic socket assembly may include separate strut connectors, as summarized above, that function to connect the distal end or portion of the strut and a distal base together.

As described elsewhere, heating a thermoplastic-fiber composite strut can be used to render a strut pliable such that it can be reformed to better fit a residual limb. Accordingly, some embodiments of a strut included a heating element embedded within the thermoplastic composite material. The heating element is configured to transmit electrical energy such that, when sufficient energy is delivered, the thermoplastic composite material is heated to a temperature that renders the thermoplastic composite material pliable.

Longitudinal struts formed from thermoplastic-fiber composite materials, as described herein, have a strikingly simple common or neutral modular form that can be modified to create highly individualized or customized strut forms for particular needs. Particulars of the thermoplastic-fiber composite material offer a number of options. The type of fiber, the orientation within the thermoplastic matrix, the bundling pattern of the fiber, the relative ratio of fiber to matrix, the number of thermoplastic-fiber composite layers, and the thickness of layers are examples of variables. Additionally, aspects of composition can vary within a single strut. For example, it may be advantageous for a proximal region of the strut to be particularly configured to provide support to an ischial tuberosity by broadening and strengthening the strut. Fortifying a strut within a particular region can be accomplished by working with these variables, by way of non-limiting example, by adding layers, or by using higher density fiber in that region of the strut. Still further, and as indicated by the ischial support example, struts within a set of struts included in an individual socket, can vary from one strut to another.

As discussed elsewhere, some aspects of customization include the ability to locate sites of curvature at any point along the strut. Sites of curvature can be created in a mold, or added later, in a reforming process. Cross sectional strut profiles have implications regarding flexibility and bias of flexibility, as well as providing different forms of contact against a residual limb, or implications for arrangements that attach the struts to tensioning mechanisms or pressure distribution elements. As discussed elsewhere, cross sectional profiles are variables, and sites of profile change can occur at any point along the length of a strut.

As noted above, in addition to the enumerated device and component-based structural aspects and embodiments of the technology, methods of forming and reforming modular components, methods of assembling a prosthetic socket, methods of replacing prosthetic socket components, methods of delivering a complete and fitted prosthetic socket within a short period of time, and methods of adjusting the assembled socket either by mechanical adjustments or by controllable exercising inwardly directed tension are provided as background information. Any method step described in the context of any single method embodiment may also be wholly relevant and usefully applicable to any other described method embodiment. It is noted that none of the described methods fall under the scope of protection of the present invention.

A first method example of the technology and associated embodiments provide a method for forming a strut for use in the assembly of a modular prosthetic socket. Forming a strut typically occurs by a molding process. Two basic types of molding may be useful approaches: in one example, a combination of heat and pressure is applied to make thermoplastic-fiber material pliable and amenable to molding into a desired for over or against a mold. In another example, heat may also be included, but it is vacuum pressure that presses pliable thermoplastic fiber composite material into a desired form over or against a mold.

Accordingly, this first method embodiment includes placing one or more appropriately sized pieces of a thermoplastic-fiber composite material proximate a strut mold, the mold being shaped as a complement to a strut of desired form. The method continues with molding the pieces of thermoplastic composite material into an integrated whole such that the material assumes the desired strut form; and separating a formed strut from the mold, the formed strut including one or more layers of thermoplastic composite material.

Some embodiments of the method, prior to placing pieces of the appropriately sized pieces into the mold, such pieces are cut from bulk sheets of such material. In various embodiments of the method, placing thermoplastic-fiber composite pieces proximate a mold refers to placement within a mold or over a mold.

In some embodiments of the method, molding the sheets of thermoplastic material into an integrated whole includes a combination of heating and pressuring such that separate sheets of the thermoplastic composite sheet pliably assume an integral form that is complementary to the mold. In other embodiments of the method, molding the sheets of thermoplastic material into an integrated whole includes a combination of heating and vacuum-based pressuring such that separate sheets of the thermoplastic composite sheet pliably assume an integral form that is complementary to the mold.

Conventional approaches to forming prosthetic sockets involve making a physical cast of a residual limb portion, and then using the cast as a mold for the socket being made. By way of clarifying aspects of the presently disclosed method, it can be understood that neither the forming or reforming methods disclosed here require the use of such a casting process. When molding of a strut occurs, per embodiments of the disclosed technology, such molding occurs within a production mold having a cavity with preset size and shape, or molding may occur as a prosthetist presses a heated and pliable strut against a portion of the residual limb of the patient being fitted. In order to contrast this latter method from the casting model described above, it can be said to be a direct molding. Direct, in this context, means "directly against the body, not against an intervening cast". Despite being "direct" in the sense that molding is being done against a portion of the residual limb rather than against or within a mold, the method further may include placing a heat-insulating fabric between the heated strut and the surface of the residual limb.

A second method example of the technology and associated embodiments provide a method for forming and reforming a strut for use in the assembly of a modular prosthetic socket by way of applying a combination of heat and pressure. "Forming" generally refers to the initial formation of a strut; "reforming" generally refers to a second event or process, whereby the already formed strut is again subjected to a combination of heat and pressure that is sufficient to render it pliable enough to be reshaped.

According, a method of forming a strut for a modular prosthetic socket includes placing one or more appropriately sized and shaped sheet pieces of a thermoplastic-fiber composite material into a mold, the mold being shaped as a complement to a strut of a desired form. The method continues by heating the mold to a temperature and for a period of time sufficient to render the thermoplastic-fiber composite material pliable within the mold such that the material assumes the form of the desired strut, cooling the mold sufficiently to allow the pliable material to solidify, and removing a formed strut from the mold.

The temperature being referred to, with reference to heating and cooling, is a glass transition temperature characteristic of the material. Above such temperature the material is pliable and may fluidize; below such temperature, the material assumes a substantially solid character.

Embodiments of the method typically further include applying pressure to mold, said pressure transferring to the thermoplastic composite material within the mold. Applying pressure typically includes applying a level of pressure that is sufficient to facilitate rendering the thermoplastic composite material pliable such that the thermoplastic composite material forms an integral strut. In particular embodiments of the method, the thermoplastic composite material includes polymethylmethacrylate (PMMA) and a fiber embedded therein.

Aspects of the method that are appropriate for some thermoplastics such as PMMA include heating the mold to a temperature of between about 177 degrees Celsius (350 degrees Fahrenheit) and about 260 degrees Celsius (500 degrees Fahrenheit). Cooling the mold includes lowering the temperature to at least about 93 degrees Celsius (200 degrees Fahrenheit).

In some embodiments, wherein the mold includes a cavity into which the one or more sheets of a thermoplastic composite material are placed, and wherein the cavity has dimensions and a shape, the method further includes forming a strut of dimensions and shape that complement the dimensions of the mold. In some of these embodiments, the method further includes providing or making use of a plurality of molds whose cavities vary in dimensions, and by using a mold with a cavity of desired dimensions, the method further includes forming a strut of desired dimensions. The method may further include providing or making use of a plurality of molds whose cavities vary in shape, and by using a mold of a desired shape, the method further includes forming a strut of a desired shape. In particular embodiments, the cavity of the mold or molds includes one or more appropriate and desired curvatures. Accordingly, in particular embodiments, the shape of the cavity of the mold includes a curve in the distal portion of the cavity having an obtuse angle, and by using a mold of a desired distal obtuse angle, the method still further includes forming a strut having a distal curve with an angle of desired degree.

Some embodiments of the method make particular use of thermoplastic-fiber composite bulk material in the form of a tape that can be used to add a circumferentially wrapped layer around a strut. Accordingly, in some embodiments, wherein the one or more sheets of the thermoplastic composite material are arranged longitudinally, and prior to placing the thermoplastic composite material in the mold, the method further includes circumferentially wrapping a portion of the thermoplastic composite material around the longitudinally aligned thermoplastic composite material.

As noted above, an already formed strut can be reformed in order to achieve a more desirable shape. Thermoplastic materials can reform an indefinite number of times. A reforming may also be referred to as a secondary forming step. In some instances a reforming step may be referred to as partially-reforming; this usage refers to the fact that while the initial forming process is directed to the strut as a whole, frequently a reforming step is directed only to a specific portion of a strut.

Accordingly, in some embodiments, the method may further include reforming a formed strut to create one or more curves in the strut; the reforming step may include applying heat to the thermoplastic material for a duration sufficient to sufficient to render the thermoplastic material pliable, and applying sufficient and appropriately directed force to the pliable thermoplastic material such that it reforms toward a desired reformed shape that conformally fits a portion of a residual limb of a patient. In particular embodiments, as may be appropriate for the thermoplastic-fiber composite material, applying heat may include heating the strut to a temperature of between about 177 degrees Celsius (350 degrees Fahrenheit) and about 260 degrees Celsius (500 degrees Fahrenheit). In typical embodiments, the range of temperature used in an initial forming step is the same range of temperatures appropriate for a reforming step.

In some embodiments of the method, a strut embodiment, upon being emerging from the forming steps, is substantially flat, and embodiments of reforming add one or more sites of curvature to the strut. More particularly, creating one or more curves may include imparting a curve within the longitudinal plane of the strut by forming an obtuse angle into the distal portion of the strut. In other method embodiments, creating one or more curves includes imparting a twist within the strut. Twists are made within or along the longitudinal axis of the strut, and may be imparted on a strut in either direction, clockwise or counterclockwise, and may include twists of up to 45 degrees.

In some embodiments of the method, reforming the formed strut is performed most specifically so as to fit a residual limb of a subject; in such embodiments, a reforming step typically includes heating the strut to a temperature of between about 177 degrees Celsius (350 degrees Fahrenheit) and about 260 degrees Celsius (500 degrees Fahrenheit). for a period of time sufficient to render the formed strut sufficiently pliable such that it is reformable. In some embodiments of the method, heating the strut includes generating heat in the strut by way of an electrically resistive heating element embedded in the strut.

As noted above, reforming a strut may be directed toward having a strut embodiment better fit an individual patient. Improving the fit of a strut may include using a portion of the residual limb, itself, as a mold for reforming the strut, a method known in prosthetic practice as "direct fitting". In spite of being referred to as direct molding, embodiments of the method typically include placing a heat-insulating fabric between the heated strut and the surface of the residual limb in order to protect the limb from excess heat. Further still, as in many aspects of prosthetic practice, reforming so as to improve fit may include the prosthetist exercising experience-based judgment in reshaping the strut, not necessarily adhering strictly to the shape as dictated by a mold, whether an inanimate mold or the residual limb, itself, as a mold.

As noted above, reforming a thermoplastic-fiber composite strut includes applying sufficient and appropriately directed force to a strut rendered pliable by heat, and applying force may include bending the pliable thermoplastic-fiber composite material against any appropriate molding surface. In an example of a direct molding method, applying sufficient and appropriately directed force includes pressing the pliable formed strut and a portion of the residual limb together so as to render the formed strut into a reformed strut.

In a direct molding approach, care to avoid burning the patient is advisable. Accordingly, such a direct molding type of reforming may include warming the strut sufficiently so as to be pliable, optionally placing a heat insulating fabric against a portion of the residual limb of the patient, and pressing the pliable formed strut and the portion of the residual limb together so as to render the formed strut into a reformed strut that improves the fit of the strut against the portion of the residual limb.

More particularly, embodiments of the method may include altering the shape of the strut according to a clinical judgment of a prosthetist so as to reform the strut such that it fits the portion of the residual limb in a biomechanically appropriate manner. A biomechanically appropriate fit may depart from a strictly conformal type of fit. The clinical judgment of the prosthetist may be informed by knowledge of any of aspects of desired or anticipated biomechanical activities of the subject. The clinical judgment of the prosthetist may further be informed by knowledge or observations of physical changes in the size or shape of the residual limb or by emergent clinical factors.

A fourth method example of the technology and associated embodiments provide a method of assembling of a modular prosthetic socket, wherein at least some of the structural components are formed from a thermoplastic-fiber composite material. Accordingly, in this method embodiment, a method of assembling a modular prosthetic socket for a residual limb of a patient includes assembling a selected set of longitudinal struts together with a selected distal socket base, the longitudinal struts typically being formed from a thermoplastic-fiber composite material, the socket base being disposed distal to a distal end of each strut, the socket base being particularly adapted and configured to support the struts. In this method embodiment, each of the selected struts, and the selected base is selected from a group of multiple struts or base components, respectively, that vary in size and/or shape. Assembling a prosthetic socket may further include making use of any of the other components described in this disclosure in the assembly process. Such preceding assembling steps yield an assembled prosthetic socket.

In alternative embodiments, one or more of the component groups may be of uniform or substantially identical form. For example, a group of distal bases may not necessarily vary in size or shape.

In some embodiments of the method, before the assembling steps, the method includes characterizing the residual limb of the patient by acquiring dimensions and shape. Any satisfactory method of acquiring dimensions and shape may be included in embodiments of the method. Available technologies include, merely by way of non-limiting examples, measurements by hand with a tape measure, computer-aided design (CAD) and computer numerical control (CNC) technologies, scanning and imagery technologies, and other suitable shape capturing technology.

In related embodiments, after characterizing the residual limb of the patient by dimensions and shape, the method may further include applying the dimensions and shape as criteria for selecting any of the set of longitudinal struts, the one or more brim members, or the base, for use in assembling the modular prosthetic socket. Embodiments of the assembled prosthetic socket circumscribe an internal proximally open space. In method embodiments that make use of the dimensions and shape of the residual limb, the method includes assembling a socket wherein the internal proximally open space is substantially complementary to the dimensions and shape of the residual limb of the patient.

In some embodiments of the method, before the assembling steps, the method may further include selecting the selected struts from a strut inventory including at least one of multiple sizes of struts or multiple shapes of struts. Similarly, before the assembling steps, the method may further include selecting the selected base component from a base component inventory including at least one of multiple sizes of struts or multiple shapes of base component. And further and similarly, before the assembling steps, the method may further include selecting the selected one or more brims from a brim inventory including at least one of multiple sizes of struts or multiple shapes of brim. Still further, the method of assembling a modular prosthetic socket may include applying at least one encircling tensioning mechanism around the proximal portion of the assembled prosthetic socket, the proximal portion including any of a proximal portion of the struts or the brim members.

The scope of this third methodological aspect may include making use of any aspect or feature of structural embodiments of the technology described herein, and it may include any method step described in the context of any other method embodiment of the technology described herein.

A fourth method example of the technology and associated embodiments provide a methods of replacing components of a modular prosthetic socket assembly, some of these embodiments include drawing modular prosthetic socket components from component inventories. Accordingly, in this embodiment, a method of replacing a modular prosthetic socket component includes providing an *existing* modular prosthetic socket, the prosthetic socket having assembleable components that include multiple longitudinal struts formed from a thermoplastic-fiber composite material and a distal socket base arranged distal to the struts. In such an assembly embodiment, the multiple struts are and the socket base are mutually connectable, and typically each of the assembleable components has been selected from an inventory having at least one of multiple sizes or shapes of the respective assembleable component.

This method embodiment typically applies to a modular prosthetic socket in which at least one of the assembleable components is in need of replacement. Accordingly, the method includes removing the existing modular prosthetic socket component in need of replacement, providing a new modular prosthetic socket component to replace the existing prosthetic socket component, and replacing the existing prosthetic socket component with the new prosthetic socket component to form an updated modular prosthetic socket for the residual limb. Several modular components of a prosthetic socket assembly are described herein, including, merely by way of non-limiting example: struts, bases, strut connectors, and pressure-distribution elements. Of these components, the struts are detailed most fully. Modular variations within groups or collections of struts include variation in composition, dimension, and shape. These various aspects of modularity are applicable both to (1) an initial assembling of a prosthetic socket and to (2) replacing components.

The modular component in need of replacement may need replacement for one or more reasons. In some embodiments of the method, the existing modular prosthetic socket is damaged or worn, but prior to being damaged or worn, was of an appropriate dimension and shape, and wherein the updated modular prosthetic socket is substantially identical in dimension and contour to the previously existing modular prosthetic socket. In other embodiments of the method, the existing modular prosthetic socket is in need of reconfiguring because the individual dimensions or the anatomical contours of the residual limb having changed, and wherein updated modular prosthetic socket differs in dimension and/or contour compared to the previously existing modular prosthetic socket.

In yet another advantageous application of the modular aspect of the provided prosthetic socket assembly and the ability to switch components in and out, such exchange of components may be advantageous for an individual who engages in different levels of activity. For example, an individual may have an ordinary lifestyle in many aspects, but also engage in more extreme or rigorous activity either as recreation or as part of a job. An individual, for example, may be an athlete, or may be required to handle heavy loads while working in a recycling plant. In this example, the individual may have a set of everyday struts for an everyday level of activity, and a set of heavy duty struts for days when the prosthetic socket is being asked to accommodate heavy loading.

Among the features and advantages of the invention is the ease with which modular components can be replaced. Replacement of a modular component (or multiple components) conserves the major portion of the socket, thus providing economic and logistical benefits. Replacement of components may be desirable for a number of reasons. For example, a component part may be worn or damaged; in which case, replacement restores the socket to a fully functional state. In these instances, replacing the existing modular component may be understood as any of maintaining, repairing, upgrading, or restoring the existing or "old" prosthetic socket.

In other instances, it may be desirable to replace a modular component so as to change the fit of the socket. Changing the fit may be in response to changes in the shape or dimensions of the residual limb, in which case existing modular components may be replaced with components of different size or shape. In still other instances, the changing of components may be in order to change the configuration of the socket as may be desired for a particular activity. For example, if the individual is engaging in a particular or a strenuous activity, reconfiguration of the socket may occur in preparation for such particular activity. In such instances as these, replacing the existing modular component may be understood as any of reconfiguring, reshaping, or resizing the original modular prosthetic socket.

The scope of this fourth methodological of the technology may include making use of any aspect or feature of structural embodiments of the technology described herein, and it may include any method step described in the context of any other method embodiment of the technology described herein.

A fifth method example of the technology and associated embodiments provide a methods for delivering a complete modular prosthetic socket to a patient in a manner that may be substantially quicker than may be feasible by prior art methods. In one embodiment, a prosthetic socket embodiment, individually fitted to a patient, is deliverable to the patient within 24 hours for the patient being engaged by professional prosthetists and technicians at a clinical facility. In particular embodiments, the prosthetic socket can be delivered and the patient discharged within less than 12 hours, or within less than 6 hours.

Accordingly, a method of delivering a modular prosthetic socket configured to fit a residual limb of a patient, the residual limb having individual dimensions and anatomical contours, the method includes receiving a patient in need of a prosthetic socket for a residual limb at a prosthetic facility, and at that facility providing assembleable prosthetic socket components having multiple longitudinal struts including a thermoplastic-fiber composite material, and a distal socket base configured to be arranged distal to the struts. The multiple struts and the socket base are mutually connectable. Typically, each of the assembleable components is selected from an inventory including at least one of multiple sizes or shapes of the respective assembleable component. The method further includes selecting one or more components from which to assemble a residual limb socket from the respective inventory of each component group such that, when assembled, the socket includes an internal space that will the fit residual limb. The method further includes assembling the selected prosthetic socket components from each of the groups of components to form the prosthetic socket for the residual limb. The method concludes by discharging the patient, now in possession of the prosthetic socket fitted to his or her residual limb, wherein the lapsed time between the receiving step and the discharging step is less than about 24 hours. In particular embodiments the lapsed time between receiving the patient and discharging the patient with a complete individually-fitted socket may occur in as few as 12 hours, 6 hours, or 4 hours.

Embodiments of this method may further include reforming steps, wherein the fit of thermoplastic-fiber composite struts to the residual limb of the patient is improved. Accordingly, the method may further include heating at least one of the selected struts sufficiently so as to render it pliable, placing a heat-insulating fabric against a portion of the residual limb of the patient, and reforming the now-heated strut by pressing it against the portion of the residual limb, the insulating fabric intervening therebetween, so as to improve the fit of the strut against the portion of the residual limb.

The scope of this fifth method example of the technology may include making use of any aspect or feature of structural embodiments of the technology described herein, and it may include any method step described in the context of any other method embodiment of the technology described herein.

A sixth method example of the technology and associated embodiments provide a method of mechanically adjusting the fit of a modular prosthetic socket to the residual limb of a patient. Such methods apply both to adjustments that can be made in the initial assembly of the prosthetic socket as well as to secondary mechanical adjustments that can be applied to a completely assembled modular prosthetic socket. These method embodiments are typically performed by prosthetist or appropriately trained technician.

Accordingly, a method of adjusting a fit of a modular prosthetic socket to a residual limb of a patient includes providing a prosthetic socket having components selected from component groups, the groups including (1) multiple struts including a thermoplastic-fiber composite material, the struts aligned with a central longitudinal axis of the socket assembly; (2) a distal socket base configured to be arranged distal to the struts, the distal base being particularly adapted and configured to support the struts. Such a prosthetic socket assembly circumscribes an internal space; the assembleable components of the socket have been selected such that, when assembled, the circumscribed internal space substantially fits the residual limb of the patient conformally. A "conformal" fit generally refers to the dimensions and shape of the internal space being a substantial complement to the dimensions and shape of the residual limb. The method continues by mechanically adjusting the arrangement between the struts and the distal base so as to improve the conformal fit of the prosthetic socket on the residual limb.

In one embodiment of this method, the struts can adjustably engage the distal base such that the struts are radially slidable between a minimal radial position and a maximal radial position, and adjusting the arrangement between the struts and the distal base includes adjusting the radial position of at least one of the struts with respect to the base. The "radial position" refers to the distance from the center of the prosthetic socket or its distal base. The "center" also defines a central longitudinal axis, ranging from the distal base to the most proximal boundary of the socket. In the proximal portion of the socket, the central longitudinal axis is disposed in the internal space circumscribed by the struts, or more particularly, circumscribed by a circle that is nominally described by the struts.

In some modular prosthetic socket embodiments, the radial position of the struts with respect to the distal base is fixable at any position between and including the minimal and the maximal radial positions. Accordingly, a method embodiment may further include fixing the radial position of the struts on the distal base. In some modular prosthetic socket embodiments, the modular prosthetic socket includes multiple strut connectors configured to connect a strut to the distal base. Accordingly, adjusting the radial position of at least one of the struts with respect to the base may include adjusting a radial position of at least one of multiple strut connectors. In another aspect, adjusting a radial position of at least one of multiple strut connectors may include adjusting a volume of the circumscribed internal space of the prosthetic socket.

In some modular prosthetic socket embodiments, the struts adjustably engage the distal base such that they are pivotable around a fixable radial position within an arc on or coplanar with the base. Accordingly adjusting the arrangement between the struts and the distal base may include adjusting the pivotable position of at least one of the struts.

In some prosthetic socket embodiments, an angle of pivot around the fixable radial position is fixable at any position within the arc of pivot; accordingly, some embodiments of the method may include fixing the angle of pivot at a given radial position. In various embodiments of the prosthetic socket and method, the arc of pivot ranges up to about 45 degrees (?) on either side of a neutral position. As noted above, some embodiments of the modular prosthetic socket have multiple strut connectors configured to connect a strut to the distal base; accordingly, in some embodiments, adjusting the arrangement between the struts and the distal base may include adjusting the pivotable position of at least one of the strut connectors around the radial position. In typical embodiments, pivoting a strut around a radial position is done for the purpose of aligning the struts of the prosthetic socket circumferentially around the socket in such a manner that best serves the anatomy of the patient and biomechanical operation of the prosthetic socket when being worn by the patient. More particularly, this pivoting feature may be advantageous in that, although a relatively small change with regard to the entirety of the circumference, such change in position can enable a position shift sufficient to avoid a site of irritation or sensitivity on the surface of the residual limb.

The scope of this sixth example may include making use of any aspect or feature of structural embodiments of the technology described herein, and it may include any method step described in the context of any other method embodiment of the technology described herein.

A seventh example of background information is embodiments which provide a methods for adjusting the fit of a modular prosthetic socket to the residual limb of a patient through the use of one or more tensioning mechanisms. In appropriate prosthetic socket embodiments, tensioning may be performed by one or more tensioning mechanisms applicable to the proximal region of the socket, as for example around the struts or brim members. These method embodiments are typically performed by the patient or wearer of the prosthetic socket, such adjustments being made according to his or her preference. These adjustments can be likened to tightening or adjusting the tension in shoelaces. Such adjustments may need to be done only once a day, but adjustments may occur multiple times during a day.

Accordingly, a method of adjusting a fit a modular prosthetic socket to a residual limb of a patient includes providing a prosthetic socket including a proximal socket portion that includes a proximal socket portion having multiple longitudinal struts, the struts including a thermoplastic-fiber composite material, each strut having a proximal portion and a distal portion, and having at least one pressure-distributing element such as a proximal brim, strut cap, or flexible internal liner supported by the struts. The proximal portion of the prosthetic socket circumscribes an internal space that is substantially complementary to the dimensionality and shape of the residual limb of the patient.

The prosthetic socket further includes a distal socket portion that includes a base arranged distal to the struts and connected to a distal portion of each strut. The prosthetic socket further includes a tensioning mechanism arranged to controllably apply inwardly directed force to the proximal socket portion; accordingly, the method includes adjusting the tensioning mechanism.

The scope of this seventh example may include making use of any aspect or feature of structural embodiments of the technology described herein, and it may include any method step described in the context of any other method embodiment of the technology described herein.

Particular embodiments of the technology relate to a modular prosthetic socket for a residual limb of a lower extremity of a patient that includes the following modular components: a distal base, strut connectors, and struts formed from a thermoplastic fiber composite material. Such a prosthetic socket embodiment includes a base selected from a collection of bases, each base having a center and a circumferential periphery; multiple strut connectors selected from a collection of strut connectors, each strut connector being adjustably connectable to the base along the periphery of the base; and multiple longitudinal struts selected from a collection of struts, each strut comprising a thermoplastic-fiber composite material, a proximal end and a distal end, each strut being connectable to the base along the base periphery via one of the strut connectors. At least one of the recited collection of bases, the collection of struts, or the collection of strut connectors comprises at least one of multiple sizes or multiple shapes of bases, struts or strut connectors, respectively. The assembled prosthetic socket embodiment circumscribes a proximally open internal space configured to conform to the residual limb of the patient.

In some of these embodiments of a modular prosthetic socket, the base, the multiple strut connectors, and the multiple longitudinal struts, collectively, include modular components of the prosthetic socket, and wherein each of the respective component collections includes multiple sizes or multiple shapes of the respective component. In some of these embodiments of a modular prosthetic socket, the multiple struts are not integrally connected with one another.

Some of these embodiments of a modular prosthetic socket further include a tensioning member coupled with the struts for applying tension to the multiple struts in a direction approximately toward a central, longitudinal axis of the socket.

In some of these embodiments of a modular prosthetic socket, thermoplastic-fiber composite material of the struts includes a polymer matrix includes a material selected from the group consisting of a polypropylene, a polyethylene, a polyacrylate, and any blend thereof. Further, in some embodiments, the thermoplastic-fiber composite material comprises a polymer matrix comprising a material selected from the group consisting of polymethylmethacrylate, polycarbonate/ABS, high density polyethylene, polyethyleneterephthalate, polyetheretherketone, Nylon, and any blend thereof.

In some of these embodiments of a modular prosthetic socket, the fiber of the thermoplastic-fiber composite material includes a fiber selected from the group consisting of carbon and glass. In some of these embodiments of a modular prosthetic socket, fiber of the thermoplastic fiber composite material is in a substantially continuous form. In some of these embodiments of a modular prosthetic socket, the fiber of the thermoplastic fiber composite material is arranged bidirectionally, with fiber populations oriented at approximately 90 degrees relative to one another. In some particular embodiments of a modular prosthetic socket, the thermoplastic-fiber composite material comprises polymethylmethacrylate polymer with carbon fibers embedded therein.

In some of these embodiments of a modular prosthetic socket, the multiple struts include at least three struts. In some of these embodiments of a modular prosthetic socket, the base includes multiple slots, each slot being configured to slidably host a strut connector, each slot being radially aligned from the periphery of the base toward the center of the base. In some of these embodiments, the strut connectors are radially adjustable relative to the base by sliding in the slots and further configured to pivot in the slots at any radial position. In some of these particular embodiments, the multiple struts, the multiple strut connectors and the base circumscribe an internal prosthetic socket volume, and wherein the internal prosthetic socket volume is adjustable by sliding one or more of the strut connectors in the slots to adjust a radial position of one or more of the struts. In some of these particular embodiments, the strut connectors are configured to be friction lockable against the base, and wherein, when locked, the strut connectors are fixed at a given radial position and pivot position.

In some of these embodiments of a modular prosthetic socket, the base includes at least one surface feature near each of the slots for limiting pivoting movement of the strut connectors in the slots. In some of these embodiments of a modular prosthetic socket, the base includes a plurality of cooperating plates.

In some of these embodiments of a modular prosthetic socket, each of the multiple strut connectors is proximally connectable to one of the multiple struts and distally connectable to the base. In some of these embodiments of a modular prosthetic socket, at least one of the struts and at least one of the strut connectors are integrated to form a single component.

In some of these embodiments of a modular prosthetic socket, each of the multiple strut connectors includes a base-contacting portion connectable to the base, and a takeoff portion connectable to one of the multiple struts. In various of these particular embodiments, an angle formed between the takeoff portion and the base contacting portion of each strut connector is between about 90 degrees and about 150 degrees. In other embodiments, the takeoff angle may range up to about 180 degrees.

In some of these embodiments of a modular prosthetic socket, the collection of struts includes struts that vary in at least one characteristic selected from the group of characteristics consisting of strut length, strut width, strut thickness, and strut contour profile.

Particular embodiments of the technology relate to a modular prosthetic socket for a residual limb of a lower extremity of a patient that includes the following modular components: a distal base, struts formed from a thermoplastic fiber composite material, and various pressure-distributing elements. Such a prosthetic socket embodiment includes a base selected from a collection of bases, each base having a center and a circumferential periphery; multiple longitudinal struts selected from a collection of struts, each strut comprising a thermoplastic-fiber composite material, a proximal end and a distal end, each strut being connectable to the base along the base periphery via one of the strut connectors; and at least one pressure-distributing element selected from a collection of pressure-distributing elements. At least one of the collection of bases, the collection of struts, or the collection of strut connectors includes at least one of multiple sizes or multiple shapes of bases, struts or strut connectors, respectively. The assembled prosthetic socket embodiment circumscribes a proximally-open internal space configured to conform to the residual limb of the patient. Some of these embodiments of a modular prosthetic socket further include an inwardly directed tensioning mechanism coupled with the multiple struts.

In some of these embodiments of a modular prosthetic socket, the at least one pressure-distributing element contacts at least one of the multiple struts or the base and is configured to distribute pressure over an area that is larger than a combined internal contact surface area of the multiple struts and the base.

In some of these embodiments of a modular prosthetic socket, the at least one pressure-distributing element includes a material selected from the group consisting of ethylenevinylacetate (EVA), low density polyethylene (LDPE), a blend thereof, other polymers, fabrics, and leather.

In some of these embodiments of a modular prosthetic socket, the at least one pressure-distributing element includes at least one proximal brim member connected to at least one of the multiple struts. And in some of these embodiments, the at least one proximal brim member includes an integrated circumferential tensioning mechanism configured to apply inwardly directed tension to the struts.

In some of these embodiments of a modular prosthetic socket, the at least one pressure-distributing element includes a flexible inner liner configured to be disposed internal to the multiple struts.

In some of these embodiments of a modular prosthetic socket, the at least one pressure-distributing element Includes at least one strut cap, each strut cap configured to fit onto the proximal end of one of the struts. And n some of these embodiments of a modular prosthetic socket, the at least one strut cap comprises one or more lateral elements configured to connect to any of a tensioning member or another strut cap.

Some of these embodiments of a modular prosthetic socket further include multiple strut sleeves, each strut sleeve configured to fit over one of the multiple struts. And in some of these embodiments, each of the strut sleeves includes at least one attachment site, the attachment site configured to attach to any of a tensioning member or another strut sleeve.

Particular embodiments of the technology relate to a method of forming a thermoplastic-fiber composite strut for a modular prosthetic socket for a residual limb of a patient. The method embodiment includes placing at least bulk form piece of a thermoplastic-fiber composite material into a mold, the mold comprising a cavity, the cavity comprising a shape complementary to a desired shape of a formed strut; heating the mold to at least a glass transition temperature of the thermoplastic-fiber composite material for a period of time sufficient to render the at least one bulk form piece pliable within the mold such that the at least bulk form pieces assumes a shape corresponding to the mold; cooling the mold sufficiently to allow the at least one bulk form piece to solidify in the shape and thus form the thermoplastic-fiber composite material strut; and removing the formed thermoplastic-fiber composite strut from the mold.

Some embodiments of a method of forming a strut further include applying pressure to mold to apply pressure to the material, during the heating step. In some embodiments of a method of forming a strut the thermoplastic-fiber composite material includes polymethylmethacrylate; and a fiber embedded in the polymethylmethacrylate. In some embodiments of a method of forming a strut, heating the mold includes heating to a temperature of between about 177 degrees Celsius (350 degrees Fahrenheit) and about 260 degrees Celsius (500 degrees Fahrenheit). In some embodiments of a method of forming a strut, cooling the mold includes lowering the temperature to at least about 200° F.

In some embodiments of a method of forming a strut, the mold includes a cavity into which the at least one bulk form piece of thermoplastic fiber composite material is placed, and wherein the cavity has dimensions and a shape, the method further comprising forming a strut of dimensions and shape that complement the dimensions of the mold. In some embodiments of a method of forming a strut, the desired shape of the strut includes at least one site of curvature.

Some embodiments of a method of forming a strut, further include reforming the already formed thermoplastic-fiber composite strut; the reforming step may include heating the thermoplastic-fiber composite material for a duration sufficient to sufficient to render the material pliable; and applying force to the pliable material to change the shape to improve a fit of the strut with of the residual limb of the patient.

In some particular embodiments a method directed to reforming a strut, the changed shape includes at least one of a curve or a twist within the strut. In particular aspects of reforming a strut, the method includes bending the pliable thermoplastic-fiber composite material against a molding surface. In particular aspects of reforming a strut, the method applying force includes pressing the pliable thermoplastic-fiber composite material against the residual limb with a heat insulating material between material and the residual limb.

Particular embodiments of the technology relate to a method of assembling a modular prosthetic socket for a residual limb of a patient. The method embodiment includes selecting a base from a collection of bases, wherein the base has a circumference; selecting multiple longitudinal struts from a collection of struts, wherein each strut includes a thermoplastic-fiber composite material, a proximal end and a distal end, and wherein the distal end of each strut is adjustably connectable to the base along the circumference; and attaching the multiple selected struts to the selected base to form the prosthetic socket. In practicing this method, at least one of the collection of bases or the collection of struts includes at least one of multiple sizes or multiple shapes of bases or struts, respectively. By way of practicing this method, the assembled prosthetic socket circumscribes a proximally open internal space configured to conform to the residual limb of the patient.

Some embodiments of a method of assembling a modular prosthetic socket further include selecting multiple strut connectors from a collection of strut connectors, each strut connector being adjustably connectable to the base along the circumference; and attaching the each of the selected struts with one of the selected strut connectors, the selected struts being attached to the base via the selected strut connectors.

Some embodiments of a method of assembling a modular prosthetic socket, before the selecting steps, further include acquiring dimensions of the residual limb of the patient; and using the acquired dimensions in performing at least one of the selecting steps.

Some embodiments of a method of assembling a modular prosthetic socket further include adjusting an attachment position of at least one of the selected struts to the base in order to improve a fit of the prosthetic socket on the residual limb. In some base embodiments, 52 the base includes multiple radially directed slots for attaching the selected struts, and accordingly, adjusting the attachment position includes sliding at least one of the struts in at least one of the radially directed slots. Further, the method of adjusting the attachment position may include pivoting at least one of the selected struts in at least one of the slots.

Some embodiments of a method of assembling a modular prosthetic socket further include selecting at least one brim member from a collection of brim members; and attaching the at least one brim member to at least one of the selected struts at or near their proximal ends.

In some embodiments of a method of assembling a modular prosthetic socket, after the attaching step, may further include replacing at least one existing prosthetic socket component. This aspect of the method includes removing at the least one component from the prosthetic socket to create a socket temporarily missing the at least one component; selecting at least one new prosthetic socket component from a collection of components, wherein the at least one new component has at least one of a different size or a different shape from the at least one removed component; and assembling the new component into the prosthetic socket missing the at least one component to create a new prosthetic socket. In such method embodiments, the at least one existing prosthetic socket component is selected from the group consisting of a base, a thermoplastic-fiber composite strut, a strut connector, and wherein the collection from which the component is selected consists of collections of such respective components.

These and other aspects and embodiments will be described in further detail below, in reference to the attached drawing figures.

### BRIEF DESCRIPTION OF DRAWINGS

Certain preferred embodiments and modifications thereof will become apparent to those skilled in the art from the detailed description below having reference to the figures that follow.
**Fig. 1** shows a side perspective view of an embodiment of a modular prosthetic socket in a fully assembled state.
**Fig. 2A** shows an exploded side perspective view of an embodiment of a modular prosthetic socket.
**Fig. 2B** shows schematic diagram of a system and method for assembling a modular prosthetic socket from inventories of modular component parts.
**Fig. 3** shows a patient with an above-knee amputation wearing an embodiment of a modular prosthetic socket suitable for this level of leg amputation.
**Fig. 4** shows a patient with an amputation at the knee, a knee-disarticulation, wearing an embodiment of a modular prosthetic socket suitable for this level of leg amputation.
**Figs. 5A and 5B** shows a side view and a perspective view, respectively, of an embodiment of a thermoplastic-fiber composite strut attached to a strut connector.
**Figs. 6A - 6E** show views of a series of thermoplastic-fiber composite struts as may be found in an inventory of struts that vary in size and/or shape.
**Figs. 7A - 7D** show a strut attached to a strut connector, and cross sectional profiles of a various thermoplastic-fiber composite struts.
**Figs. 8A and 8B** show a flow diagrams for forming **(****Fig. 8A****)** and reforming **(****Fig. 8B****)** a thermoplastic-fiber composite strut.
**Fig. 9A and 9B** shows a schematic cross-sectional views of sections of a thermoplastic-fiber composite strut; **Fig. 9A** shows a 3-layered strut section and **Fig. 9B** shows the layers separated out as they would have been prior to molding them together.
**Figs. 10A - 10D** show a schematic views of aspects of forming and reforming a section of a thermoplastic fiber composite strut.
**Figs. 11A and 11B** show aspects of an embodiment of a method of forming a multilayered thermoplastic-fiber composite strut; **Fig. 11A** shows a section of a multilayered strut and **Fig. 11B** shows the strut as a circumferentially wrapped layer of thermoplastic-fiber composite tape is being applied to the strut.
**Fig. 12** shows an aspect of reforming a thermoplastic-fiber composite strut known as "direct molding", wherein a strut has been heated sufficiently to make it pliable, and then is placed against the body and pressed against a portion of the residual limb of a patient by a prosthetist in order to impart a body-conforming shape to the strut.
**Figs. 13A and 13B** show embodiments of a thermoplastic-fiber composite strut with a resistive heating element embedded therein, the element allowing the strut to be self-heating for thermal reforming. **Fig. 13A** shows a strut with a serpentine heating element; **Fig. 13B** shows a strut with a mesh heating element.
**Figs. 14A - 14C** show an embodiment of a thermoplastic-fiber composite strut in an initial state, as it was originally formed, and two examples of the strut after being thermally reformed to better fit against a portion of the residual limb. **Fig. 14A** shows the strut in its originally formed shape; **Fig. 14B** shows the strut after it has been reformed to include a site of curvature; **Fig. 14C** shows the strut after it has been reformed to include an axial twist of several degrees.

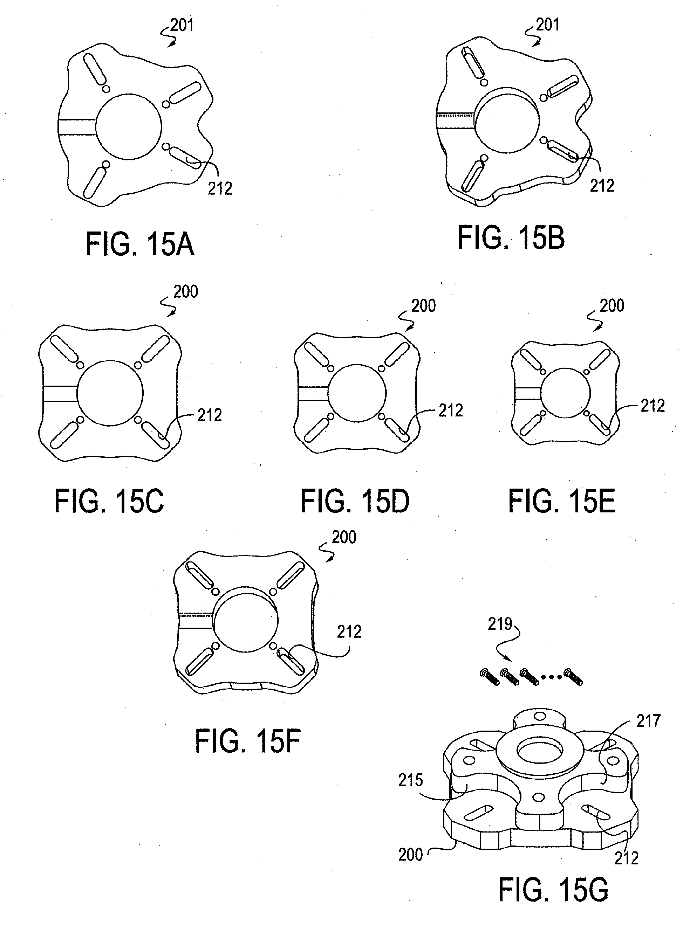

**Figs. 15A - 15G** show views of embodiments of a distal base for a modular prosthetic socket, each having four strut connecting sites. **Figs. 15A and 15B** show an embodiment wherein strut connecting sites are distributed in an arrangement with intervening angles of about 120°, 90°, 60°, and 90°. **Figs. 15C - 15F** show distal bases wherein the strut connecting sites are equally distributed at about 90°. **Figs. 15C - 15E** show distal bases that are identical in shape, but vary in size, as would components in an inventory. **Fig. 15G** shows an embodiment of a distal base for a modular prosthetic socket having a covering plate arranged over a base plate that includes strut connecting slots.

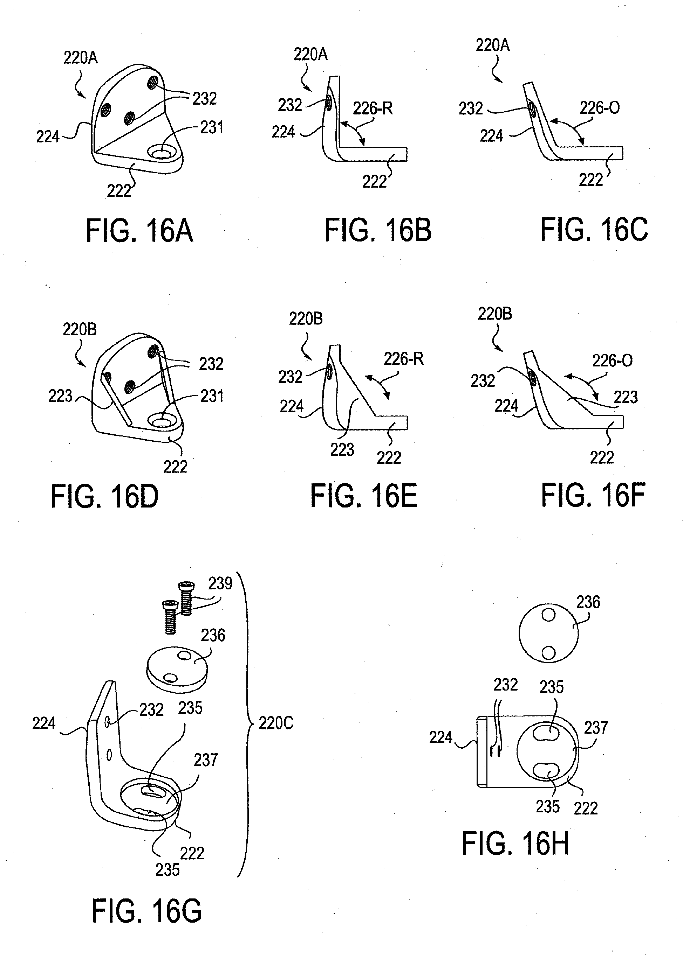

**Figs. 16A - 16H** show views of various embodiments of strut connectors for a modular prosthetic socket. **Figs. 16A - 16C** show an embodiment having a single attachment hole at the vertex of a triangular base. **Figs. 16A and 16B** show an embodiment wherein a connector backside is at right angle to a horizontal base. **Fig. 16C** shows an embodiment wherein the backside resides at an obtuse angle with respect to the horizontal base. **Figs. 16E - 16F** show an embodiment similar to those of **Fig 16A - C****,** this particular embodiment further having buttress supports extending from the lateral edges of the backside to the horizontal base. **Figs. 16D and 16E** show an embodiment wherein a connector backside is at right angle to a horizontal base. **Fig. 16F** shows an embodiment wherein the backside resides at an obtuse angle with respect to the horizontal base. **Figs. 16G - 16H** show an alternative embodiment of a strut connector, this embodiment having two attachment sites (to twin slots within a distal base) and a rotatable disc that cooperates in the attachment mechanism.

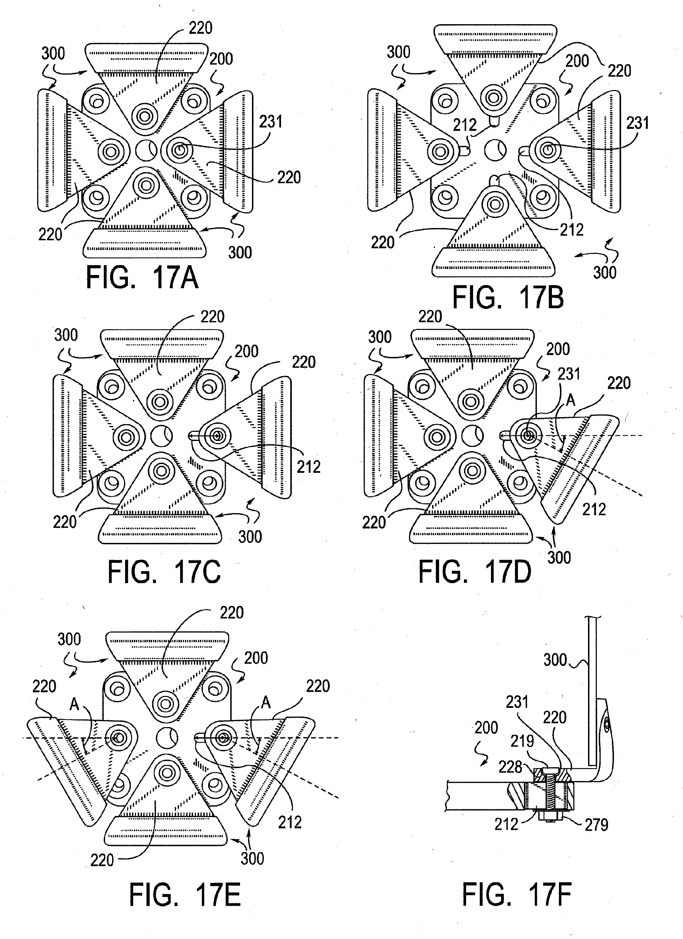

**Figs. 17A - 17F** show top views and a side view of an embodiment of a base for a modular prosthetic socket with the strut connectors positioned on a distal base into configurations that vary according to the radial position of the strut connectors and their degree of pivoting at their attachment site. This embodiment of a distal base accommodates four strut connectors, circumferentially spaced apart at 90° intervals. **Fig. 17A** shows four strut connectors, each of the four positioned at a minimal radial position. **Fig. 17B** shows four strut connectors, each of the four positioned at a maximal radial position. **Fig. 17C** shows four strut connectors, with one of the four (shown on the right) positioned at a maximal radial position, the other three being at a minimal radial position. **Fig. 17D** shows four strut connectors arranged as in **Fig. 17C****,** but with the strut connector on the right pivoted clockwise at an angle of about 20°. **Fig. 17E** shows four strut connectors arranged as in **Fig. 17C****,** but with the strut connector on the left and on the right, each pivoted at an angle of about 20°. **Fig. 17F** shows a side view of a distal base for a modular prosthetic socket with a strut connector positioned thereon, within a radial slot.
**Figs. 18A and 18B** show, respectively, top and side views of an embodiment of a strut connector that includes a secondary pivot external to the primary pivot, the second pivot allowing a restoration of the angle of the strut toward the center of the distal base after that particular strut has been rotated at the primary pivot site.
**Fig. 19A** shows a top perspective view of an embodiment of a distal base similar to that of **Fig. 14G** with a with a single strut attached thereto by way of a strut connector; this view shows how a base plate and a top plate can cooperate to provide a strut connecting site that further stabilizes the strut, and provides a boundary to the pivoting latitude.
**Fig. 19B** shows an alternative embodiment of a strut that has an integrated strut connector portion on its distal end.
**Figs. 20A** - **20D** show side views of thermoplastic struts that have a varying profile, ranging from substantially straight to having two or more sites of curvature.
**Figs. 21A** - **21G** show face views of thermoplastic-fiber composite struts that have one or more tab-like features extending laterally.
**Figs. 22A** - **22K** show views of various arrangements thermoplastic-fiber composite struts with pressure-distribution elements, such as strut caps, brim elements, and strut sleeves attached thereto.
**Figs. 23A** - **23** **C** show views of modular prosthetic socket embodiments, each with a different arrangement of pressure distributing elements, including strut caps and strut brims, each arrangement including a circumferential tensioning member.
**Fig. 24** shows an embodiment of a modular prosthetic socket that is particularly configured to accommodate a bulbous residual limb.
**Fig. 25A and 25B** show views of an embodiment of a modular prosthetic socket arranged with a flexible inner liner and a tensioning mechanism; **Fig. 25A** shows the prosthetic socket and flexible inner liner without tension being applied and **Fig. 25B** shows the prosthetic socket and flexible inner liner in a tensioned state.
**Figs. 26A and 26B** show views of an embodiment of a modular prosthetic socket in two configurations; **Fig. 26A** shows a socket with the struts and strut connectors positioned on a distal base within a relatively small radius, while **Fig. 26B** shows the same socket with the struts and strut connectors positioned on a distal base within a relatively large radius.
**Figs. 27A and 27B** show views of an embodiment of a modular prosthetic socket in two configurations; **Fig. 27A** shows a socket with one the struts and its strut connector at a neutral non-pivoted position on a distal base, while **Fig. 27B** shows the same socket with the struts and strut connectors positioned at a pivoted position. The pivoted position allows the strut to move away from a site of irritation on the residual limb.
**Figs. 28A** - **28C** and **Figs. 29A** - **29C** show views of a modular prosthetic socket **100** being worn by a patient on residual limb **700,** depicting two aspects of prosthetic socket strut flexing while walking. **Figs. 28A** - **28C** show flexing in response to loading and unloading of weight on the struts during a stride. **Figs. 29A** - **29C** show strut flexing in response to forward and rearward forces imparted to the residual limb during a stride.

### DETAILED DESCRIPTION

Device, system, and methods of the disclosed technology relate to modular prosthetic sockets for residual limbs, and to the materials included within prosthetic socket components. Technology disclosed herein is related to the subject matter of U.S. Patent Application No. 13/675,761 of Hurley and Williams, entitled "Modular prosthetic sockets and methods for making same", as filed on November 13, 2012. Novel aspects of the technology relate to a modularity of the system, wherein primary structural elements are provided as interchangeable components, and to characteristics of component construction and materials. The use of particular materials, such as thermoplastics and thermoplastic-fiber composite materials, in prosthetic components is disclosed in U.S. Provisional Patent Application No. 61/783,662 of Williams and Hurley, entitled "Modular prosthetic socket with components having thermoplastic-fiber composite materials", as filed on March 14, 2012. Modularity of a prosthetic socket assembly and the material properties of modular components both contribute to practical attributes of the technology, such as optimizing the fit of a prosthetic limb socket, allowing a fast fitting and assembly session, and allowing a fast reconfiguration or adjustment of the system as may be needed.

Fitting of an assembled modular prosthetic socket to the residual limb of a patient may be understood as being of various types or levels, depending on factors taken into consideration for the fitting. Fitting may involve any of (1) a skilled prosthetist or professional technician who takes full advantage of the structural variation within the prosthetic component inventories when selecting components for assembly into a socket and (2) exploiting attributes of component materials of the provided technology, particularly to properties of thermoplastic-fiber composite materials, and (3) making mechanical adjustments of adjustable aspects of an assembled prosthetic, or exercising various mechanical options during the assembly of a socket. The skilled prosthetist or professional technician, in performing these various types of fitting options may also draw on the capabilities of a larger system of information flow and component logistics as disclosed in U.S. Provisional Patent Application No. 61/916,579 of Hurley and Pedtke, as filed on December 16, 2013.

As disclosed in U.S. Patent Application No. 13/675,761, the provided technology includes a modular aspect in that a prosthetic socket can be assembled from an inventory of structural components. The components within an inventory, for example, an inventory of formed struts includes standardized strut forms that may vary in dimension and/or shape.
Additionally, struts may be initially formed according to specifications specific to the patient. Accordingly, a conformal or complementary fitting can be accomplished by selecting a set of struts from the variety of strut forms, which, when assembled, provide the best possible fit as derived from standard strut forms.

This basic level of fitting is a conformal arrangement, wherein a prosthetic socket conformally complements a residual limb in terms of dimensions and shape. This type of fitting relates to the residual limb as a static form, in that the fitting is to a residual limb, which, when captured only in terms of static dimensions and a static shape, does not necessarily include movement or apply other biomechanical considerations.

For some individuals, this basic level of fit may be sufficient and fully satisfactory. For some individuals, however, it may be apparent to a prosthetist that the level of fit may be improved, as for example, by reforming or partially reforming any one or more of the struts included in a prosthetic socket, as described below.

Thermoplastic components of a modular prosthetic socket, such as the struts, can be reformed, per the properties of thermoplastics that allow for an indefinite number of cycles of warming a solid form into a pliable and reshapable form, and cooling to return to a solid form with a different shape. Thermoplastic reforming of an already initially formed component, according to methods described herein, may provide more advanced levels of fit. Reforming of a thermoplastic component, such as a strut, typically begins with a strut that has been selected for assembly in a prosthetic socket so as to produce a socket that has a good conformal fit. Reforming the strut to alter its shape in some way is typically done to improve the level of fit.

An improved level of fitting achieved by a reforming process can raise the level of fit according to dynamic considerations, such as a fitting that accommodates and is appropriate for a range of motion that the residual limb may be expected to exercise, and to changes in shape or dimension that may accompany such motion, or which may vary according to load and weight distribution incurred during daily activity. To some extent, these variable dynamic aspects of a residual limb may be directly observable by a prosthetist, and can be directly incorporated into a fitting process.

An improved level of fitting achieved by a reforming process may take biomechanical aspects into consideration, such as habits or practices of the individual being fitted, or of more general physical attributes, such as body weight or other particular strengths or weaknesses of the individual. To accomplish this biomechanically appropriate level of fitting, in addition to practicing at least a conformal level of fitting, a prosthetist needs to understand details of the subject being fitted that may not be immediately observable during a fitting session, but which can be understood by way of gathering aspects of the subjects personal and medical history, and forward-looking personal aspirations.

An improved level of fitting achieved by a reforming process may take into account temporary or transient situations. For example, an individual may have some days that include a lot of physical activity, or an unusual activity, or a day that may be relatively sedentary. By way of another example, an individual may incur muscular soreness, or an injury, or an ulcer. In a slightly longer term, a subject may gain or lose weight, with directly related accompanying changes in the residual limb. Change in weight by also have delayed or more subtle consequences, such as a change in gait. Any of these eventualities may indicate the desirability for a changed shape in a particular socket component or the configuration of the socket as a whole. By way of example, if an individual develops an ulcer or is showing signs of skin breakdown at a site that contacts the strut of a prosthetic socket, per embodiments of the technology, the curvature of the strut can be adjust such that pressure exerted by the strut at the point of body contact is minimized.

These various types of considerations that may enter into improving fit are described in order to facilitate an understanding of the technology, and an underlying rationale of fitting as provided by embodiments of the technology. Any of these types of fitting may be generally understood as a "custom" fitting, or an "individualized" fitting, in that every aspect of fitting described herein relates to optimizing fit for each individual being fitted, in accordance with the various and numerous attributes of the individual that are specific to the individual. These various types of fitting are not mutually exclusive. Further, as noted, while improving the fit of a prosthetic socket may occur by reforming particular components, it may also occur by way of selecting components to be included in the socket assembly, or by reconfiguring the prosthetic socket assembly into alternative configurations, or by making mechanical adjustments.

Reforming a modular prosthetic socket component, such as a strut, so as to provide an improved fit of the strut to a residual limb, is made possible by the material composition of particular strut components, per embodiments of the disclosed technology. In some embodiments, the material composition includes a thermoplastic polymer; in other embodiments, the material composition may include a thermoplastic-fiber composite material. Typical thermoplastic-fiber composite material included in struts, for example, may include a polymer, such as polymethylmethacrylate (PMMA), which is fortified with embedded fiber, such as glass fiber, carbon fiber, ballistic fiber, or any other suitable or particularly advantageous fiber. Accordingly, a reforming step typically includes heating the initially formed piece until it is pliable, and followed then, by appropriate bending or shaping toward a desired shape.

Remolding and reforming are substantially similar terms, just as are molding and forming. Molding and remolding generally refer more to the aspect of the process whereby an article, such as a strut, is shaped in accordance with a physical mold or cast, or against a portion of a residual limb. Forming and reforming relate more particularly to shaping or reshaping of an article or a portion thereof, such as a strut, without particular emphasis regarding the physical model being used to determine the shape arrived at.

Remolding (as may follow an initial molding or forming) can be accomplished with various methods that are directed to conforming the struts and/or other aspects of the socket to comfortably and effectively complement the contours of the residual limb. One such example of a method is to place a thermal barrier over the residual limb; padding or inner liners can be fit prior to the structural frame of the socket. The struts and/or other aspects of the socket are then heated to an appropriate molding or heat labile temperature, and the socket component is placed in the correct orientation on the residual limb. A two-part sealing bag is then placed over the limb portion and strut, and air is pulled out of the bag such that the sealing bag pushes or sucks down to the shape of the residual limb with the struts also being pushed into the shape of the residual limb. A prosthetist can then use his or her hands and/or a fitting jig to distribute pressure in select areas to maximize biomechanical control and/or comfort.

In one example, fitting may include a mechanical adjustment of an assembled prosthetic socket that is already has a level of conformal fit. U.S. Patent Application No. 13/675,761, as referenced above, discloses examples of mechanical adjustments to attain better fit. Adjustment, by way of analogy, can be likened to lacing shoes that are already of the best available or initial fit. Although a prosthetist may perform such mechanical adjustments, per embodiments of the technology, some adjustment capabilities may be particularly in the realm of the patient, such as performing tensioning adjustments by way of tensioning and pulley arrangements. This type of adjustment is made primarily to suit the personal preference of the patient. Again, in a manner akin to the shoe lacing analogy, the major aspects of fit, in a conformal sense are not altered by this type of adjustment; what changes is the overall enclosed volume of the prosthetic socket assembly and the consequent change in tightness, as experienced by the patient. Mechanical adjustment of fit may well involve adjustment of prosthetic socket components that include thermoplastic-fiber composite materials, but such adjustments do not necessarily involve the thermoplastic properties of the materials.

A prosthesis wearer can improve or adjust fit during daily use by making mechanical adjustments of an assembled prosthetic socket. Mechanical adjustment may occur, by way of example, by adjusting a tensioning mechanism that is connected to socket components.

In another example, fitting may include mechanical adjustments made during the assembly of a prosthetic socket. These mechanical adjustments differ from those described immediately above in that these generally are options exercised by the prosthetist (not the patient) as he or she is assembling the prosthetic strut, making judgments based on clinical experience and knowledge of the patient. By way of example, embodiments of the technology described herein include strut connectors or base-strut connectors disposed on a distal socket base, to which the distal ends of struts are attached. Such strut connectors may be variably positioned. In one example, the strut connectors may be positioned at varying distances from the center of the socket base (this may be referred to as the radial position), or at varying distances from the central longitudinal axis of the prosthetic socket assembly as a whole. In another example, the strut connectors may include a freedom of pivotability, wherein the pivot represents a movement through an arc, a portion of a circumferential rotation. Both of these examples have proximal-ward and volume implications for the prosthetic socket as a whole with regard to fitting; the variations in distal attachment sites manifest as variation in volume circumscribed by an internal aspect of the assembled socket, as defined by the radial positioning of the struts collectively.

From another perspective, fitting may be understood as being applicable to individual socket components, but also to the configuration of the prosthetic socket as a whole. Accordingly, and by making use of inventories of component groups, a prosthetic socket can be reconfigured by removing an existing component and replacing it with a new component, as for example, a new component that differs in size or shape with respect to the existing component. In some instances, removing and replacing parts is undertaken to replace an old part that is worn or damaged. In this case, the overall socket configuration is not being reconfigured with regard to its shape. This non-reconfiguring part exchange may be understood more simply as a repair, restoration, or maintenance of the socket, conserving its original or intended configuration.

Configuring (and reconfiguring) a socket may further include exercising assembly options, such as varying the number of struts (*e.g.,* three struts, four struts) and/or varying radial spacing of struts around the central longitudinal axis of the socket, or by varying the attachment angle of the proximal base of a strut with respect to the longitudinal axis of the prosthetic socket.

Although configuring and reconfiguring a prosthetic socket relates to the selection of modular components and replacement of such components, it may further relate to structural options provided by the technology even as provided with a given set of components. By way of contrast, even absent the adjustable capabilities provided by tensioning (as noted above), the configuration of a prosthetic socket is not wholly or fixedly determined by the selection of components. By way of example, as provided by some embodiments, the positioning of struts with respect to a distal base can be changed by adjusting the radial position of the struts from the center of the base, or by rotationally pivoting the strut from a given radial position.

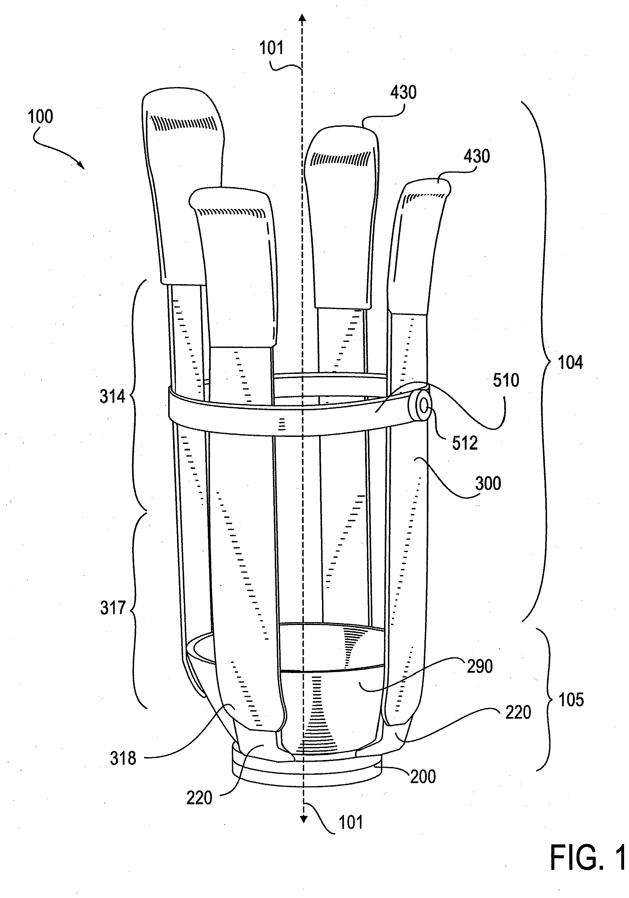

**Figs. 1 - 28C** illustrate various embodiments, examples, and aspects of the technology as described above. These figures depict examples of the structure of a modular prosthetic socket, particularly embodiments that include multiple longitudinal struts formed from thermoplastic-fiber composite materials attached to a distal base. A particular focus includes structural and functional details of how the struts attach to the base by way of strut connectors and the aspects of the base that accommodate the strut connectors. By way of the strut connectors and their sites of attachment to the base, in various embodiments, several degrees of freedom and adjustability are provided. All of these forms of adjustability and variability of configurations are applied toward attaining and maintaining a clinically optimal fit of the prosthetic socket on the residual limb. Aspects of the adjustable arrangement of struts with regard to a distal socket base have implications with regard to adjustability of the struts as they project proximally from the base and, thus, how the prosthetic socket fits a residual limb. Aspects of methods are also illustrated, with particular attention to methods of forming and reforming thermoplastic-fiber composite struts. Such methods of forming and reforming are also related to adjustability and long-term maintenance of the fit of a modular prosthetic socket on a residual limb.

In the following description and in the attached drawing figures, a given numerical label may be used to refer to the same component part in different embodiments. For example, a strut of a prosthetic socket may be referred to as strut **300** in multiple different embodiments, rather than labeling different embodiments of struts with different numbers. Struts may have any of a number of different sizes, shapes and material properties in alternative embodiments, but some or all of these embodiments may be labeled with the same number below and in the attached drawing figures. This labeling consistency is used to facilitate understanding of the description and should not be interpreted as suggesting that there is only one embodiment of any given component.

Referring now to **Fig. 1****,** in one embodiment, a modular prosthetic socket **100** may include four thermoplastic struts **300,** each of which is attached to a strut connector **220,** which in turn is connected to a distal base **200.** A central longitudinal axis **101** is shown. The prosthetic socket 100 has a proximal portion **104** and a distal portion **105.** These portions 104, 105 are identified for general orienting purposes; there is no bright line demarcation between the proximal and distal portions, although the distal base **200** is clearly included in the distal portion 105.

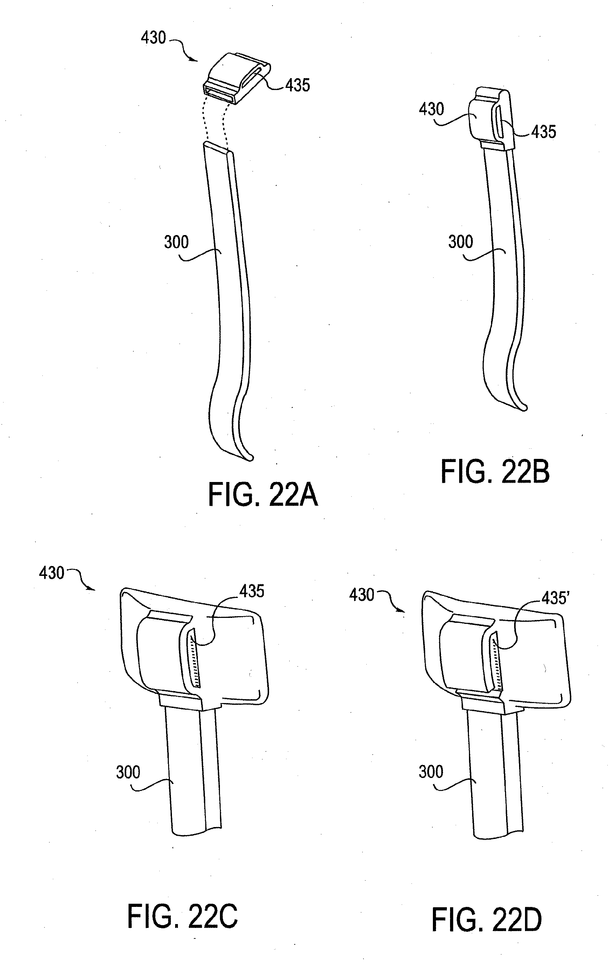

Struts **300** may also be divided into a proximal portion **314** and a distal portion **317.** As with the socket 100 as a whole, these proximal 314 and distal 317 strut portions have no bright line demarcation, but are used for general orientation when describing the struts 300 or elements associated with them. Distal ends **318** of the struts **300** are connected or fastened to strut connectors **220.** Details of the strut connectors 220 and their relationship to the distal base 200 and the struts 300 are detailed in **Figs. 16A - 19** that follow. The proximal ends **315** of the struts **300** are not visible in **Fig. 1****,** as they are covered by embodiments of strut caps **430.** Strut caps **430** are included within a broader group of components referred to as pressure distributing elements. Other pressure distributing elements include brim elements **420** and a flexible inner liner **410,** as depicted in **Figs. 22A** - **23C****,** and described below. An embodiment of a distal cup **290** is disposed above the distal base embodiment **200,** nested within the distal ends of struts **300.**

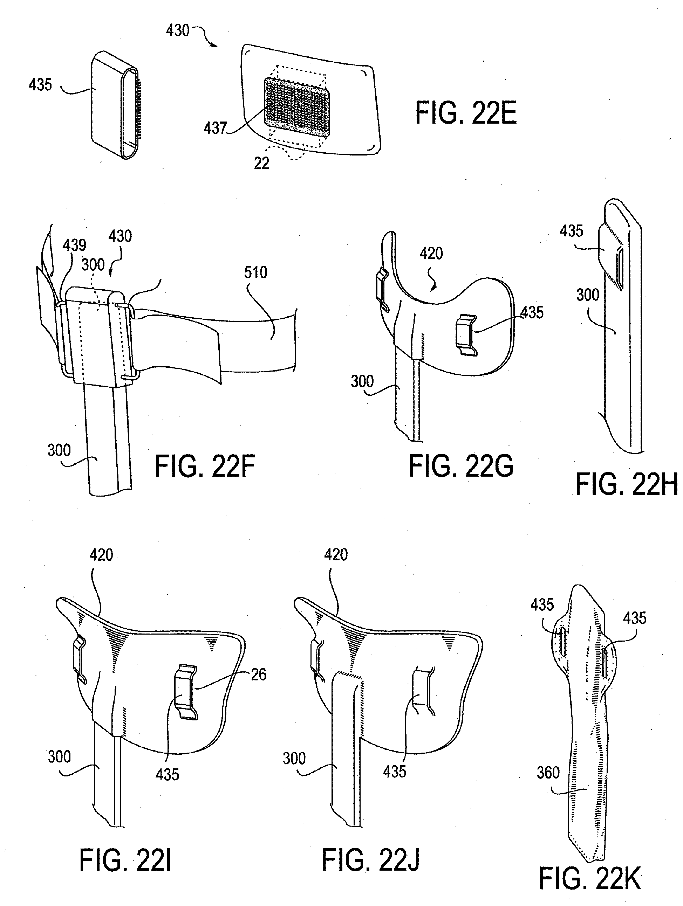

**Fig. 1** further shows an embodiment of a tensioning mechanism **510** arranged around a generally central section of struts **300.** Tensioning mechanism **510** is but one example of a number of different types of tensioning members. This particular arrangement includes a strap **510** with a tightening mechanism **512.** Modular prosthetic socket embodiments may include one or more of such tensioning mechanisms **512,** distributed along the length of the prosthetic socket. Other embodiments of tensioning mechanisms are depicted in **Figs. 22F** - **23C****,** and described below.

Struts **300,** a distal cup or pad **290,** and strut caps **430,** collectively, form a proximally open space or cavity that is configured to accommodate a residual limb, such as a lower limb. In other embodiments of a modular prosthetic socket assembly, other components, particularly various embodiments of pressure distributing elements, may participate in defining this proximally open cavity. All embodiments, however, are configured to individually fit, at least in a conformal sense, the size and shape of the residual limb of the patient for whom the prosthetic socket is intended. An extensive description of the various types of fitting that may be ascribed to the relationship between (1) the space nominally defined by the inner boundary of the limb-hosting space of the socket and (2) the dimensions and contours of the residual limb itself is provided above.

Struts **300,** despite being narrow, having minimal if any lateral curvature, and having only generally simple longitudinal curvature, nevertheless can very effectively define a complex shape with a fidelity that conformally fits a residual limb as well as larger, shell-like sections of prior art sockets, with broad surfaces, and having curves played out in two planes. As discussed below, the relatively simple structure of the struts 300 permits the use of thermoplastic-fiber composite materials, the fiber being of a continuous form. The use of such materials, in turn, advantageously provides high strength, a favorable strength/weight ratio, and a resilience to the socket as assembled.

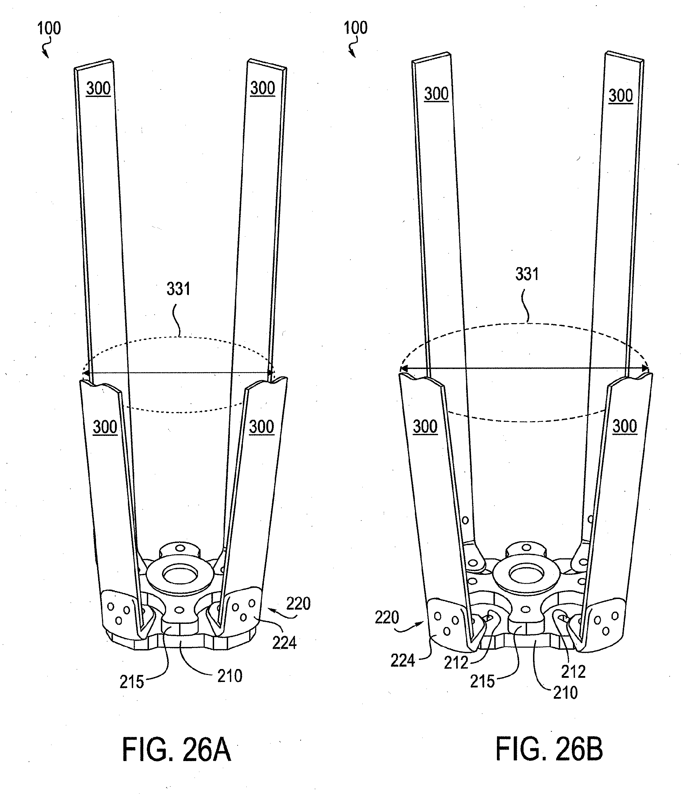

Since the struts **300** provide the major structural boundary of the modular prosthetic socket 100 (setting aside liner embodiments that may be inserted within the socket), the socket 100 has a considerable amount of open space e.g., spaces between the struts 300 as well as the proximal opening of the socket 100. The open aspect is advantageously associated with an ability to allow escape of heat and moisture from the residual limb. If the cross sectional profile of an embodiment of a socket is taken approximately at a socket midpoint (see **Figs. 26A and 26B****,** for example), each strut **300** may occupy an arc of no greater than about 25 angular degrees of that midpoint circumference, for example. Similarly, in a typical embodiment, the total enclosing coverage of struts **300** around a midpoint circumference is no greater than about 100 angular degrees.

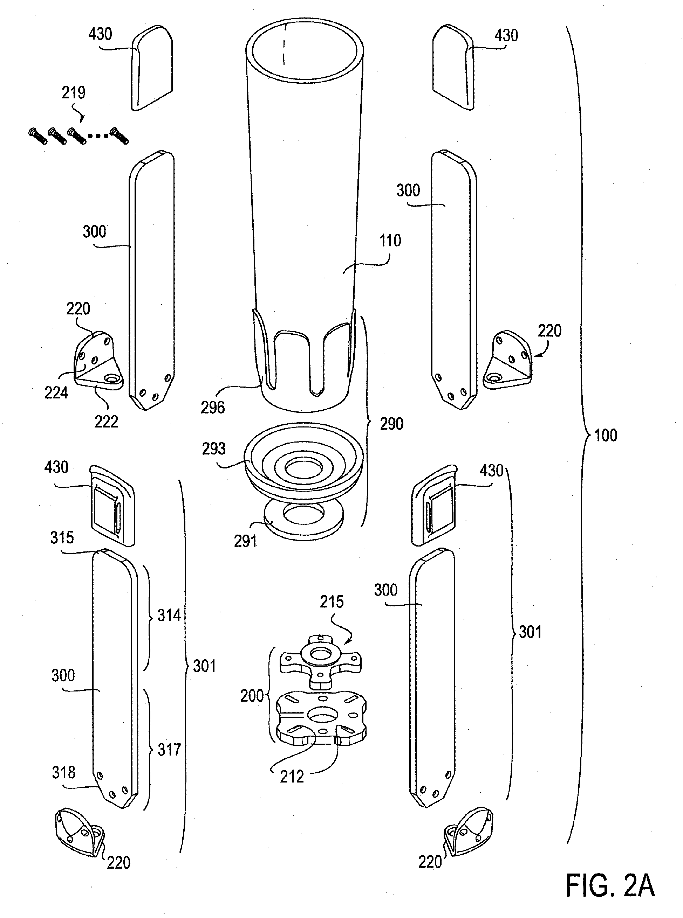

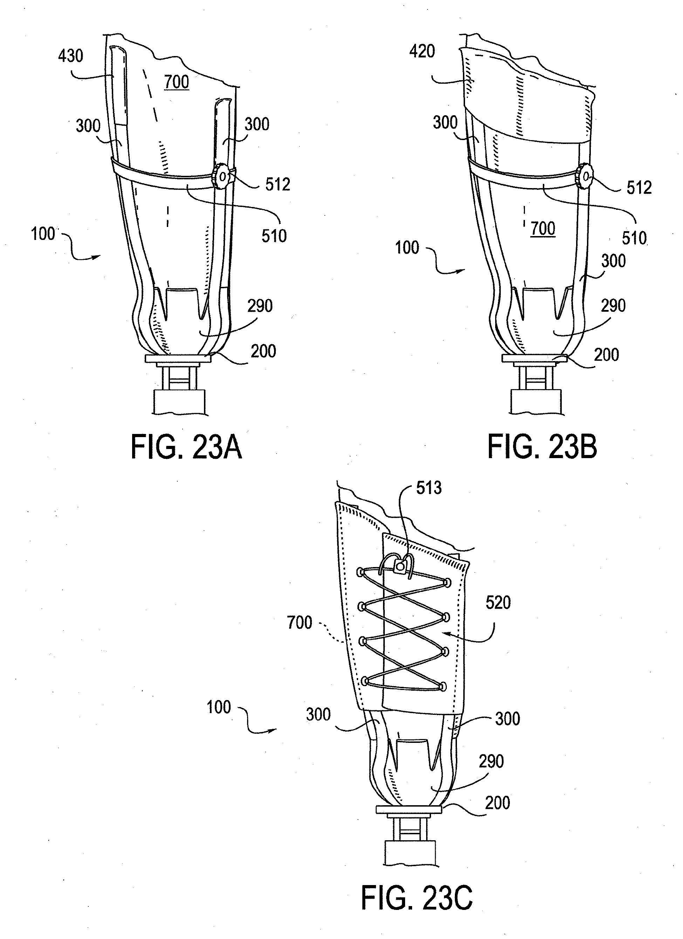

**Fig. 2A** is an exploded view of the modular prosthetic socket **100.** Distal base embodiments **200** may include one or more cooperating plates. A distal base assembly **200,** per this embodiment, includes a lower plate **210** and an upper plate **215.** An embodiment of a distal cup **290** is shown above the distal base **200.** Distal cup embodiments may also be considered an assembly, optionally including one or more cooperating cups. This particular embodiment includes two cups a lower pad **291** and an upper pad **293.** In an arrangement such as this, the lower pad **291** would typically be of relatively high durometer, and the upper pad **292** would typically be of a relatively low durometer. A roll-on liner **110** is shown above the distal cup **290;** the roll-on liner **110** is supported by a firm liner support cup **296,** which may also be considered a part of distal cup **290,** or in some embodiments represent the distal cup **290** in its entirety. Thus distal cup **290** may thus include multiple cooperating components such as lower pad **291,** upper pad **293,** and liner support cup **296.** In some embodiments, distal cup **290** may be a single component, or particular components may be recognizable as having a form such as lower pad **291,** upper pad **293,** and liner support cup **296,** but be integrated such that they are conjoined. In some embodiments, any one of these single components may comprise the entirety of a distal cup, and thus may be referred to as distal cup **290,** as for example, in **Figs. 23A** - **23C****.** In some embodiments, the roll-on liner **110** may include moisture management features.

A set of four longitudinal struts **300** are arrayed about the distal base **200,** the distal cup **290,** and the roll-on liner **110.** Struts **300** may be considered to be part of a strut assembly **301** that further includes a distally connected a strut connector **220** and a proximally connected pressure distribution element, such as a strut cap **430.** Typical embodiments of a modular prosthetic socket assembly include four longitudinal struts **300,** as shown here, although other embodiments may have fewer or more than four. Each strut **300** has a proximal portion **314,** a proximal end **315,** a distal portion **317** and a distal end **318.** In this embodiment, the struts **300** are arrayed at 90° angular intervals, per the arrangement of strut connecting sites on the distal base assembly **200.** Other angular intervals possible in alternative embodiments (see **Figs. 15A and 15B****,** for example).

An embodiment of a strut connector **220** is shown proximate the distal end **318** of each strut **300.** Strut connectors 220, in an assembled socket 100, connect both to the distal end of a strut 300 and to the distal base **290,** and by such arrangement, struts 300 are connected to the distal base.

A strut cap **430** is shown proximate the proximal end **315** of each strut **300.** When assembled, the strut cap 430 is disposed over the proximal end of a strut **300.** Strut caps **430** are an example of a pressure distributing element of the modular prosthetic socket assembly **100** Pressure distributing elements are configured to distribute pressure on a residual limb away from the site of contact between a structural element, such as a strut, and the area on the residual limb that the structural element contacts. Other pressure distributing elements include a proximal brim **420** and a flexible inner liner **410** (not shown in **Fig. 2A**).

**Fig. 2A** also provides a perspective with regard to various methods associated with the modular prosthetic socket, such as methods of assembling a modular prosthetic socket, replacing components of an existing modular prosthetic socket, mechanically adjusting the fit of a modular prosthetic socket, and making a modular prosthetic socket within 24 hours or less. In particular instances, a modular prosthetic socket can be assembled and delivered to patient within 12, 8, or even as few as 4 hours from the time first introduction to a prosthetist. These rapid turn-around times depend on the modular aspects of the assembly and having appropriately stocked inventories of components on hand.

To assemble the modular prosthetic socket **100,** in one embodiment, upper distal plate **215** and lower distal plate **210** can be assembled together to form distal base **200.** Distal bases may include one or more cooperating plates; any single plate embodiment, or any plate of a multiple-plate embodiment may be referred to as distal base **200.** Struts **300** and strut connectors **220** can be assembled together. Strut connectors **220** can be assembled together with distal base **200,** slidably fitting into radial slots **212** of lower base plate **210.** Fastening elements **219,** generally threaded bolts, are used to attach the various modular components together. Threaded fastening elements are generally advantageous in the assembly of a modular prosthetic socket because, in many aspects, the socket is configured for disassembly with an ease that is equal to that of assembly. Fastening elements, **219,** typically threaded bolts, may be used variously to attach struts to strut connectors **220,** strut connectors to a base **200,** and to connect base plates together if there is more than one base plate.

Strut caps 430 are connected to the proximal ends of struts **300.** The actual order of assembly steps can vary. The basic structural skeleton of a modular prosthetic socket embodiment is represented by the sum of correctly assembled component struts **300,** strut connectors **220,** and distal base 290. Further liner or residual limb support elements, such as the gel liner **110** and liner support cup **296,** can be slipped into the assembled socket 100 from the proximally open end of the socket **100.**

**Fig. 2A** also provides a perspective on the wide variety of sizes and shapes of prosthetic sockets **100** that can be assembled from modular components that vary in size or shape, using common connecting features. For example, as shown in various figures and described in further detail below, distal base embodiments **200** can vary both in size, and in the number of strut connecting sites. Strut connectors **220** can vary in shape, primarily with regard to the angle of the back portion **224** with respect to base portion **222.** And the size and shape of struts **300** can vary in multiple ways. For example, any of length, width, and thickness can vary. Cross-sectional profile can vary. And curvilinear forms can vary widely, and be made to conform to the shape of an individual residual limb.

Aspects of replacing modular components can also be understood from **Fig. 2A****.** Any particular "existing" component can be removed, leaving the remaining portion of the assembled socket intact, and the removed component can be replaced with a "new" component part. For example, if a strut **300** is damaged, it can be unbolted from its strut connector **220** and replaced with a new strut. Such a component replacement can also be made in order to improve the fit of the socket to a residual limb. For example, if a residual limb has atrophied, an original or existing strut may not fit a particular portion of the residual limb as it did originally, or as would be desired. In this instance, a "new" strut, one with a different shape, for example, could be selected and put into the same position as the "old" strut.

In some embodiments, it may be beneficial to remove an existing strut **300** and thermally reform it to a more desirable shape. This may be done, for example, to improve the fit of a given modular prosthetic socket.

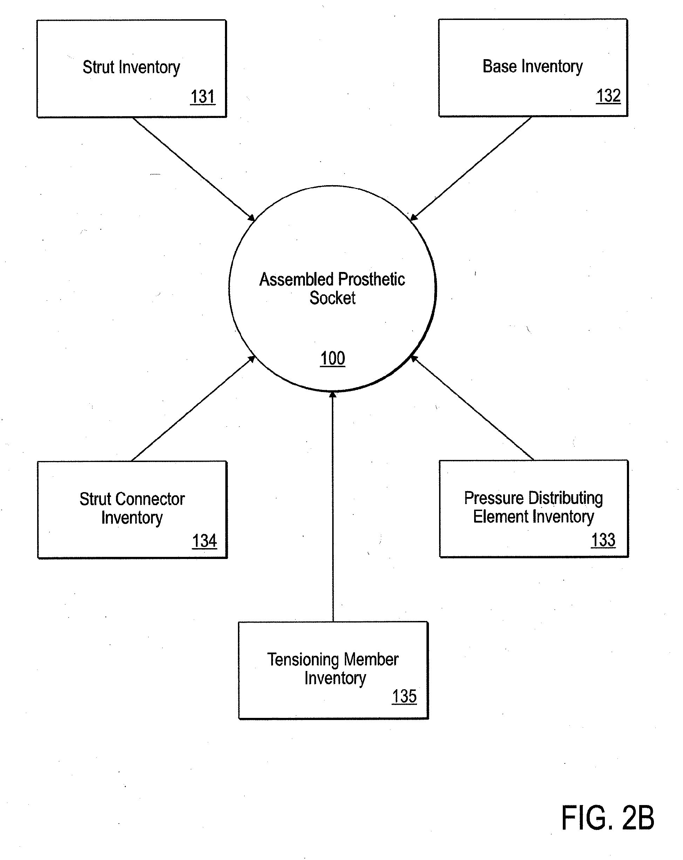

**Fig. 2B** is a schematic diagram of a system for assembling a modular prosthetic socket **100** from modular component parts. Arrayed around an assembled modular prosthetic socket are inventories of modular component parts. These inventories include a strut inventory **131,** a distal base inventory **132,** a pressure distribution element inventory **133,** a tensioning member inventory **134,** and a strut connector inventory **135.** Inventories may also be generally referred to as "groups" or "collections" of components. These are non-limiting examples of modular components that may be used in the assembly of a modular prosthetic socket. As described elsewhere, an inventory may be an actual physical inventory, or it may be a virtual or catalogue-based inventory. Inventories may also be used to package kits of components, or alternatively, a kit may also be considered a small inventory itself. Inventories of modular components typically include like components, with portions such as connecting sites in common, but otherwise including variations in size and/or variations in shape. In some instances, modular components may also differ from each other in material composition. **Fig. 2B** may also be interpreted as a diagram depicting a method of assembling a modular prosthetic socket in that components may be selected from such inventories **(131** - **135)** and assembled together to create a modular prosthetic socket of desired size and shape.

A strut inventory **131** may include struts **300** that can vary in size and shape. Size variations may include dimensions such as length, width, and thickness. Shape variations may include numerous contour profiles. Composition variations of struts **300** include variations in the thermoplastic composition of the thermoplastic fiber composite material as well as the fiber composition. Other strut embodiments of include struts **303,** which include lateral tab elements **304.** A distal base inventory **132** may include bases **200** of different dimensions and shapes, and as well as the number of struts that can connect to the base. **Figs. 15A -15E** provide examples of bases that are alike in shape, but vary in size. **Figs. 15A** and **15B** shows bases **201,** which vary from bases **200** in the angular distribution of strut connecting sites or radial slots **212** around the periphery of the base. A pressure distributing element inventory **133** may include one or more basic types of pressure distributing elements such as brims **420,** strut caps **430,** or a flexible inner liners **410.** All of these pressure distributing elements can have variation in size and/or shape, and yet still have connection or attachment elements that allow them to assemble with other modular components. A tensioning member inventory **134** may include tensioners, such as straps or cord **510** or laceable corsets **520.** Each of these types of tensioning members can vary in size and/or shape, and yet be able to be applied to an assembled prosthetic socket **100.** A strut connector inventory **135** may include strut connectors **220,** or variations thereof, such as strut connectors **220A, 220B** and **220C,** as seen in **Figs. 16A** - **16H****.** Strut connectors **220A** and **220B** differ in shape by virtue of the addition of buttress-like portions **223** of strut connector **22B.** Each of these strut connector embodiments may vary in shape by virtue of variation in takeoff angle **226,** which can be a right angle **226R (****Fig. 16E****)** or an obtuse angle **226-O (****Fig. 16F****),** which can range between about 90° up to about 150° or more.

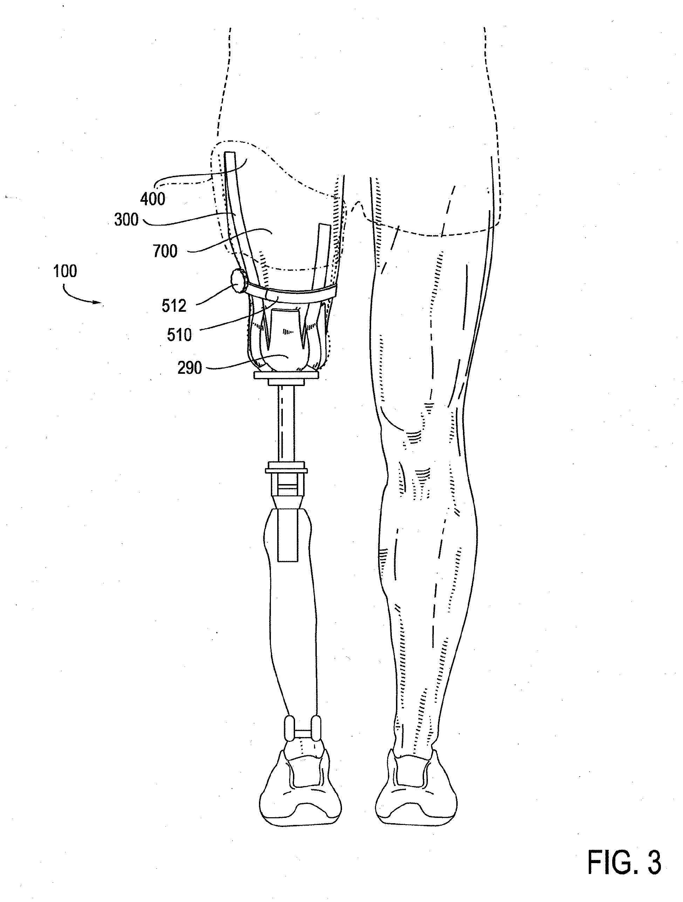

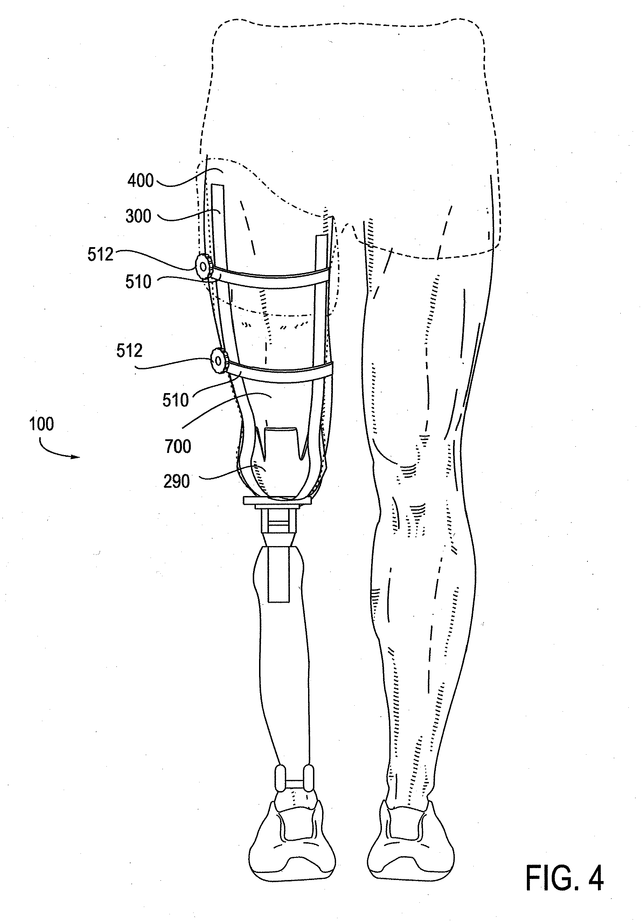

**Figs. 3** **and** **4** each show a patient wearing an embodiment of a modular prosthetic socket assembly on a residual limb **700,** as described herein. **Fig. 3** depicts a patient with an above-knee (transfemoral) amputation wearing an embodiment of a modular prosthetic socket suitable for this level of leg amputation. **Fig. 4** shows a patient with an at-the-knee (knee-disarticulation) amputation wearing an embodiment of a modular prosthetic socket suitable for this level of leg amputation. The modular prosthetic socket assemblies in the **Figs. 3** **and** **4** are similar for both levels of amputation, differing substantially only in the length of the struts **300** and in the number of tensioning mechanisms **510.** These particular embodiments of a modular prosthetic socket include a brim element **400** rendered transparently and with a dotted outline, which is one of several pressure-distributing elements described herein. Pressure distributing elements are typically attached to one or more struts, but may include attachments to a distal base **200** as well. The modular prosthetic assembly for the patient with the transfemoral amputation (**Fig. 3**) has relatively short struts, and is making use of a single tensioning mechanism. The modular prosthetic assembly for the patient with an at-the-knee amputation (**Fig. 3**) has long struts, and is making use of two tensioning mechanisms. **Figs. 3** **and** **4** both also show prosthetic elements distal to the socket, including one or more prosthetic joints, a lower prosthetic limb, and a prosthetic foot.

In some embodiments, the modular prosthetic sockets of **Fig. 3** and **Fig. 4** could have been drawn from the same inventory. Such an inventory would include struts **300** of different length, or struts that could be cut to different lengths, and a selection of tensioning mechanisms. With reference to methods of practicing the disclosed technology, such methods include assembling a selected set of longitudinal struts together with a selected base, wherein such components are drawn from a group or inventory of components that vary in size and/or shape. By such a method, prosthetic sockets may be assembled that fit patients as varied as those depicted in **Figs. 3** **and** **4****.**

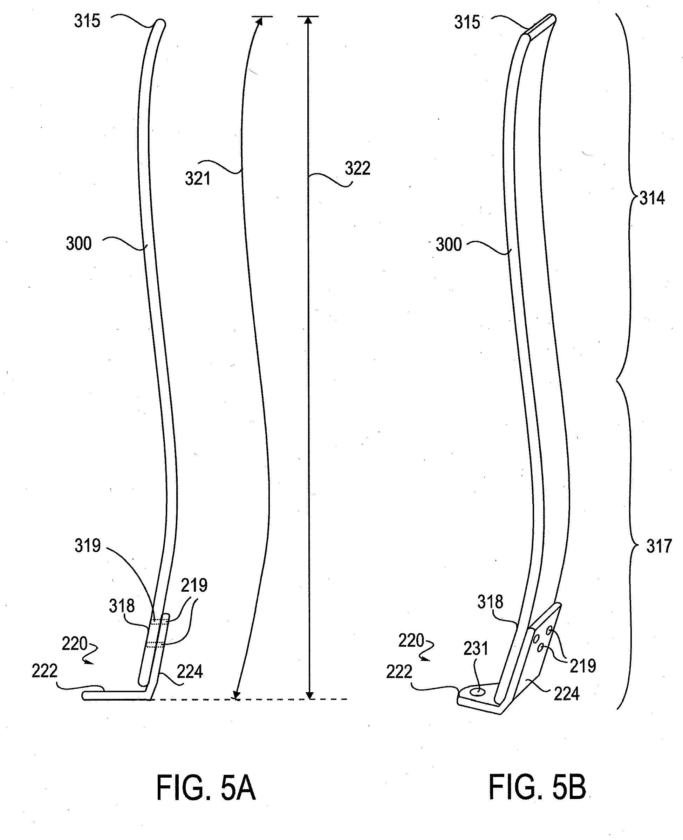

**Figs. 5A and 5b** show views of an embodiment of a thermoplastic-fiber composite strut **300** attached to a strut connector **220.** **Fig. 5A** is a side view; **Fig. 5B** is a perspective view. Strut **300** has a proximal portion **314** and a distal portion **317.** When incorporated into a fully assembled socket, the proximal and distal portions of the strut **300** are associated with the proximal portion **104** and the distal portion **105** of the socket as a whole, as seen in **Fig. 1****.** With continued reference to **Fig. 1****,** it can be seen that a strut connector **220** connects to distal base **200,** and that the distal portion of a strut may be associated with a pressure-distributing element such as a strut cap **430.** Generally, struts **300** are typically long and rectangular, having a length, a width, and a thickness, all of which may vary, and examples of which may be included in an inventory of struts. Inasmuch as struts **300** may also have sites of curvature, it can be useful to identify a total length **321** and a projected length **322.**

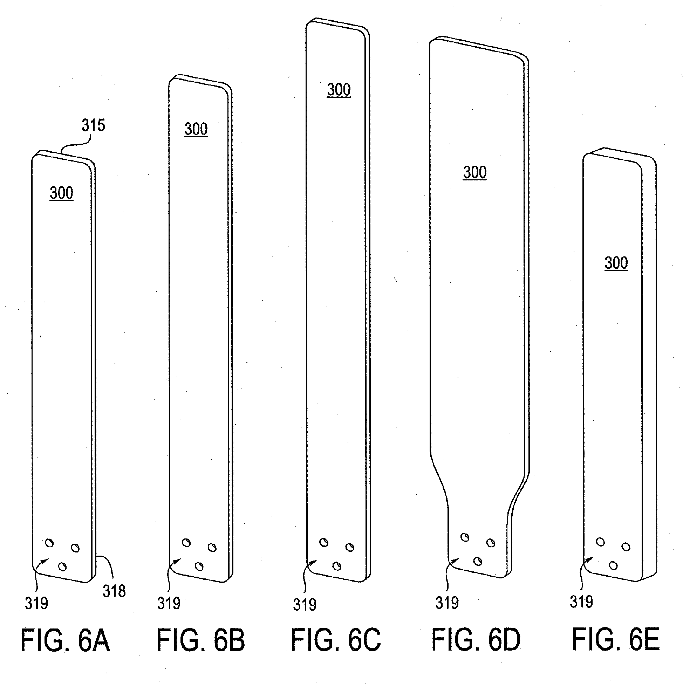

**Figs. 6A - 6E** show views of a series of thermoplastic-fiber composite struts **300** that are modular in character. Such a modular series may be found in an inventory or group of struts that vary in size and/or shape but nevertheless have attachment features **319** in common that are used for their assembly into a complete modular prosthetic socket **100,** as seen in **Fig. 1****.** The attachment sites **319** are mateable with attachment sites **319** that all strut connectors **220** have. (Strut connectors **220** are described in further detail below, particularly in the context of **Figs. 16A - 16I****.**)

Struts can be dimensionally characterized by length, width, and thickness. **Struts 300** of **Figs 6A - 6C** have identical width and thickness, but vary in length. Strut **300** of **Fig. 6A** is relatively short, that of **Fig. 6B** is of medium length, and that of **Fig. 6C** is relatively long. Strut **300** of **Fig. 6D** has a length identical to that of **Fig. 6C****,** but is wider. Strut **300** of **Fig. 6E** differs from the other struts depicted by having greater thickness. In spite of these dimensional variations, all struts **300** of **Figs. 6A - 6E** have identical attachment sites **319,** by which the struts attach to a strut connector.

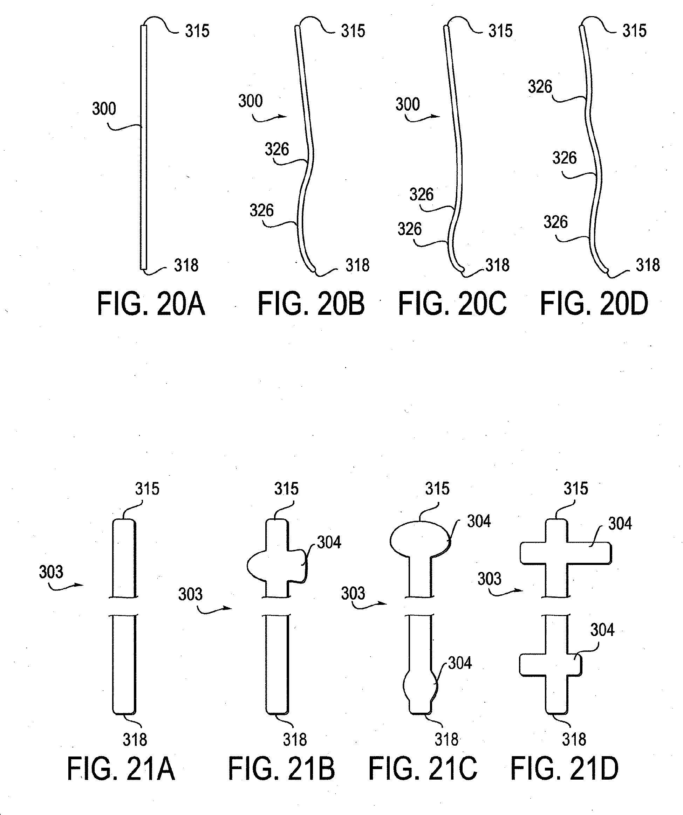

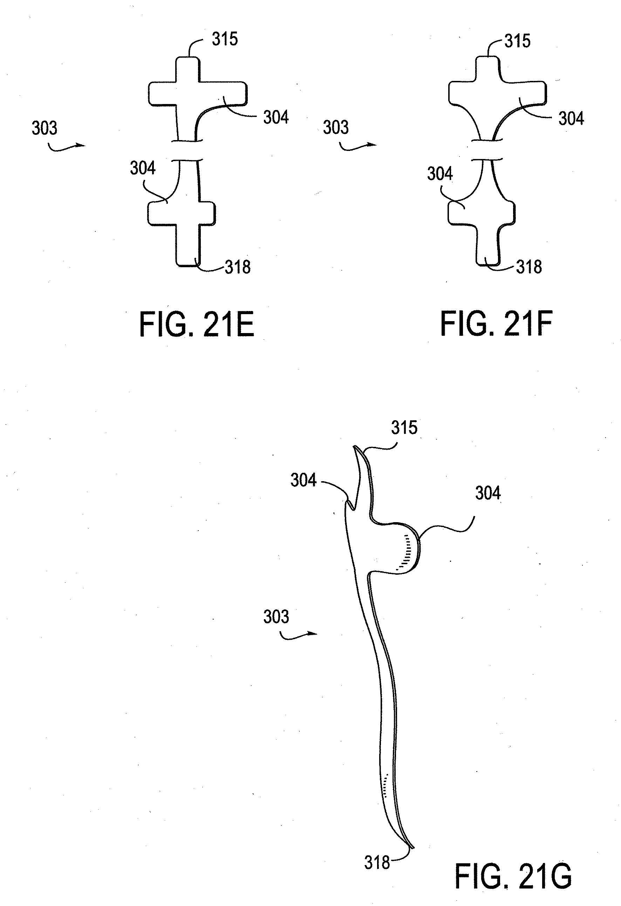

As alternative embodiments, struts **300** may include cut out portions or fork-like longitudinal cleavages (not shown). In still other embodiments, struts may include tabs that extend laterally (see **Figs. 21A - 21G**). Struts may further include embodiments wherein elements that can be separate modular components are integrated directly into a strut, such as pressure distributing elements, or a strut connector **220.**

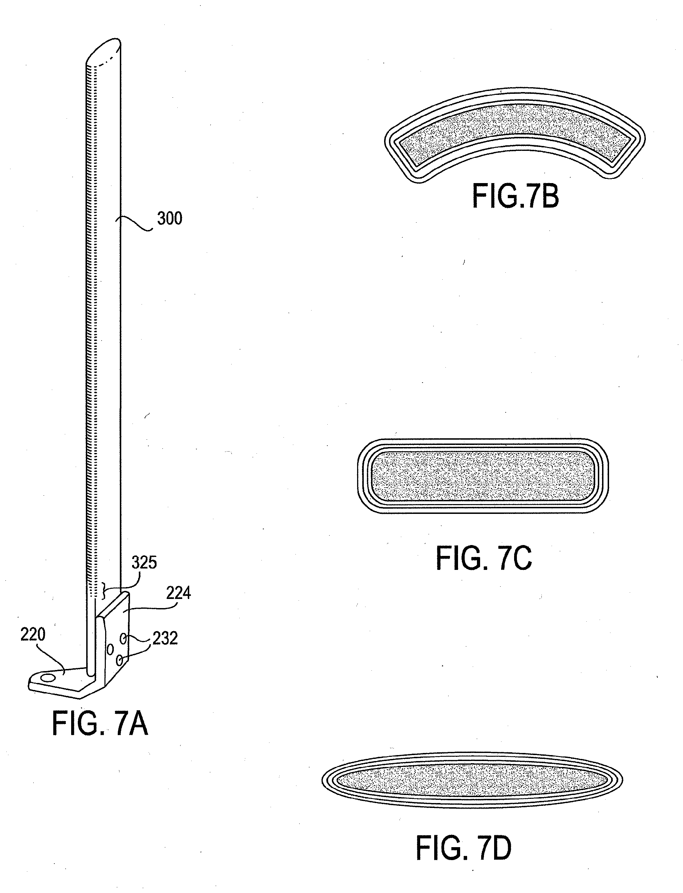

**Figs. 7A - 7D** show a thermoplastic-fiber composite strut embodiment **300** and cross sectional views of a various optional cross sectional profiles. **Fig. 7A** shows a perspective view of a strut **300;** **Figs. 7B - 7D** show example profiles such as inward-facing concave surface (**Fig. 7B**), a flat or rectangular cross section (**Fig. 7C**), and an oval cross section (**Fig. 7D**). These are non-limiting examples of many suitable cross sectional profiles. Modular components can vary in shape in addition to varying in dimension, while still maintaining common attachment features that allow them to be assembled with other components. The examples of variation in shape provided in **Figs. 7A - 7D** relate to variation in cross sectional profile that may occur in embodiments of struts **300.** **Fig. 7A** provides a perspective view of a strut **300** that is similar to that seen in **Fig. 5B****.** In typical strut embodiments **300,** the distal end **318** of a strut has a substantially flat or rectangular cross-sectional profile, as seen in **Fig. 7C****.** The corners of the cross-sectional profile can be beveled or rounded to varying degree. A flat profile in the distal region of a strut is generally advantageous as in that it is compatible with the flat surface of a strut connector **220** to which it is attached. Accordingly, if a strut embodiment has a cross sectional profile that is different (such as those seen in **Figs. 7B** or **7D**) from such a flat profile, the profile needs to change in a lofting point or region **325** to an oval cross sectional profile, shown here in a distal portion of strut **300.** Strut embodiments may have more than one loft region, where cross-sectional profile changes, and a loft region may occur at any point along the length of a strut.

These various cross-sectional profiles are merely examples of numerous variations in a strut cross-sectional profile. **Fig. 7B****,** for example, presents a concave inward-facing surface, but in a variation it could present a convex inward-facing surface. These various cross sectional profiles are typically provided to a strut during a forming process, whereby a forming mold has a cavity that imparts the cross-sectional profile. In other method embodiments, an alteration in an original cross-sectional profile, such as a rectangular profile, could be altered during a thermal reforming process, as described elsewhere herein.

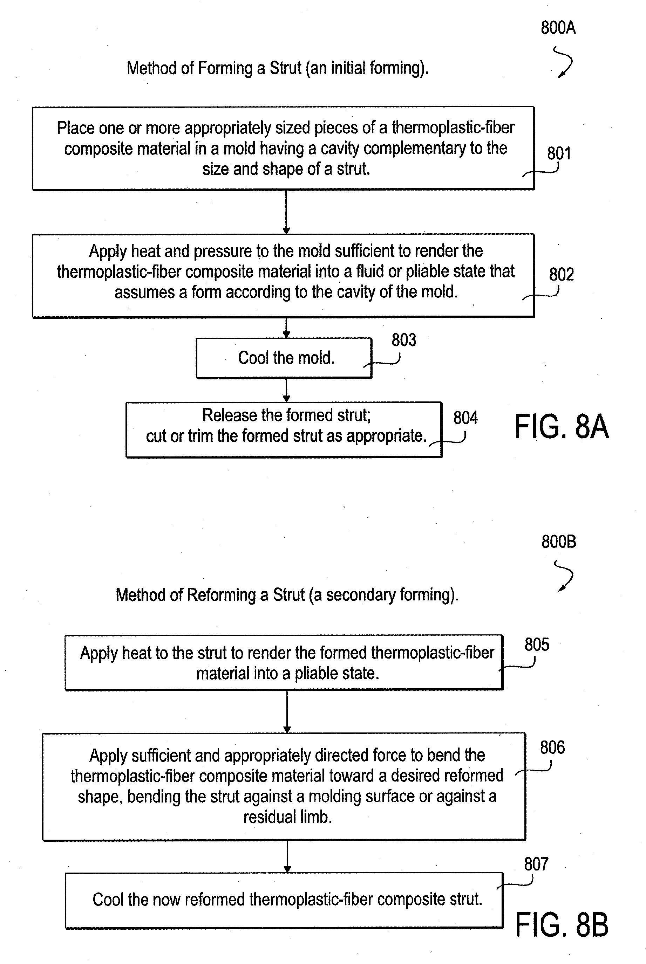

**Figs. 8A** and **8B** areflow diagrams illustrating a method for forming 800A (**Fig. 8A**) and reforming 800B (**Fig. 8B**) a thermoplastic-fiber composite strut **300.** As described in detail above, thermoplastics, when subjected to sufficient heat and with optional application of sufficient pressure, become fluidized or malleable or pliable to the extent that separate thermoplastic layers can anneal, bond, or comingle, and the overall form of bulk materials can take on a form complementary to a cavity of mold in which in which the bulk or starting materials are placed. A particular feature of thermoplastics, in contrast to thermoset plastics, is that the heat and pressure driven process can be repeated indefinitely without damaging or stressing an article. In this disclosure, a thermal forming process or method refers to an initial process, whereby bulk material or materials are first thermally rendered into an integrated article. Any secondary thermal forming process is referred to as a reforming process. In general, the conditions of heat and pressure that are suitable for an initial forming process are suitable for a reforming process. In some instances, a lower temperature or a lesser amount of heat input per unit material may be suitable in a reforming process as compared to an initial forming process.

In one embodiment, a method for forming a thermoplastic-fiber composite strut **800A** may begin by placing one or more appropriately sized pieces of a thermoplastic-fiber composite material in a mold having a cavity complementary to the size and shape of a strut 801. The method continues by applying an amount of heat and pressure to the mold sufficient to render the thermoplastic-fiber composite material into a fluid or pliable state that the material assumes a form according to the cavity of the mold, such heat and pressure being maintained for a sufficient duration 802. The method typically concludes by cooling the mold (either by passive or active cooling) 803, and releasing the formed strut from the mold. In some instances, the formed strut may need to be cut or trimmed 804.

Referring now to Fig. 8B, in some embodiments, a formed strut **300** may be subjected to a thermal reforming process 800B (or "secondary forming process"). In alternative embodiments, reforming may occur immediately or soon after a forming process, thus being essentially an extension of the forming process itself, or it may occur at any subsequent time as a completely separate process. In one embodiment, a reforming method may begin by applying heat to the formed strut to render the thermoplastic-fiber material of the strut into a pliable state 805. In some instances, pressure may be advantageously applied as well, to accelerate or help evenly distribute heating. The method continues by applying sufficient and appropriately directed force to bend the thermoplastic-fiber composite material toward a desired reformed shape 806. This application of force may include bending the strut against a molding surface, or against a residual limb, as in a direct molding process. A final step includes cooling the now reformed thermoplastic-fiber composite strut 807. Cooling may be passive or active. In the event that a first attempt to move the shape of a formed strut to a desired shape is not satisfactory, the method may be repeated.

As described in the summary section, an initial forming process may be commonly referred to as molding, inasmuch forming typically occurs within or against a molding surface. If a mold has a fully contained cavity, physical pressure can be applied to the mold. In other examples, a mold need not be fully enclosed, in which case a vacuum may be applied to draw moldable materials against a mold surface. In some particular method embodiments that are applicable to making thermoplastic-fiber composite struts, as described herein, fully enclosed molds are used, and both heat and pressure are applied to the mold.

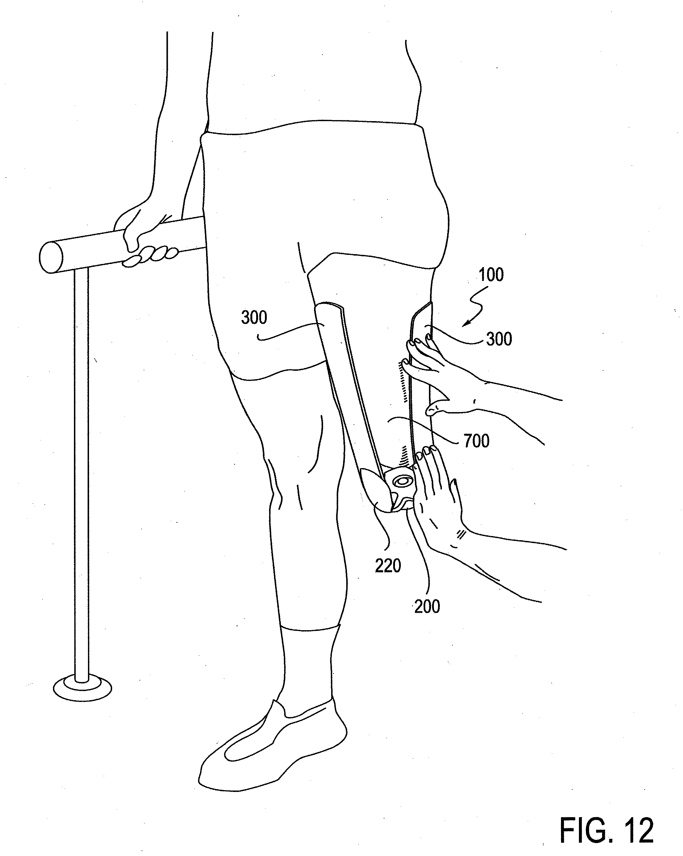

A mold may be used in a reforming process, but more typically, a heated strut may be manually bent against a molding surface or object. In some embodiments, a molding surface may be any suitable inanimate surface. Remolding is typically done in order to individualize a strut, accordingly there is typical no mold, and the bending is an ad hoc event, fully in the hands of the individual prosthetist, working with an individual patient. In some embodiments, accordingly, a portion of a residual limb of a patient may serve as a mold. In the prosthetic arts, this is referred to as "direct molding" and is described further below in the context of **Fig. 12****.**

Thermal forming 800 and reforming 810 of the thermoplastic-fiber composite material of struts **300** is a process largely dependent on the physicochemical attributes of the thermoplastic portion of the composite material. However, the embedded fiber portion of the composite material is also a factor in the methods; to some extent the fiber plays a constraining role. In many embodiments the fiber included in the thermoplastic-fiber composite materials of the struts is in a continuous or substantially continuous form. Fibers, such as carbon or glass fibers, are substantially non-stretchable and non-compressible; accordingly, the freedom of reformability of an article containing them is constrained. Such constraints would manifest, for example, if a reforming of broad surfaces of thermoplastic-fiber composite material to create complex contours were to be attempted. Whereas the thermoplastic portion of a composite material is highly amenable to reshaping that involves sites of stretch or compression, the continuous fiber resists such reshaping if it creates sites of stretching or compression.

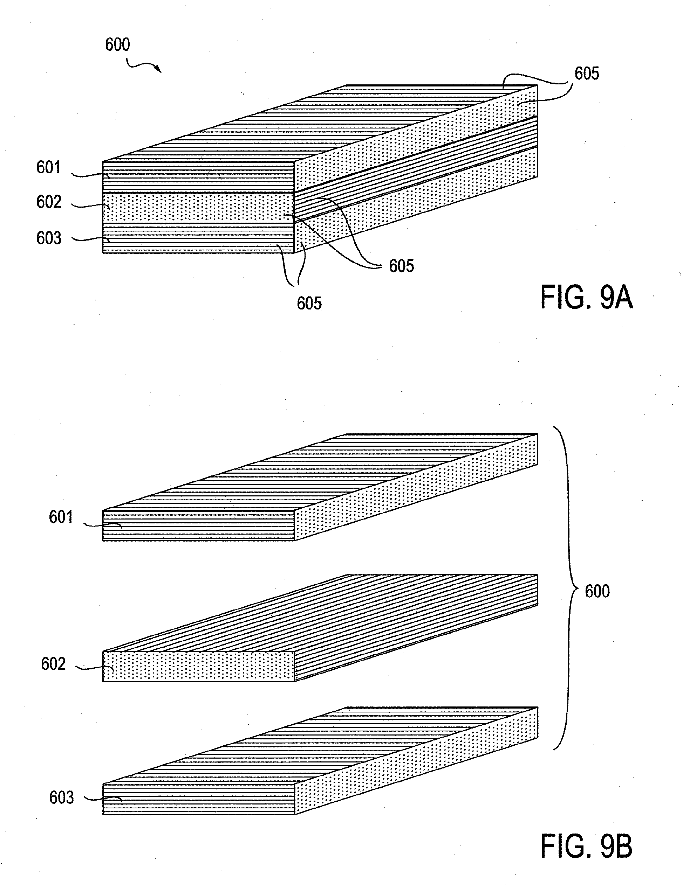

**Figs. 9A** - **11B** show aspects of forming and reforming thermoplastic-fiber composite struts **300,** as outlined in **Figs. 8A and 8B****.** **Figs. 9A and 9B** illustrate a section of a thermoplastic-fiber composite material of a strut; **Fig. 9A** shows a 3-layered strut section **600,** and **Fig. 9B** shows the layers separate, as they would have been prior to molding them together. The example depicted shows a material with three thermoplastic fiber composite layers (**601, 602, 603)** each layer having embedded continuous fiber. In each of these layers, the population of fibers embedded therein is wholly parallel. In other embodiments, fiber may be arranged in alternative patterns. In this particular embodiment, layers **(601, 602, 603)** are arranged such that they alternate with regard to the orientation of their embedded fibers, the fiber of each layer being orthogonal to the fiber of their neighboring layer. These figures generally draw attention to the presence of fiber within the thermoplastic matrix of materials included within the struts. In some embodiments, the struts may be substantially formed, in their entirety, from thermoplastic fiber composite material. In general, the processes associated with forming and molding thermoplastic-fiber articles are well known in the art. Aspects of the technology described herein that manifests as prosthetic socket structural component draws freely from any thermoplastic-fiber molding process that is known. Simple examples are provided in order to convey basic aspects of the technology, albeit directed specifically toward making thermoplastic-fiber (continuous fiber) composite components for a modular prosthetic socket.

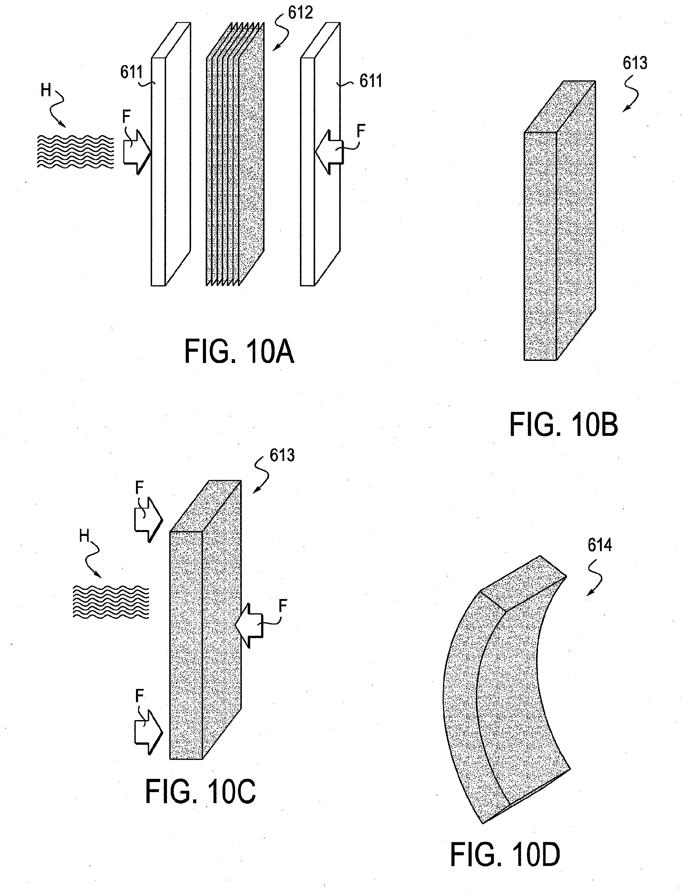

**Figs. 10A** - **10D** show a schematic views of aspects of forming and reforming thermoplastic fiber composite portions of struts. In **Fig. 10A****,** several layers of a bulk form thermoplastic material **612** are shown disposed within walls of a mold **611.** With application of heat **H** and pressure or force **F,** the original separate layers are integrated into a single article, such as a strut portion **613** for a modular prosthetic socket system, as in **Fig. 10B****.**

In a reforming process, strut portion **613** is being subjected to heat **H** and force **F.** The heat is conveyed by a temperature that is sufficiently high, and of sufficient duration that the strut becomes pliable or malleable. In that state, amenable to reforming, a force of sufficient strength, and appropriately directed, is applied to the strut such that the shape moves toward a desired form **614,** such as that depicted in **Fig. 10D****.**

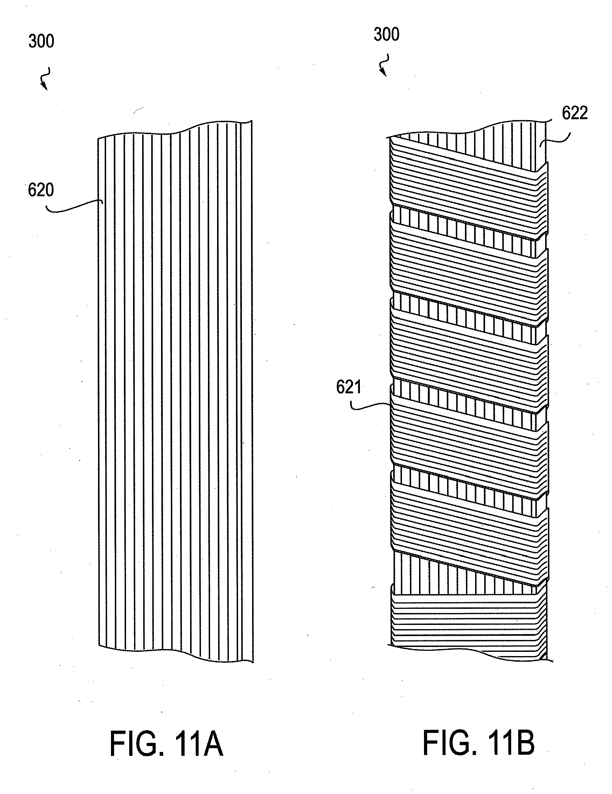

**Figs. 11A and 11B** illustrate another embodiment of a method of forming a multilayered thermoplastic-fiber composite strut **300.** **Fig. 11A** shows a section of a multilayered strut and **Fig. 11B** shows the strut as a circumferentially wrapped layer of thermoplastic-fiber composite tape is being applied to the strut. Some embodiments of thermoplastic fiber composite struts include layers of raw bulk material. These may be sections of bulk tape, or cut sections of larger sheets. Such laminated structures are known to have a vulnerability to delamination when stressed. Such a multi-layered thermoplastic fiber composite strut portion **620** is shown in **Fig. 11A****.** One approach to stabilizing the integrity of annealed layers under stress includes wrapping the multilayered structure circumferentially with a bulk form thermoplastic-fiber composite tape **621.** Wrapping could be included in an initial forming process. Alternatively, a strut **620** could be formed, subsequently wrapped circumferentially with one or more layers of bulk material tape, and then subjected to sufficient heat that the strut emerges, reformed, as an integrated thermoplastic fiber composite strut portion **622,** as in **Fig. 11B****.**

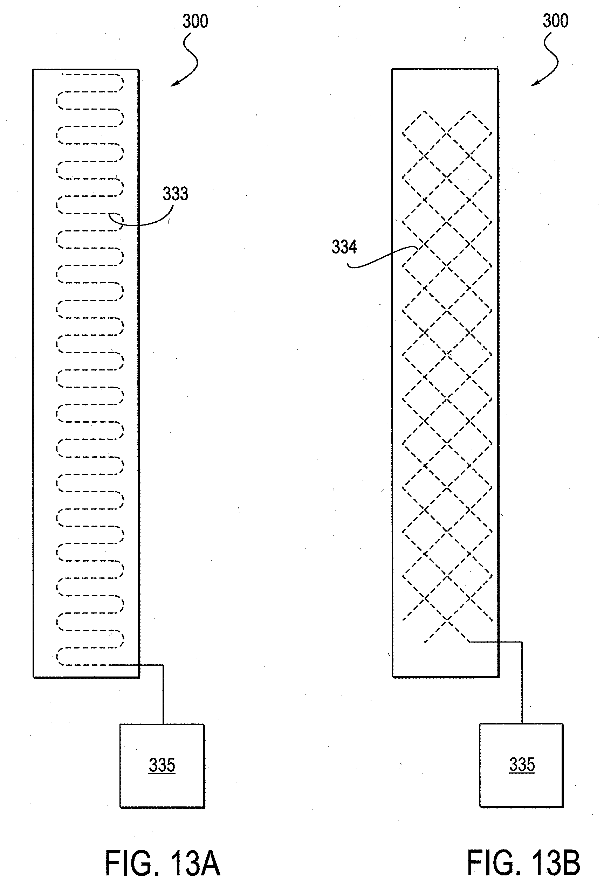

**Fig. 12** shows an aspect of reforming a thermoplastic-fiber composite strut known as "direct molding". In this embodiment, strut **300** has been heated sufficiently to make it pliable, and then is placed against the body (with an intervening thermal protective layer) and pressed against a portion of the residual limb of a patient to impart a body-conforming shape to the strut. Heating may be accomplished by any of several approaches. For example, a strut may be heated externally by a heater, such as a hairdryer, or a strut can be heated in an oven. In a particular embodiment, heating may occur through the use of a built-in resistive heating system, as described further below in the context of **Figs. 13A and 13B****.**

Strut **300** is shown as still being included within an assembled, or partially assembled, modular prosthetic socket **100.** In alternative instances, strut **300** may be removed from socket 100 prior to this direct molding method. In other embodiments, it may be advantageous to reform the strut 300 when it is included within an assembled or partially assembled modular prosthetic socket 100.

It is often a part of the method to protect the residual limb with an intervening thermally protective layer. Another aspect of the method may involve selecting a position for the strut **300** in a desirable location on the residual limb for the reforming process. Toward this end, the method may further include a step of marking the residual limb or an overlying liner with an outline of where the strut contacts the body, and then making use of that marking when placing the strut against the residual limb.

Still another aspect of the method may include focusing the heating step on a particular area within the strut **300,** in contrast to a method where the strut is heated with substantially equivalent heat throughout the structure of the strut. Focusing the heat on a particular region of the strut can be a helpful aspect of the method, wherein the prosthetist is intending to reform the strut.

**Figs. 13A and 13B** show embodiments of a thermoplastic-fiber composite strut **300** with a resistive heating element 333, 334 embedded therein, thus allowing the strut 300 to be self-heating for thermal reforming. **Fig. 13A** shows a strut with a serpentine heating element **333;** **Fig. 13B** shows a strut with a mesh heating element **334.** Each strut 300 may further include a connection to a power supply **335.** A typical use of a built-in resistive heating element 333, 334 is to warm the strut 300 to pliability prior to a reforming process, such process undertaken to effect a change in the shape of the strut. In some particular embodiments, the resistive heating elements 333, 334 are arranged such that heat can be directed to particular regions of the strut, while other portions remain unheated. Some embodiments of a modular prosthetic socket may include sensors and microprocessors in communication with strut elements, such sensors and microprocessors able to facilitate or automate aspects of controlling a thermal reforming process.

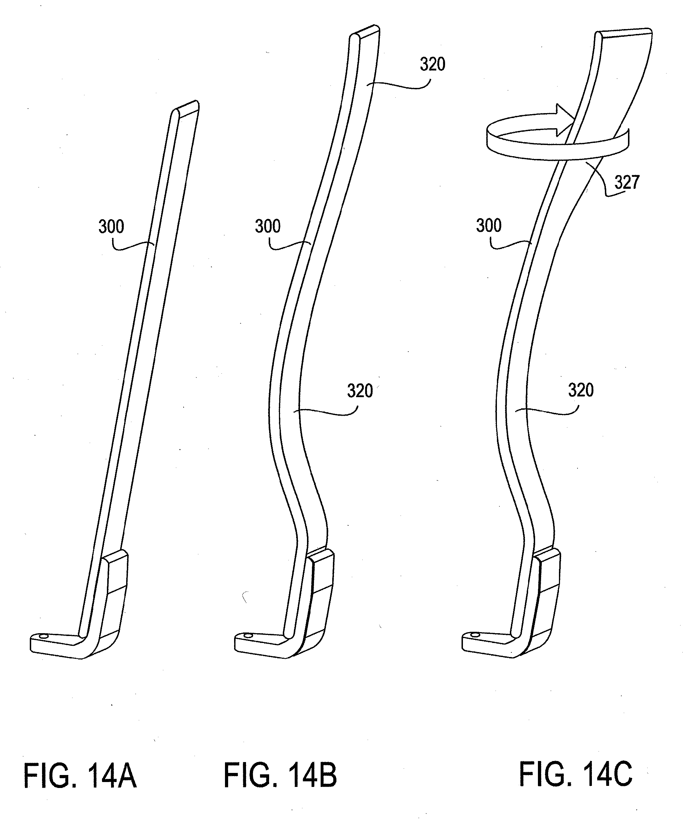

**Figs. 14A - 14C** show an embodiment of a thermoplastic-fiber composite strut **300** in an initial state, as it was originally formed, and two examples of the strut after being thermally reformed to better fit against a portion of the residual limb. **Fig. 14A** shows a strut **300** in its originally formed shape.The shape as shown here is substantially flat and advantageous for requiring a simple mold, and a good neutral starting shape that can be later reformed toward a desired shape for better fitting of a residual limb. **Fig. 14B** shows a strut **300** after it has been reformed to include a curve **320**. Curves 320 may be imparted at any point along the length a strut, and more than one curve 320 may be included. Multiple curves 320 can be imparted in a reforming process at the same time, or they can be added serially. **Fig. 14C** shows a strut **300** after it has been reformed to include a twist **327** of several degrees. Twists **327,** as imparted by a reforming process, may be advantageous for the fitting of modular prosthetic sockets to particular residual limbs.

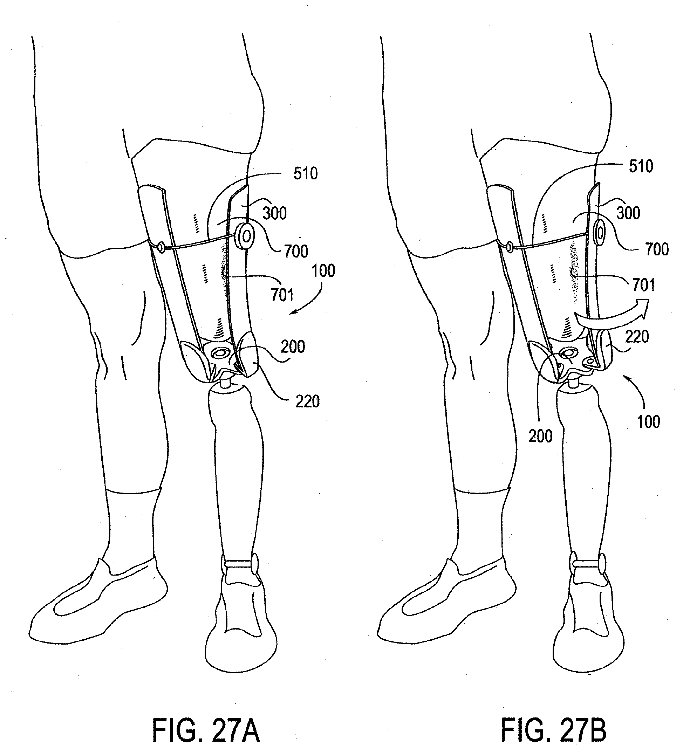

Reforming of modular prosthetic components such as struts **300** may be performed to improve fitting of a prosthetic socket to a residual limb, in any aspect of fitting as described herein. Fitting may include biomechanical considerations that are not apparent in an approach that adheres strictly to fitting in a conformal sense. Another example where reforming to alter strut shape may be advantageous involves providing relief at a site of irritation or injury on a residual limb. In some instances, the struts and the socket may fit well overall, but one strut nevertheless creates irritation or is coincidentally positioned near such a site. In this case, a small reformation, moving a section of a strut a few millimeters outward for example, may be very helpful clinically. Another example of providing relief for a site of irritation is depicted in **Figs. 27A and 27B****,** which includes a mechanical adjustment of a modular prosthetic socket. Methods of forming and reforming are described elsewhere in detail, and depicted schematically in **Figs. 8A - 12****.**

**Figs. 15A** - **15G** show views of embodiments of a distal base **200** and **201** for a modular prosthetic socket **100,** each having four strut connecting sites (or strut connector connecting sites), as represented by radial slots **212.** Four is a typical number of strut connecting sites on a distal base. In other embodiments, not shown, distal bases may include either fewer or more than four strut connecting sites. **Figs. 15A and 15B** show a distal base embodiment **201** wherein strut connecting sites are distributed in an arrangement with intervening angles of about 120°, 90°, 60°, and 90°. **Figs. 15C** - **15F** show distal base embodiments **200** wherein the strut connecting sites are equally distributed at about 90°. Distal base embodiments may include strut connecting sites circumferentially distributed in any clinically suitable arrangement; the examples provided here by distal bases **200** and **201** are merely non-limiting examples. The 120° angular spacing between neighboring struts (distal base embodiment **201**) is an example of a relatively wide spacing between struts that may be positioned within a complete socket such that when being worn by a patient, the wide spacing occurs on a medial aspect of the residual leg, this being advantageous for minimizing interference with the opposite intact leg. Fastening elements **219** are shown; these are typically threaded bolts so as to facilitate both assembly and disassembly.

**Figs. 15C** - **15E** show distal bases **200** that are identical in shape, but vary in size (the base in **Fig. 15C** is large, that in **Fig. 15D** is medium, and that in **Fig. 15E** is small). Any distal base, including embodiments **200** and **201** may include more than one component plate, the plates typically cooperating structurally and functionally.

Such variations in size and shape may be included in an inventory of distal base components, as are included in systems and kits of modular prosthetic sockets. Inventories of components from basic components groups (such as struts and distal bases, as well as other components) are included in embodiments of the technology such as modular prosthetic socket systems and kits. These inventories provide a supply of modular components that vary in size and/or shape, but nevertheless maintain commonality at attachment sites for connecting to other components. Modular component inventories are described elsewhere herein; briefly, they are simply an available selection of groups of components. An inventory of distal bases would have a supply of distal bases of different sizes and shapes that could be drawn from in order to assembly a complete modular prosthetic socket **100.** **Fig. 15G** shows an embodiment of a distal base for a modular prosthetic socket having a proximal plate **215** arranged over a base plate **200** that includes strut connecting slots **212.**

Proximal plate **215** has four surface features **217** within the distal base near each of the slots for limiting pivoting movement of the strut connectors in the slots. In the example shown in **Fig. 15G** and elsewhere, such surface features include one or more indented portions **217** in upper or proximal plate **215.** Other arrangements on distal base **200,** such as surface features on the base **200** that similarly provide the same type of containment of swivel or pivot are included within the scope of the technology. These indented portions **217** that expose the portions of the lower base plate that include radial slots **212.** The exposed area defined by indented portions **217** and the radial slots **212** cooperate to form a strut-connector connecting or attachment site. Radial slots **212** host strut connectors in such a manner that the connectors can radially slide in and out, and pivot or swivel at any point along the line. Sliding and pivoting are allowed when bolts **219** are loose; sliding and pivoting are disallowed when bolts are tight, thereby creating a friction lock between the base of a strut connector and an upper surface of the base plate to which the strut connector is connected. The indented portions **217** of the proximal plate **215** provide a boundary of the arc in which strut connectors can pivot or swivel. Indented portions **217** may be considered an example of any feature on a distal plate that limits the pivot or swivel of a strut connector **220.** Strut connectors are described in detail below.

**Figs. 16A** - **16I** show views of various embodiments of strut connectors **220A, 220B, 220C** for a modular prosthetic socket. Strut connectors will be generically referred to as strut connector **220,** embracing all of the variations, such as **220A (****Figs 16A-16C****), 220B (****Figs. 16D-****16E),** and **220C (****Figs. 16G-16H****).** All strut connectors **220** have a base portion **222** and a back or "takeoff" portion **224.** Strut connectors **220** connect struts to a distal base 200 (Figs. 15A - 15G), having an attachment hole **231** for the distal base on base portion **222** and attachment holes **232** for a strut on the back portion **224.**

Strut connectors 220 are also modular, in that they can have different shapes, but maintain common connecting features for the struts and the distal base. An inventory of components from which to assemble a modular prosthetic socket may include strut connectors of varying shape, such variable aspects manifesting in the assembled socket primarily in the shape of the proximal portion of the socket. Any of the dimensions of a strut connector can vary, including the width, the length of the base portion **222,** and the length or height of the back portion **224.** As discussed below, the angle **226** (226-R, 226-O) disposed between the base and back portions (the "takeoff" angle) may also vary.

In various embodiments of a modular prosthetic socket assembly, strut connectors **220** are a significant determinant of the shape of the socket 100, particularly in its distal portion **105** (see **Fig. 1**), proximate the distal base. In addition to effecting shape or distal cross-sectional profile of an assembled socket, the strut connectors 220 provide a significant level of adjustability in the overall cross-sectional profile and volume of the socket. Aspects of these functionalities are described further below, and shown in **Figs. 17A - 18B****,** and **Figs. 26A - 26B****.**

**Figs. 16A - 16C** show a strut connector embodiment **220A** having a single attachment hole **228** at the vertex of a triangular base. **Figs. 16A and 16B** show an embodiment wherein a connector backside **224** is at right angle to a horizontal base **222.** The angle between the base portion **222** and the back side **224** of a strut connector **220** is referred to as a "takeoff angle **226 (.** Takeoff angle, in addition to referencing the base portion of the socket, also relates directly to the angle of a distal portion 317 of the strut **300** with respect to the central longitudinal axis **101** of a modular prosthetic socket as a whole (**Fig. 1**). The smallest takeoff angle **226** is approximately 90° with respect to the base portion of a socket connector and the distal base as a whole; this minimal angle configures a strut connected to the connector such that it is parallel to the central longitudinal axis 101 of socket 100. Takeoff angles **226** may also be obtuse, for example, the takeoff angle 226-O shown in **Figs. 16C and 16F** are approximately 110° with respect to the base portion of a socket connector. Accordingly, takeoff angles **226** may vary from about 90° to about 160°. Such a variations in takeoff angle represents a modular aspect of strut connectors, as discussed above, and would manifest in strut connector inventories.

**Fig. 16C** shows an embodiment wherein the backside **224** resides at an obtuse takeoff angle **226-O** with respect to the horizontal base. **Figs. 16E - 16F** show an embodiment similar to those of **Fig 16A** - **C****,** this particular embodiment further having buttress supports **223** extending from the lateral edges of the backside **224** to the horizontal base **222.** **Figs. 16D and 16E** show an embodiment wherein a strut connector backside **224** is at right angle **226-R** to a horizontal base. **Fig. 16F** shows an embodiment wherein connector backside **224** resides at an obtuse angle **226-O** with respect to the horizontal base. **Figs. 16G** - **16H** show an alternative embodiment of a strut connector, this embodiment having two attachment sites **235** (twin slots within a distal base) and a rotatable disc **236** that inserts into circular receptacle **237.** Fasteners **239** connect to twin tracks in the base (not shown).

The takeoff angle **226** of strut connectors **220,** *i.e.,* the angle of back portion **224** with respect to base portion **222,** may be formed in alternative ways. In some embodiments, for example, a strut connector may have a 90° takeoff angle but further include an insertable triangular wedge (not shown) that fits into the front facing aspect of the strut connector, and which has a sloping face. In this manner, a variable takeoff angle for the strut connector, as a whole, is provided by wedges having a front facing slope of varying angle. This arrangement essentially transforms the profile of an embodiment such as seen in **Fig. 16B** into a profile such as that seen in **Fig. 16C****.** In this type of modular prosthetic socket system embodiment, accordingly, an inventory of modular components may include wedges that have varying front facing slopes but all nevertheless fit into a front facing aspect of a common strut connector.

**Figs. 17A - 17E** show top views of an embodiment of a distal base **200** for a modular prosthetic socket with the strut connectors **220** positioned on the distal base into configurations that vary according to the radial position (ranging between close to the center of the base and close to the periphery of the base) of the strut connectors and their degree of pivoting at their attachment site. The attachment site of the strut connectors on the base is largely obscured by the strut connectors **220,** themselves, in these views, but includes radial slots **212,** some of which are partially visible in **Figs. 17B- 17E****.** Struts **300** are connected to the strut connectors, but only visible in a narrow cross sectional profile as they are projecting forward from the base that is seen in a top facing view.

The embodiment of a distal base **seen in** **Figs. 17A - 17E** accommodates four strut connectors **220,** circumferentially spaced apart at 90° intervals. **Fig. 17A** shows four strut connectors **220,** each of the four positioned at a minimal radial position on base **200** within radial slots (not visible). **Fig. 17B** shows four strut connectors **220,** each of the four positioned at a maximal radial position; with the strut connectors moved to their maximal radial position, the inner portion of radial slots **212** become visible. **Fig. 17C** shows four strut connectors **220,** with one of the four (shown on the right) positioned at a maximal radial position (and a portion of radial slot **212** thus visible), the other three strut connectors being at a minimal radial position. **Fig. 17D** shows four strut connectors **220** arranged as in **Fig. 17C****,** but with the strut connector on the right pivoted clockwise at an angle **A** of about 20°. **Fig. 17E** shows four strut connectors arranged as in **Fig. 17C****,** except that strut connector **220** on the left, still in its minimal radial position, is pivoted counterclockwise at an angle **A** of about 20°.

**Fig. 17F** shows detail of how a friction locking mechanism controls movement of the strut connectors **220** with respect to the distal base. **Fig. 17F** is a cross sectional side view of a distal base **200** for a modular prosthetic socket with a strut connector **220** positioned thereon, within a radial slot **212.** Strut **300** is attached to strut connector **220.** A fastener or fastening element **219** such as a threaded bolt extends through aligned holes in the distal base and the strut connector. This arrangement represents a friction locking mechanism. When bolt **219** is tightened against nut **279,** drawing the base of the strut connector against the upper surface of the distal base, the strut connector cannot slide or rotate. When the bolt is loosened, typically by a prosthetist or shop technician, the strut connector is free to slide and rotate within the slot.

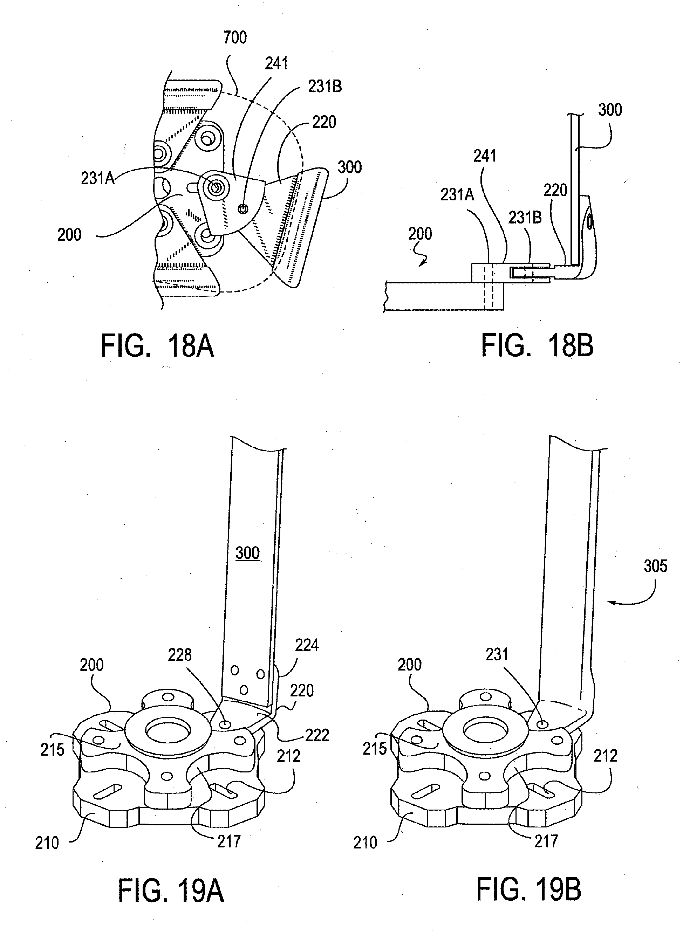

**Figs. 18A and 18B** show, respectively, top and side views of an embodiment of a strut connector **220** that includes an intermediate linking pivotable element **241** disposed between base **200** and the main connector body **220.** Intermediate connector element **241** attaches to base **200** by way of threaded bolt (not shown) through a first hole **231a.** As seen in cross sectional side view (**Fig. 18B**), intermediate connector element **241** has a lateral slot that accommodates an internally projecting piece of strut connector **220.** A second hole **231b** through both the intermediate connector element and the internally projecting piece of connector **220** can host a bolt connecting the two pieces together. When the bolt is loose, strut connector **220** can pivot with respect to the intermediate connector element **241.** **Fig. 18A** shows the function of this second pivoting mechanism. A cross sectional profile of a distal aspect of residual limb **700** is shown. Were it not for the second pivoting mechanism, as a consequence of the pivoting around connecting hole **213a,** the internal face of strut **300** would not remain tangent to the surface of the residual limb **700.** By virtue of the pivotability around hole **231B,** the internal face of strut **300** can be maintained or restored such that it aligns tangentially with the surface of residual limb **700.**

It may be appreciated that the arrangement of strut connectors **220** with respect to distal base **200** provides at least two levels of structural adjustment that project proximally by way of struts **300,** and which have implications for the size and shape of the internal proximally open space defined collectively by the struts, distal base, and any pressure distributing elements that may be present. Further, the pivotability of the strut connectors **220** within radial slots **212** of the distal base **200** provide an adjustability with regard to the circumferential distribution of struts around the distal base, or around the central longitudinal axis **101** of a socket **100** as a whole. These aspects of the technology are described further below, in the context of **Figs. 26A** - **27B****.**

**Figs. 19A and 19B** show an embodiment of a strut **300** and an alternative embodiment **305** for inclusion in a modular prosthetic socket as described herein, each embodiment attached to a distal base **200.** **Fig. 19A** shows a top perspective view of an embodiment of a distal base **200** similar to that of **Fig. 14G** with a with a single strut **300** attached thereto by way of a strut connector **220;** this view shows how a base plate **210** and a top plate **215** of base **200** can cooperate to provide a strut connecting site that further stabilizes the strut, and provides a boundary to the pivoting latitude. **Fig. 19B** shows an embodiment of a strut **305** with an integrated connector portion on its distal end.

**Figs. 20A** - **20D** show side views of thermoplastic-fiber composite struts **300** that have a varying side profile, ranging from substantially straight to having two or more sites of curvature. Each strut has a proximal end **315** and a distal end **318.** The strut **300** of **Fig. 20A** is straight, having no significant curvature. The strut **300** of **Fig. 20B** has a two sites of curvature **326** in its distal portion. The strut **300** of **Fig. 20C** also has a two sites of curvature **326** in its distal portion, but the sites of curvature are spaced more closely together than those seen in **Fig. 20B****.** The strut **300** of **Fig. 20D** has three sites of curvature **326** distributed through its length.

These various configurational variations of thermoplastic-fiber composite struts **300 (****Figs. 20A** - **20D****)** may be the result of at least two method processes. In one example, these variations could represent initial forms of struts, directly from a mold, each being the product of a thermal forming process. In such an instance, struts as seen in **Figs. 20A** - **20D** could all be stocked in an inventory or collection of modular strut forms. In another example, the straight strut **(****Fig. 20A****)** could be strut delivered directly from a mold, a product of a thermal forming process, and each of the struts in **Figs. 20B** - **20D** could be the products of a secondary thermal process, a reforming of a stock item in an inventory such as the strut seen in **Fig. 20A****.**

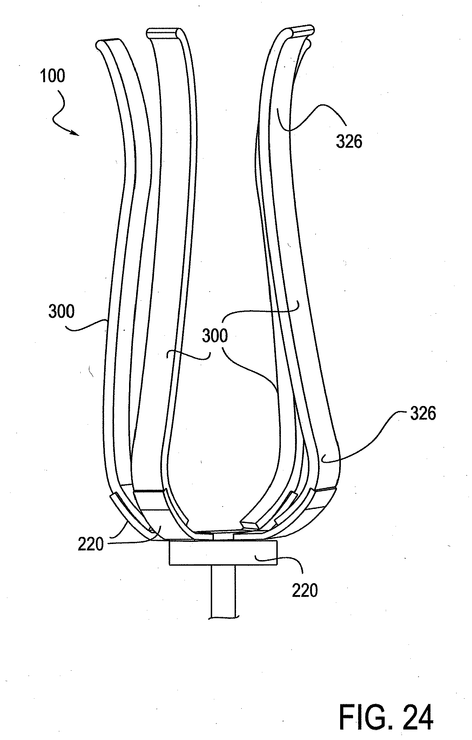

Further with regard to curvature in struts **300,** as noted, struts emerging from a mold could be considered modular variants that could be included in a collection, a grouping, or an inventory. Sites of curvature can be characterized in several ways. For example, curves may be concave or convex with regard to their internal aspect, facing internally toward the central longitudinal axis of the strut. The directionality or bias of curves may commonly alternate along a length of a strut, for example, a convex curve following a concave curve, to form an S-shaped curve. An example of such S-shaped curvature is seen in **Fig. 24****.** Curves may further be characterized with regard to their relative acuity or obtuseness, the degree of angulation per unit length. Other examples of curved struts are provided in **Figs. 15B****,** **14C****,** **21G****,** **22A and 22B****.**

**Figs. 21A** - **21G** show face views of thermoplastic-fiber composite struts **303** that differ from strut **300 by** having a proximal end **315** and a distal end **318,** and further having one or more tab-like features **304** extending laterally. Tabs **304** may be disposed either in the proximal portion of the strut and/or in the distal portion. They may serve one or more purposes. In one example, the tabs are, themselves, pressure-distributing elements, engaging in distributing pressure laterally away from the main linear body of a strut. In a second example, tabs **304** may serve as anchoring sites either for tensioning features, or larger pressure distribution elements. In a third example, strut tabs may serve as pressure deflecting elements that specifically distribute pressure away from a tensioning element that otherwise would press too hard against a site on the residual limb.

**Fig. 21A** shows a baseline example of a strut **300** unadorned with any tabs. **Fig. 21B** shows a baseline example of a strut **303,** with rounded tabs **304** on either side of a proximal portion of a strut, disposed at a point distal to the proximal end of the strut. **Fig. 21C** shows a baseline example of a strut **303,** with rounded tabs **304** positioned both at the proximal end of the strut, and in the distal portion, but short of the distal end. **Fig. 21D** shows a baseline example of a strut **303,** with rectangular tabs **304** disposed asymmetrically on either side a strut, and in both the proximal and distal portions of the strut. **Fig. 21E** shows a baseline example of a strut **303,** with modified rectangular tabs **304** disposed on either side of a strut, the modification of the rectangle including a rectangular corner assuming an arc of repose profile. **Fig. 21F** shows a baseline example of a strut **303,** with tabs **304** arranged in a manner similar to that seen in **Fig. 21F****,** with all centrally facing rectangular corners modified into an arc of repose profile.

There are several embodiments of methods of fabricating struts **303** that include tabs **304.** In some embodiments, the tabs are derived from the same or closely related thermoplastic-fiber composite material with which the main body of the strut is made. In some embodiments, the tab forms are included in a mold in which struts are initially formed. In some embodiments, the tabs are added to the main body of the strut after the strut has been made. Some of these methods include a thermal joining of the tab forms and a strut body such that the strut and associated tabs become an integrated article. In some embodiments of a prosthetic socket **100,** tabs 304 of neighboring struts may be joined by a tensioning element.

**Figs. 22A** - **22K** show numerous of various arrangements of thermoplastic struts **300** with strut caps **430** or brim elements **420** attached thereto, and typically configured such that the strut cap or brim element supports a circumferentially arranged tensionable element or member **510.** Tensioning elements may take any suitable form, such as a strap, or a cord, or lacing, and may be elastic or substantially non-elastic. Tensioning elements typically are arranged circumferentially around the socket, or they are more generally included in a system that ultimately applies inwardly directed force on the struts from a circumferential vantage. From such vantage, the struts are forced centrally, toward each other, generally narrowing a nominal circle that represents the cross sectional profile of the space included within the struts, collectively. A given tensioning element may be supported any one or more of the multiple longitudinal struts of a given modular prosthetic socket embodiment. Tensioning element embodiments are described further below in the context of **Figs. 23A** - **23C****.**

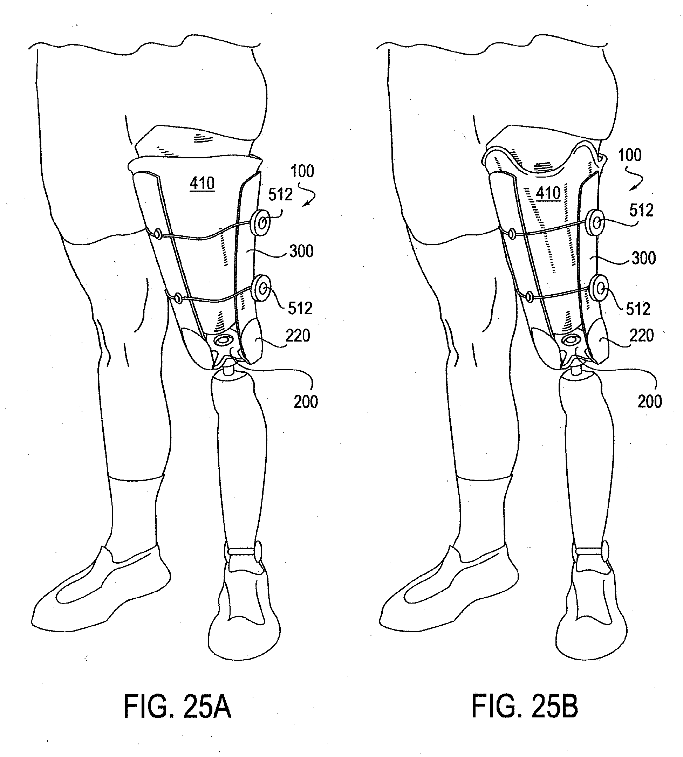

Embodiments of strut caps, brim elements, and a flexible inner liner are may all be understood as pressure distributing elements. Each element is configured to distribute pressure impinging on a residual limb away from sites where struts would otherwise focus concentrated pressure on the surface of a residual limb. Strut caps and brim elements differ primarily in their width, strut caps being generally narrow, brim elements being more expansive and more highly contoured. Strut caps and brim elements are typically fixed to one or more struts, and are positioned at or near the proximal ends of struts. A flexible inner liner **410** (as in **Figs. 25A and 25B**) is even more expansive, fully embracing the residual limb. All pressure-distributing elements have an internal aspect that faces toward the residual limb, and an external aspect facing outward.

**Figs. 22A and 22B** show a strut **300** and strut cap **430,** the strut cap configured slip over a distal end of the strut, and then being secured thereto. A belt-loop element **435,** typically on an exterior aspect of the strut cap, is configured to accommodate a tensioning element or member (not shown). **Figs. 22C and 22D** show an alternative configuration of a strut cap **430,** in which the strut cap has a wider lateral aspect, similar in shape and pressure distributing ability to the strut tabs **304,** as shown in **Figs. 21A** - **21G****.** Strut cap **430** in **Fig. 22C** has tensioning element holder or guide **435,** through which a tensioning element may be threaded. Strut cap **430** in **Fig. 22D** has tensioning element holder or guide **435',** that can be slipped over a circumferential tensioning element.

**Fig. 22C** shows an external aspect of a strut cap **430** with a tensioning element guide **435** that is configured to support a tension element such as a strap or a cord by having the element threaded therethrough like a belt through a belt loop. **Fig. 22D** shows an external aspect of a strut cap that is configured to support a tensionable element with a tensioning element holder **435** configured as clasping mechanism.

**Fig. 22E** shows a strut cap **430** with a hook and loop attachment arrangement that stabilizes a tensionable element across an external aspect of a strut cap. In this embodiment, a first mateable portion **437** of a hook and loop attachment on the strut cap **430** is configured to match up against a second mateable portion of a hook and loop attachment on tensioning guide **435.** This arrangement permits a stable coupling a strut cap and a tensioning element that can be easily repositioned.

**Fig. 22F** shows an arrangement in which a tensionable element **510** in the form of a wide strap is threaded through a tension-lockable belt looping mechanism **439** positioned bilaterally on either side of a tensioning element guide **435** that can slip over the end of a strut **300.**

**Fig 22G** shows a pressure-distributing element in the form of a brim element **420** that has two tensioning element guides **435** positioned on its external surface. This brim embodiment **420,** with raised upper lateral surfaces, is fitted over the end of strut **300.** **Fig. 22H** shows a strut with a belt-loop mechanism **435** integrated into an external aspect of the strut..

**Figs. 22I** and **22J** each show brim elements **420** arranged at the proximal ends of struts **300.** The embodiment of **Fig. 22I** shows an arrangement in which the proximal end of a strut is inserted into a pocket on the back aspect of a brim element **420.** **Fig. 22J** shows an arrangement in which strut **300** and brim element **420** are seamlessly integrated. The brim elements **420** of **Figs. 22I and 22J** both have a high profile all the way across the top or proximal edge of the brim; this configuration contrasts with the scooped central portion of the proximal edge of the brim **420** shown in **Fig. 22G****.**

**Fig. 22K** shows a fabric sleeve **360** fitted over a single strut **300** with bilateral attachments **435** suitable for attaching either to a tensioning member or an adjacent strut sleeve. A fabric sleeve may be placed over a strut and any associated pressure-distributing element. Sleeves may be advantageous for their "soft-good" character that provides an interface friendly to the residual limb, and which further may include attachment features that can support tensioning elements and/or fixed-length connections to other struts or other pressure-distributing elements. The corset-lace arrangement of tensioning element as seen in **Fig. 23C** is another example of a soft-good feature.

**Figs. 23A - 23C** show views of modular prosthetic socket embodiments, each with a different arrangement of pressure distributing elements, including strut caps and strut brims. Each embodiment may include a roll-on gel liner **110** (as in **Fig. 2**) but which is omitted in these views. **Figs. 23A - 23C** each show a prosthetic socket **100** having struts **300,** distal base **200,** and an embodiment of a distal cup **290.** They differ with regard to their respective arrangements of tensioning elements and pressure-distribution elements. **Fig. 23A** shows prosthetic socket **100** fitted with strut caps **430** as a pressure distributing element, and a tensioning band **510** and an adjustment mechanism **512** arranged circumferentially around the struts **300.** **Fig. 23B** shows prosthetic socket **100** fitted an integrated brim **420** as a pressure distributing element, and a tensioning band **510** and an adjustment mechanism **512** arranged circumferentially around the struts **300.**

**Fig. 23C** shows prosthetic socket **100** fitted with laceable corset **520** as a combined pressure distributing element and tensioning mechanism that is arranged circumferentially around the struts **300** or more generally within or proximate the circumference nominally defined by the struts. Tension adjustment mechanism **513** is shown at the proximal end of the lacing mechanism. In related embodiments, there may be more than one tensioning mechanism, allowing tensioning to independently adjustable in different longitudinal sections of the corset. This arrangement, as noted above, has a soft-goods character that is friendly to the residual limb. A corset **520** such as this is typically fabricated from fabric or leather, and may also be considered as a type of sleeve, or include specific aspects that act as sleeves, enclosing or wrapping the strut, or wrapping one of more pressure-distributing elements associated with a strut, such as a strut cap or brim element.

In the context of discussing tensioning elements, it may be understood that the struts **300,** longitudinally disposed, have a neutral radial position with respect to the central longitudinal axis of the socket **100** as a whole. Tensioning elements **510,** when applying tension, pull the struts inward. Resistance to such an inward pull is provided by the integrity of the distal attachment of the struts to the distal base **200,** and by the strength and resilience of the strut throughout its length. The thermoplastic-fiber composition of struts, as described herein, is a major structural factor underlying strut strength and resilience, as distributed with substantial uniformity throughout the length of the strut. The presence of a residual limb within the socket also will provide resistance to an inwardly directed tension on the struts. The socket, itself, has no integral structure that connects the struts in any portion proximal to the distal base **200** that resists circumferential compression of the struts. With regard to an outwardly directed tension or constraint on the radial position of the struts, outwardly directed tension is typically provided only by presence of the residual limb. Outwardly directed force is resisted by the same strength and resilience of the struts that resists radial compression, and by a tensioning element, if present on the modular prosthetic socket assembly.

Inwardly directed tensioning around a socket **100** tends to drive the struts **300** radially inward. Soft prosthetic socket elements such as a liner, and the contained residual limb will be compressed inward at regions of contact with the struts, but bulge or buckle outward at regions between strut contact regions. A residual limb has a cross sectional area at each point along its length. The struts in a neutral position (untensioned, and the socket not hosting a residual limb) that nominally defines a circle or near-circle with a cross sectional area. The degree of radial compression exerted by a prosthetic socket on a residual limb can be expressed by comparing the cross sectional area of the socket (struts in a neutral position) to the cross sectional area of the residual limb when it is not in the socket. Inventors have estimated that in typical embodiments of the modular prosthetic socket **100,** when fitted appropriately to a residual limb, the area of a circle described by the struts in a neutral position is not less than 75% of the corresponding cross sectional area of the residual limb. Ranging upward from there, in some embodiments, the cross sectional area of the socket with the struts in a neutral position is substantially equal to the corresponding cross sectional area of the residual limb. Accordingly, in some embodiments, the prosthetic socket itself exerts some degree of compression on the residual limb, and in other embodiments, compression, in substantial entirety, may be provided only by tensioning arrangements.

**Fig. 24** shows an embodiment of a modular prosthetic socket **100** that is particularly configured to accommodate a bulbous residual limb (not shown). Bulbous residual limbs are relatively common; these residual limbs have a distal portion that flares out from a narrower more proximal portion. These types of limbs are often difficult to fit with prior art prosthetic sockets. Embodiments of a modular prosthetic socket **100,** as described herein, can easily accommodate such a residual limb. The strut connectors **220** all have an obtuse takeoff angle **226,** shown here as being about 150°. In their distal portion, struts **300** have a site of curvature **326** with a concave inward facing aspect. In their proximal portion, struts **300** have a site of curvature **326** with an outwardly flaring aspect. In donning such a socket, a patient with a bulbous limb would encounter no difficulty spreading the struts to accommodate the bulbosity, and tensioning elements (not shown) could appropriately tighten the socket along the full length of the socket.

**Figs. 25A and 25B** show views of a patient wearing an embodiment of a modular prosthetic socket **100** arranged with a flexible inner liner **410** nested within the socket, and two tensioning elements **510,** one proximal and one distal. **Fig. 25A** shows the inner liner without tension being applied and **Fig. 25B** shows the liner in a tensioned state. Embodiments of flexible inner liner **410** are sufficiently flexible that they bend under sites of pressure. As the patient adjusts the tensioning elements **510** by way of tensioning adjuster **512,** the struts **300** are drawn closer to each other and more tightly around the residual limb. In response to inward pressure from the strut, the flexible inner liner buckles in slightly as the sites of contact with the struts, and bulges outward slightly in the inter-strut regions.

Adjusting tensioning elements around a socket **100** may also be understood as exercising a method of adjusting or improving the fit of a socket on a residual limb. Practice of this method of fitting or improving the fit of a socket is typically in the hands of the patient, unlike other mechanical adjustments that can be made on the socket, which are typically in the hands of a prosthetist or trained technician. Improving the fit by adjusting tension can be understood as creating greater comfort for the patient, but in a more critical interpretation, improving the fit can be understood as making the fit more biomechanically appropriate, and as improving the functionality of the socket.

**Figs. 26A and 26B** show views of an embodiment of a modular prosthetic socket **100** in two configurations; **Fig. 26A** shows a socket **100** with the struts **300** and strut connectors **220** positioned on a distal base **200** within a relatively small radius, while **Fig. 26B** shows the same socket **100** with the struts and strut connectors **220** positioned on a distal base within a relatively large radius. Four struts **300** are shown; the two struts in the foreground are truncated to expose a view into an internal space within the socket. Prior art devices typically are of fixed non-adjustable dimensions, particularly at their distal end. These figures show how, in contrast, embodiments of a modular prosthetic socket, as described herein, are eminently adjustable at their distal end. Further, such distal-based adjustability projects forward or proximally by way of the struts, thereby supporting an adjustable quality to the shape and volume of the socket as a whole.

The configurations of distal base **200** in **Figs. 26A and 26B** correspond to the configurations see in **Figs. 17A and 17B****,** respectively. In **Fig. 26A** all of strut connectors **220** are locked into their respective minimal radial position. They are as close to the center of the distal base **200** (or the socket in general) as they can be within the confines of radial slots **212.** In **Fig. 26B** all of strut connectors **220** are locked into their respective maximal radial position. They are as far from the center of the distal base as they can be within the confines of radial slots **212.**

A circle **331** is drawn at approximately a midpoint of the struts **300;** this circle **331** represents a cross sectional profile of the internal volume defined collectively by the struts **300** and the distal base **200.** It can be seen, according to the laws of geometry, that what may appear to be minor radial expansion of the strut connectors **220** (**Fig. 26A** *vs.* **Fig. 26B**) manifests as a significant expansion in cross sectional area, which would, in turn, translate into an even larger relative increase in volume.

In addition to the structural aspects of the provided technology just described, these features support particular method embodiments. For example, a method of adjusting the fit of a modular prosthetic socket **100** may include mechanically adjusting the arrangement between the struts **300** and the distal base **200** so as to improve the conformal fit of the prosthetic socket on the residual limb. This type of adjusting may typically occur over an extended period of time during which the residual limb of a patient may expand or atrophy; in such a circumstance, a prosthetist could mechanically adjust the patient's prosthetic socket so as to improve the fit. In another instance, this type of adjustment may occur during the initial assembly of a prosthetic socket, or during an initial fitting of a prosthetic socket to a patient. The various sizes of a distal base **200,** as seen in **Figs. 15A - 15C** represent a way for a modular prosthetic socket system to be readily adaptable to be able to fit residual limbs of different width and volume by exploiting modular aspects of the technology; the adjustable features described here **(****Figs. 26A and 26B****)** expand that same general type of adjustability or adaptability such that it is available as an option within an individual modular prosthetic socket.

**Figs. 27A and 27B** show views of an embodiment of a modular prosthetic socket **100** in two configurations; **Fig. 27A** shows a socket **100** with one the struts **300** and its strut connector **220** at a neutral non-pivoted position on a distal base **200,** while **Fig. 27B** shows the same socket **100** with the struts and strut connectors **220** positioned at a pivoted position. These figures relate to the pivotability or swivelability of strut connectors **220** on a distal base **200,** as seen particularly well in **Figs. 17D and 17E****.** As can be seen, depending on the particulars of the dimensions of the distal base, the radial position of the strut connector **220,** and the confines of the strut connector site as determined by the configuration of base **200,** a strut connector can swivel up to about 45° from a central or neutral position within radial slot **212.** This angular degree of pivoting is reduced considerably as it translates into a pivoting of an attached strut, in the context of the full 360° circumference embodied by the struts collectively. However, being able to adjust the circumferential position of a strut only a few degrees, for example 5°, can be clinically significant.

**Figs. 27A and 27B** provide an example where a small degree of circumferential adjustability of a strut position can be important. It is not uncommon for patients to develop a so-called "hot-spot", a site of irritation on the limb that will only grow worse if its aggravated. Skin breakdown and/or infection can ensue. Such a site of irritation **701** is seen on residual limb **700** on the depicted patient. In **Fig. 27A****,** that site of irritation **701** is located directly below indicated strut **300.** The patient visits his prosthetist, and the prosthetist then pivots the strut connector **220** at the base of strut **300** such that the strut rotates laterally a few degrees (see arrow), exposing the site of irritation **701** and thereby providing it relief, and allowing it to heal.

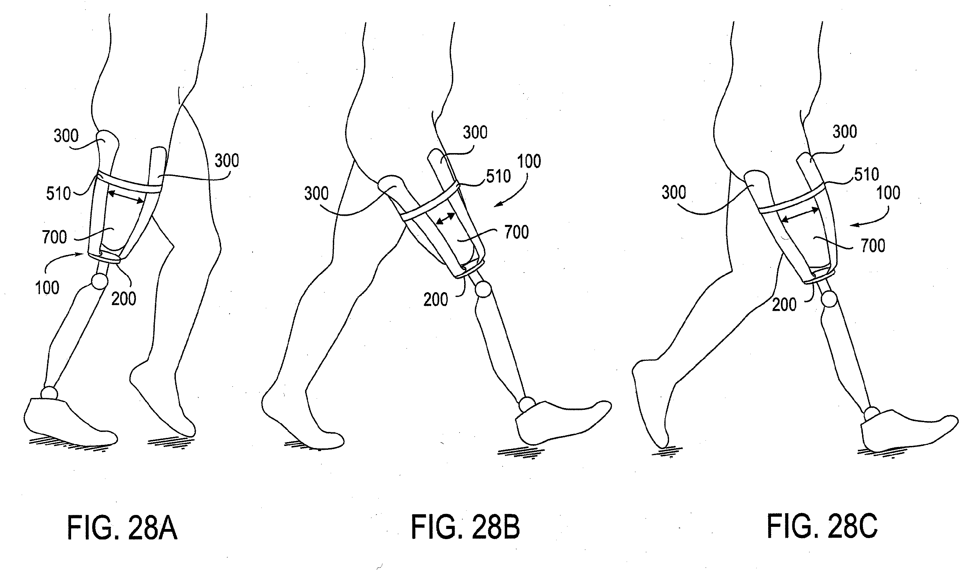

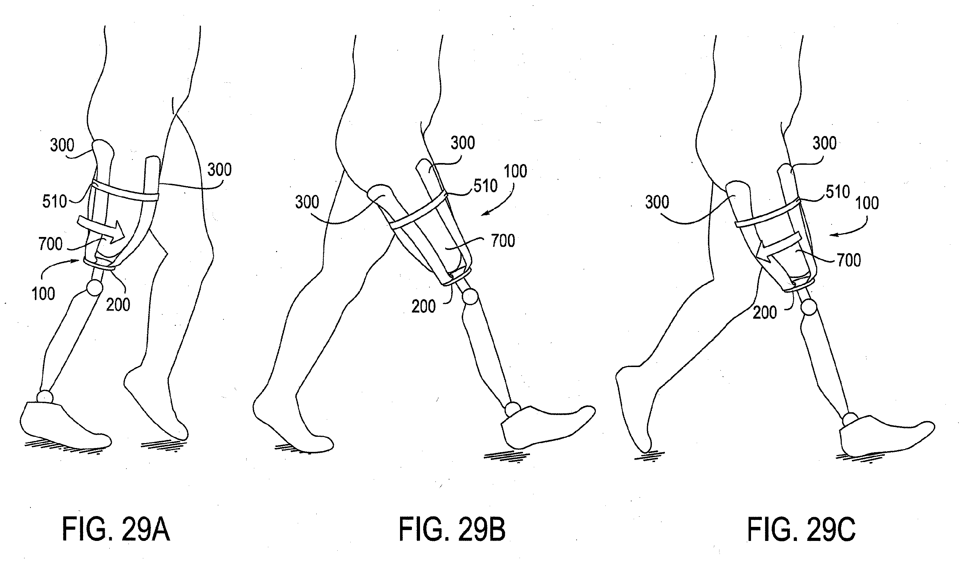

**Figs. 28A - 28C** and **Figs. 29A - 29C** show views of a modular prosthetic socket **100** being worn by a patient on residual limb **700** while walking. These two sets of figures depict separate aspects of a complex flexing of struts **300** during a stride. **Figs. 28A - 28C** show flexing in response to loading and unloading of weight on the struts during a stride. **Figs. 29A** - **29C** show flexing in response to forward and rearward forces imparted to the residual limb during a stride.

In **Fig. 28A****,** the patient's right leg is under a load, the right foot is starting to push off the ground and forward, as the left foot is swinging forward. In **Fig. 28B****,** the right leg (residual limb **700**) has swung forward and is momentarily free of load, now being absorbed by the left leg. In **Fig. 28C****,** the heel of the right foot has made contact with the ground and the right leg, once again, absorbing load. Thus, in **Figs. 28A and 28C****,** the right leg (residual limb **700**) is absorbing load, and in **Fig. 28B** it is unloaded. As the leg absorbs load, the struts **300** generally flex outward. As load is released, struts **300** generally flex inward. There may be differences in flexure according to the circumferential position of the struts. For example, as load is released from a leg, a strut in a posterior position may tend to flex inward more than an anterior strut.

**Figs. 29A** - **29C** show views of a modular prosthetic socket being worn by a patient while walking that are similar to those of **Figs. 28A** - **28C****,** except that these views show an aspect of the struts flexing in response to back and forth movement of the residual limb during strides. In **Fig. 29A** force originating at the point of contact of the right foot with the ground imparts forward directed force to residual limb **700,** driving an anterior strut **300** forward. A posterior strut **300** is consequently pulled forward by tensioning member **510.** In **Fig. 29B****,** the struts **300** assume a nominal neutral position. In **Fig. 29C****,** a net rearward force is imparted to the residual limb **700,** consequently causing a rearward flexion of struts **300.**

Patients evaluating an embodiment of the modular prosthetic socket **100** have commented on the flexing aspect of the socket during an evaluation; they speak of this flexing as an advantage, something that feels right, and helps their gait. It is understandable that kinetic energy derived from a heel strike that could be absorbed and released as body weight (as in **Figs. 28A** - **28C**) is being shifted away from the leg that struck the ground would benefit that following step, and generally enhance the gait. The forward and rearward flexing of the socket struts detailed in **Figs. 29A** - **29C** further enhance the totality of strut flexion that supports the stride.

Inventors theorize that the flexing of modular prosthetic socket may relate to several aspects of the socket. First, the struts, by virtue of their thermoplastic-fiber composite material, in the dimensions and configuration embodied by struts **300,** may be appropriately balanced between stiffness and compliance, such that the struts are generally non-flexing when the patient is standing still, or making small movements. However, body weight loading of a leg, as for example, by the force delivered by a heel strike during a purposeful walk, coupled to fore and aft forces associated with forward stride movement, may be sufficient to create the flexing. Second, the flexing may further be dependent on or enabled by an appropriate level of circumferential tensioning of the socket, as provided by adjusting tensioning elements. Third, the structure of the modular prosthetic socket may be particularly amenable to flexing from their fixed position on the distal base because of the lack of any circumferential integral structure in the socket that would preclude or constrain an inward flexing of the struts.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. Thus, it is intended that the scope of the present invention herein disclosed should not be limited by the particular disclosed embodiments described above, but should be determined only by a fair reading of the claims that follow.

## Claims

1. A modular prosthetic socket (100) for a residual limb of a lower extremity of a patient, the socket (100) comprising:
a base (200) having a center and a circumferential periphery;
multiple strut connectors (220) adjustably connected to the base along the periphery, wherein each of the multiple strut connectors (220) comprises;
a base portion (222);
a back portion (224), wherein a takeoff angle (226) between the base portion (222) and the back portion (224) is selected to provide the prosthetic socket (100) with a desired cross-sectional profile and volume;
a base attachment hole (231) on the base portion (222) for attaching the strut connector to the base (200); and
a back portion attachment hole (232) on the back portion (224); and
multiple longitudinal struts (300), each strut (300) comprising a thermoplastic-fiber composite material, a proximal end and a distal end, and wherein each strut (300) is connected to the base (200) along the periphery via one of the strut connectors (220),
wherein the prosthetic socket (100) circumscribes a proximally-open internal space configured to conform to the residual limb of the patient.

2. The modular prosthetic socket (100) of claim 1, wherein the base (200), the multiple strut connectors (220), and the multiple longitudinal struts (300) comprise modular components of the prosthetic socket (100).

3. The modular prosthetic socket (100) of claim 1, further comprising a tensioning member (510) coupled with the multiple struts (300) for applying tension to the multiple struts (300) in a direction approximately toward a central, longitudinal axis of the prosthetic socket (100).

4. The modular prosthetic socket (100) of claim 1, wherein the thermoplastic-fiber composite material comprises a polymer matrix comprising a material selected from the group consisting of polymethylmethacrylate, polycarbonate/ABS, high density polyethylene, polyethyleneterephthalate, polyetheretherketone, Nylon, and any blend thereof

5. The modular prosthetic socket (100) of claim 1, wherein the thermoplastic-fiber composite material comprises a fiber selected from the group consisting of carbon and glass.

6. The modular prosthetic socket (100) of claim 1, wherein the thermoplastic-fiber composite material comprises a fiber in a substantially continuous form.

7. The modular prosthetic socket (100) of claim 1, wherein the thermoplastic-fiber composite material comprises polymethylmethacrylate polymer with carbon fibers embedded therein.

8. The modular prosthetic socket (100) of claim 1, wherein the multiple struts (300) comprise at least three struts.

9. The modular prosthetic socket (100) of claim 1, wherein the base (200) comprises multiple radial slots (212), wherein each of the multiple radial slots (212) is configured to slidably host a one of the multiple strut connectors (220), and wherein each of the multiple radial slots (212) is radially aligned from the periphery of the base (200) toward the center of the base (200).

10. The modular prosthetic socket (100) of claim 1, wherein the takeoff angle is between 90 degrees and 160 degrees.

## Patentansprüche

1. Ein modularer Prothesenschaft (100) für einen Stumpf einer unteren Extremität eines Patienten, wobei der Schaft (100) Folgendes beinhaltet:
eine Basis (200), die eine Mitte und eine Umfangsperipherie aufweist;
mehrere Strebenverbinder (220), die verstellbar entlang der Peripherie mit der Basis verbunden sind, wobei jeder von den mehreren Strebenverbindern (220) Folgendes beinhaltet:
einen Basisanteil (222);
einen hinteren Anteil (224), wobei ein Abzweigwinkel (226) zwischen dem Basisanteil (222) und dem hinteren Anteil (224) so ausgewählt ist, dass dem Prothesenschaft (100) ein gewünschtes Querprofil und Volumen bereitgestellt wird;
ein Befestigungsloch (231) für die Basis auf dem Basisanteil (222) zum Befestigen des Strebenverbinders an der Basis (200); und
ein Befestigungsloch (232) für den hinteren Anteil auf dem hinteren Anteil (224); und
mehrere Längsstreben (300), wobei jede Strebe (300) ein thermoplastisches Faserverbundmaterial, ein proximales Ende und ein distales Ende beinhaltet und wobei jede Strebe (300) entlang der Peripherie über einen der Strebenverbinder (220) mit der Basis (200) verbunden ist,
wobei der Prothesenschaft (100) einen proximal offenen Innenraum umschreibt, der dazu ausgelegt ist, mit dem Stumpf des Patienten übereinzustimmen.

2. Modularer Prothesenschaft (100) nach Anspruch 1, wobei die Basis (200), die mehreren Strebenverbinder (220) und die mehreren Längsstreben (300) modulare Komponenten des Prothesenschafts (100) beinhalten.

3. Modularer Prothesenschaft (100) nach Anspruch 1, der ferner ein Spannelement (510), das mit den mehreren Streben (300) gekoppelt ist, zum Aufbringen von Spannung auf die mehreren Streben (300) in einer ungefähr zu einer mittigen Längsachse des Prothesenschafts (100) verlaufenden Richtung beinhaltet.

4. Modularer Prothesenschaft (100) nach Anspruch 1, wobei das thermoplastische Faserverbundmaterial eine Polymermatrix beinhaltet, die ein Material beinhaltet, das aus der Gruppe ausgewählt ist, die aus Polymethylmethacrylat, Polycarbonat/ABS, Polyethylen hoher Dichte, Polyethylenterephthalat, Polyetheretherketon, Nylon und einer Mischung davon besteht.

5. Modularer Prothesenschaft (100) nach Anspruch 1, wobei das thermoplastische Faserverbundmaterial eine Faser beinhaltet, die aus der Gruppe ausgewählt ist, die aus Kohlenstoff und Glas besteht.

6. Modularer Prothesenschaft (100) nach Anspruch 1, wobei das thermoplastische Faserverbundmaterial eine Faser in einer im Wesentlichen fortlaufenden Form beinhaltet.

7. Modularer Prothesenschaft (100) nach Anspruch 1, wobei das thermoplastische Faserverbundmaterial Polymethylmethacrylatpolymer mit darin eingebetteten Kohlenstofffasern beinhaltet.

8. Modularer Prothesenschaft (100) nach Anspruch 1, wobei die mehreren Streben (300) mindestens drei Streben beinhalten.

9. Modularer Prothesenschaft (100) nach Anspruch 1, wobei die Basis (200) mehrere radiale Schlitze (212) beinhaltet, wobei jeder von den mehreren radialen Schlitzen (212) dazu ausgelegt ist, einen der mehreren Strebenverbinder (220) verschiebbar aufzunehmen, und wobei jeder von den mehreren radialen Schlitzen (212) radial von der Peripherie der Basis (200) zu der Mitte der Basis (200) hin ausgerichtet ist.

10. Modularer Prothesenschaft (100) nach Anspruch 1, wobei der Abzweigwinkel zwischen 90 Grad und 160 Grad beträgt.

## Revendications

1. Emboîture prothétique modulaire (100) pour un membre résiduel d'une extrémité inférieure d'un patient, l'emboîture (100) comprenant :
une base (200) ayant un centre et une périphérie circonférentielle ;
des connecteurs de montant multiples (220) connectés de manière ajustable à la base le long de la périphérie,
dans laquelle chacun des connecteurs de montant multiples (220) comprend :
une partie de base (222) ;
une partie arrière (224), dans laquelle un angle de dégagement (226) entre la partie de base (222) et la partie arrière (224) est sélectionné pour fournir l'emboîture prothétique (100) avec un profil transversal et un volume souhaités ;
un orifice de fixation de base (231) sur la partie de base (222) destiné à fixer le connecteur de montant à la base (200) ; et
un orifice de fixation de partie arrière (232) sur la partie arrière (224) ; et
des montants longitudinaux multiples (300), chaque montant (300) comprenant un matériau composite de fibres thermoplastiques, une extrémité proximale et une extrémité distale, et dans laquelle chaque montant (300) est connecté à la base (200) le long de la périphérie via un des connecteurs de montant (220),
dans laquelle l'emboîture prothétique (100) délimite un espace interne ouvert de manière proximale configuré pour se conformer au membre résiduel du patient.

2. Emboîture prothétique modulaire (100) selon la revendication 1, dans laquelle la base (200), les connecteurs de montant multiples (220), et les montants longitudinaux multiples (300) comprennent des composants modulaires de l'emboîture prothétique (100).

3. Emboîture prothétique modulaire (100) selon la revendication 1, comprenant en outre un élément tendeur (510) couplé aux montants multiples (300) destiné à appliquer une tension aux montants multiples (300) dans une direction approximativement vers un axe longitudinal, central de l'emboîture prothétique (100).

4. Emboîture prothétique modulaire (100) selon la revendication 1, dans laquelle le matériau composite de fibres thermoplastiques comprend une matrice polymère comprenant un matériau sélectionné parmi le groupe constitué par le polyméthacrylate de méthyle, le polycarbonate/ABS, le polyéthylène haute densité, le polytéréphtalate d'éthylène, le polyétheréthercétone, le Nylon, et tout mélange homogène de ceux-ci.

5. Emboîture prothétique modulaire (100) selon la revendication 1, dans laquelle le matériau composite de fibres thermoplastiques comprend une fibre sélectionnée parmi le groupe constitué par le carbone et le verre.

6. Emboîture prothétique modulaire (100) selon la revendication 1, dans laquelle le matériau composite de fibres thermoplastiques comprend une fibre sous une forme substantiellement continue.

7. Emboîture prothétique modulaire (100) selon la revendication 1, dans laquelle le matériau composite de fibres thermoplastiques comprend un polymère de polyméthacrylate de méthyle avec des fibres de carbone incrustées dans celui-ci.

8. Emboîture prothétique modulaire (100) selon la revendication 1, dans laquelle les montants multiples (300) comprennent au moins trois montants.

9. Emboîture prothétique modulaire (100) selon la revendication 1, dans laquelle la base (200) comprend des fentes radiales multiples (212), dans laquelle chacune des fentes radiales multiples (212) est configurée pour recevoir de manière coulissante un des connecteurs de montant multiples (220), et dans laquelle chacune des fentes radiales multiples (212) est alignée de manière radiale de la périphérie de la base (200) vers le centre de la base (200).

10. Emboîture prothétique modulaire (100) selon la revendication 1, dans laquelle l'angle de dégagement est entre 90 degrés et 160 degrés.
